# EUROPEAN PATENT APPLICATION

(11) **EP 4 653 432 A1**
(43) Date of publication of application: **26.11.2025**
(21) Application number: 24178069.1
(22) Date of filing: 24.05.2024
(51) Int. Cl.: C07D 405/14, A61P 31/12, A61P 33/02, A61P 37/00, C07D 413/14, C07D 417/14, C07D 471/04

(54) **AUTOPHAGY INDUCING COMPOUNDS AND USES THEREOF IN THE PREVENTION OR THERAPY OF DISEASES**

(71) Applicant: Samsara Therapeutics Inc., Lewes, DE 19958 (US)
(72) Inventor: HAMLEY, Peter, Oxford OX4 4GA (GB); GALLOWAY, Warren, 61476 Kronberg im Taunus (DE); ATKINSON, Ben, Oxford OX4 4GA (GB)
(74) Representative: Krauss, Jan

(57) **Abstract**

The invention relates to pharmaceutical compositions and methods of preventing and/or treating autophagy related diseases and disorders. The present invention relates to compounds according to Formula (I) or salts, solvates and/or hydrates thereof, wherein said compounds induce and/or stimulate the process of autophagy, as well as uses of the compounds in the treatment and prevention of autophagy related diseases and disorders. Examples are cancer, age-related diseases, and viral infection.

## Description

The invention relates to pharmaceutical compositions and methods of preventing and/or treating autophagy related diseases and disorders. The present invention relates to compounds according to Formula (I) or salts, solvates and/or hydrates thereof, wherein said compounds induce and/or stimulate the process of autophagy, as well as uses of the compounds in the treatment and prevention of autophagy related diseases and disorders. Examples are cancer, age-related diseases, and viral infection.

### Background of the invention

Autophagy is the process of removing unnecessary organelles and proteins from cells that are lost or lost function, it helps maintain cell homeostasis and is a cell survival mechanism.

The cell-autonomous antimicrobial defense functions of autophagy, demonstrated initially in the case of streptococci and Mycobacterium tuberculosis have been extended to a wide variety of microbes with a caveat that most highly adapted pathogens have evolved specific protective mechanisms against autophagic elimination of microbes. Other studies have uncovered orderly intersections between autophagy and innate and adaptive immunity, T cell development, differentiation and homeostasis, and inflammatory responses. Thus, autophagy plays a large role in various diseases such as cancer, inflammatory disease, degenerative neurological disease, and immune disease.

Autophagy is a cell survival mechanism that is induced in stressed cells.

Towers and Thorburn (in: Therapeutic Targeting of Autophagy. EBioMedicine. 2016;14:15-23. doi:10.1016/j.ebiom.2016.10.034) disclose that autophagy is widely accepted as cytoprotective against neurodegenerative diseases and a variety of clinical interventions are moving forward to increase autophagy as a therapeutic intervention. Autophagy has both positive and negative roles in cancer and this has led to controversy over whether or how autophagy manipulation should be attempted in cancer therapy. Nevertheless, cancer is the disease where most current activity in trying to manipulate autophagy for therapy is taking place and dozens of clinical trials are using autophagy inhibition with Chloroquine or Hydroxychloroquine in combination with other drugs for the treatment of multiple neoplasms. They review recent literature implicating autophagy in neurodegenerative diseases and cancer and highlight some of the opportunities, controversies and potential pitfalls of therapeutically targeting autophagy.

Mulcahy Levy and Thorburn (in: Autophagy in cancer: moving from understanding mechanism to improving therapy responses in patients. Cell Death Differ 27, 843-857 (2020). https://doi.org/10.1038/s41418-019-0474-7) disclose that autophagy allows for cellular material to be delivered to lysosomes for degradation resulting in basal or stress-induced turnover of cell components that provide energy and macromolecular precursors. These activities are thought to be particularly important in cancer where both tumor-promoting and tumor-inhibiting functions of autophagy have been described. Autophagy has also been intricately linked to apoptosis and programmed cell death, and understanding these interactions is becoming increasingly important in improving cancer therapy and patient outcomes. In the review, they consider how recent discoveries about how autophagy manipulation elicits its effects on cancer cell behavior can be leveraged to improve therapeutic responses.

Grainger et al. (1995, Nature Medicine 1: 1067-1073) and Reckless et al. (1997, Circulation 95: 1542-1548) have demonstrated that tamoxifen, a potent inducer of autophagy, inhibited atherosclerosis in mice models by suppressing the diet-induced formation of lipid lesions in the aorta by lowering of low-density lipoprotein (LDL) cholesterol.

CN 102516239A discloses aromatic thiazole micro-molecular organic compounds with the structural formula represented by Formula (I), or hydrates thereof or pharmaceutically acceptable salts thereof. The compounds of the invention or compositions containing the compounds can be used for sun protection, anti-ultraviolet and skin damage protection as a cosmetic additive and for inhibition of the cell apoptosis caused by excess ultraviolet irradiation and the expression of cyclooxygenase COX2.

US 2004-0116425A1 discloses related compounds useful in the treatment of diseases associated with prenylation of proteins and pharmaceutically acceptable salts thereof, to pharmaceutical compositions comprising same, and to methods for inhibiting protein prenylation in an organism using the same.

WO 2009-103432A2 extremely broadly relates to molecular probes of the formula (I) L1-R1-L-A-X as defined herein that allow for the observation of the catalytic activity of a selected caspase, cathepsin, MMP and carboxypeptidase in in vitro assays, in cells or in multicellular organisms.

In WO 2010-147653A1 compounds are extremely broadly disclosed for treating ophthalmic conditions related to mislocalization of opsin proteins, the misfolding of mutant opsin proteins and the production of toxic visual cycle products that accumulate in the eye.

WO 2017-216579A1 relates to a heterocyclic compound 1,1'-(((propane-2,2-diylbis(4,1-phenylene))bis(oxy))bis(ethane-2,1-diyl))dipyrrolidine and its medical uses, for example as an autophagy inducer.

WO 2023-089074A1 relates to pharmaceutical compositions and methods of treating autophagy related diseases and disorders, in particular for a systemic treatment thereof. The present invention relates to compounds according to Formula (I) or salts, solvates and/or hydrates thereof, wherein said compounds induce and/or stimulate the process of autophagy, as well as uses of the compounds in the treatment and prevention of autophagy related diseases and disorders. Examples are cancer, age-related diseases, and viral infection that can be effectively treated with compounds that are effective when provided systemically.

The development of more selective autophagy inducers is needed if they are to become medicinally useful in the treatment and/or prevention of diseases where autophagy plays a role, in particular in the central nervous system (CNS). It is an object of the present invention to provide effective agents that can be used for the prevention and treatment of conditions and diseases that can be treated/prevented by inducing and/or stimulating of autophagy, in particular cancer, age-related diseases, and infections. Other objects and advantages will become apparent to the person of skill when studying the present description of the present invention.

In a first aspect of the present invention, the above object is solved by a compound according to Formula (I), 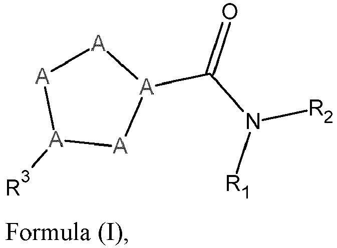 wherein
R¹ is selected from methyl, ethyl, and isopropyl
R² is a chemically possible ring of 5 or 6 atoms selected from saturated C and N and comprising at least one N, wherein the ring is optionally substituted with at least one C₁ to C₄ alkyl or halo,
A is selected from chemically possible saturated or unsaturated C, N, O, and S, comprising at least one N,
R³ is selected from 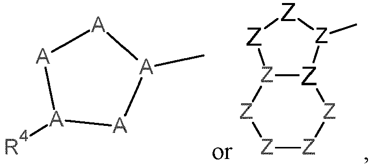 optionally substituted with - CN, halo or methyl, wherein A is as above, and Z is independently selected from chemically possible combinations of C, CH, N and NH
R⁴ is selected from hexyl, -C(CH₃)₃, or 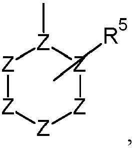
wherein Z is above and R⁵ is independently selected from H, straight or branched C₂ to C₆ alkyl, straight or branched C₁ to C₄ hydroxyalkyl, such as, for example, -CH₂-CH₂-OH, straight or branched C₁ to C₄ hydroxyalkoxy, such as, for example, -O-CH₂-CH₂-OH, halo, trifluoromethyl, cyclopropyl, or cyano, and
a physiologically acceptable salt, a solvate, a hydrate, an enantiomer or a polymorph thereof.

For example, A is independently selected from chemically possible combinations of C, CH, CH₂, N, NH, O, and S, comprising at least one N.

The compounds described herein were shown surprisingly to be highly effective autophagy inducers/stimulators. The present inventors thus invented compounds for the induction and/or stimulation of autophagy in disease- and undesired conditions-related cells of a patient or subject. In the case of a compound represented by the general Formula according to the present invention, the autophagy is effectively induced and/or stimulated. It was surprisingly found that the compounds according to Formula I, as an autophagy inducer, were effective and exhibit many advantages compared to other autophagy-related treatment options. In comparison with prior art compounds as tested (including data not shown), an improvement of the present invention lies in the unexpected observation that the compounds described herein are highly effective autophagy inducers (see Examples below).

Based on these results this is evidence that these compounds are active in autophagy-related diseases as disclosed herein. Furthermore, examples for this activity are shown in the Examples below which also relate to specific activities in models of viral infections and diseases involving protein misfolding. By way of an exemplar medical application, the compounds E and SLN5-X-0978 (Formula 157) were surprisingly efficacious (see Examples below).

Preferred is a compound according to the present invention according to the following formula II, 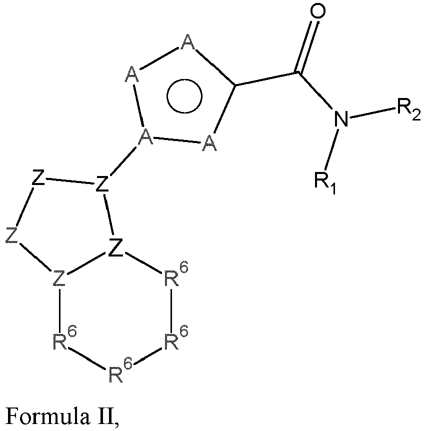
wherein R1, R2, Z and A are as above, R6 is independently selected from chemically possible combinations of C, CH and CH2 and wherein the rings are optionally substituted with -CN, halo or methyl,
a physiologically acceptable salt, a solvate, a hydrate, an enantiomer or a polymorph thereof.

Preferred is a compound according to the present invention according to the following formula III, 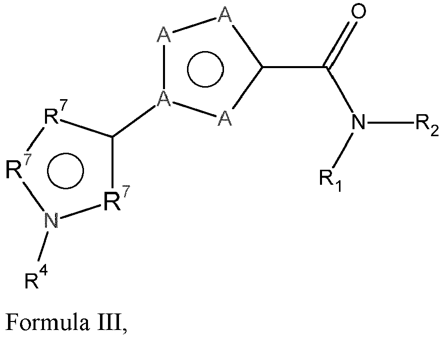
wherein R1, R2, R4 and A are as above, wherein the rings are optionally substituted with -CN, halo or methyl,
R7 is independently selected from chemically possible combinations of C, CH, N and NH, optionally substituted with cyano, and
a physiologically acceptable salt, a solvate, a hydrate, an enantiomer or a polymorph thereof.

Preferred is a compound according to the present invention selected from the following group 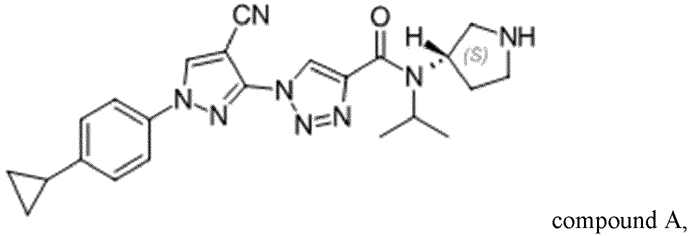 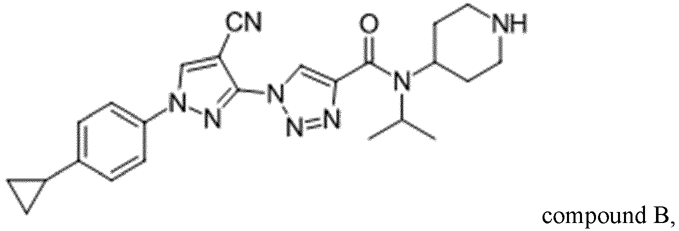 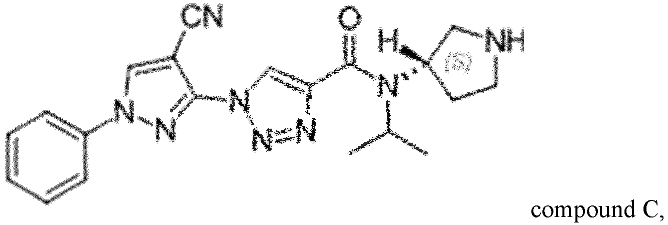 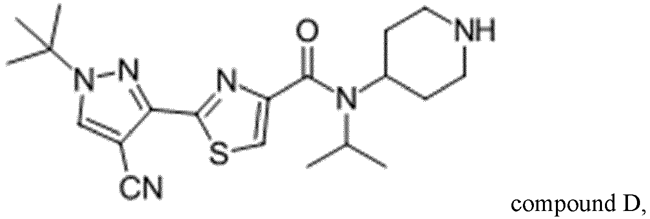 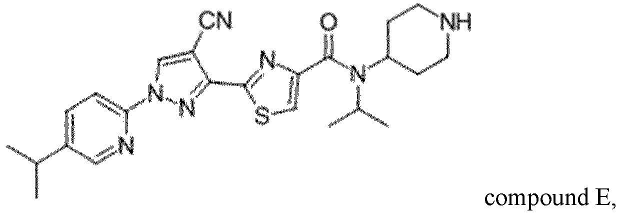 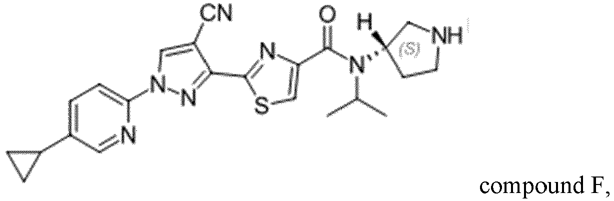 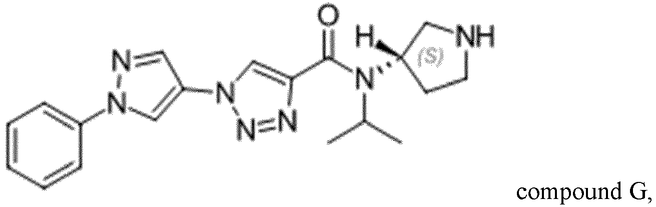 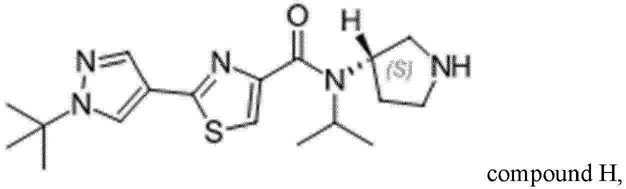 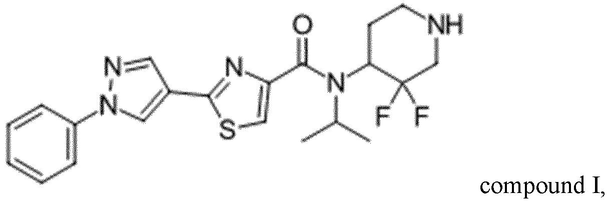 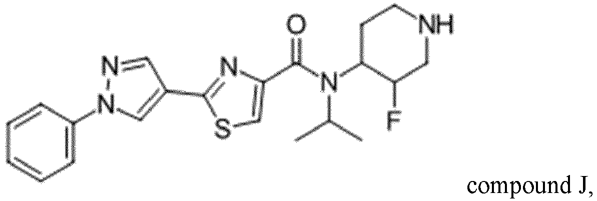 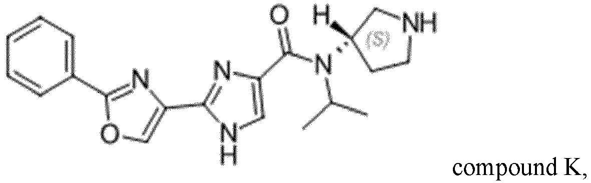 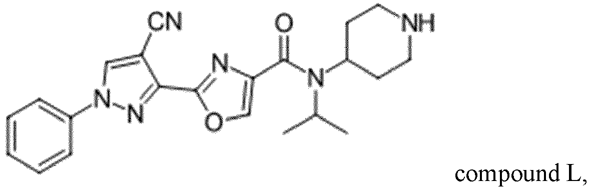 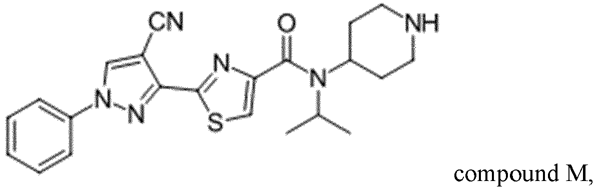 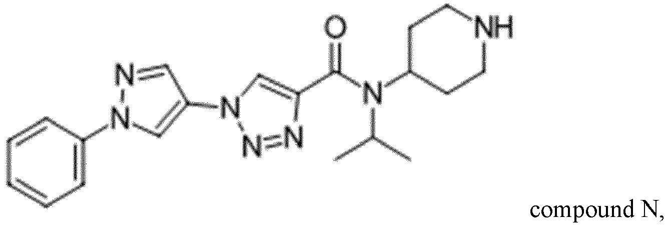 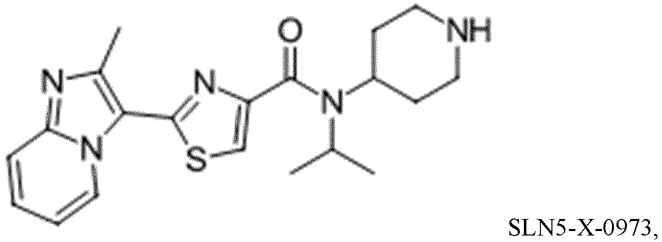 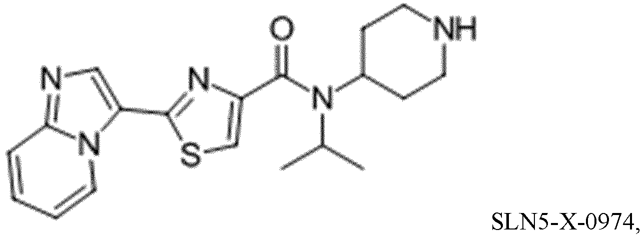 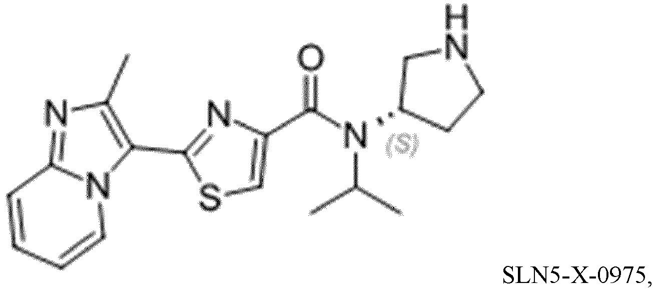 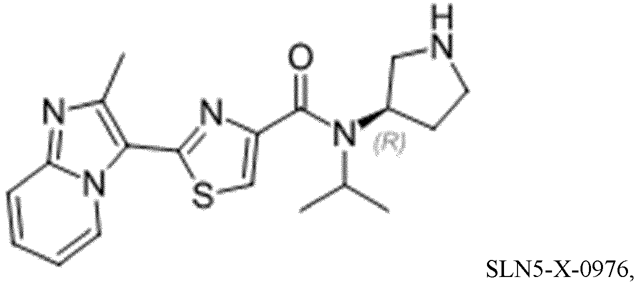 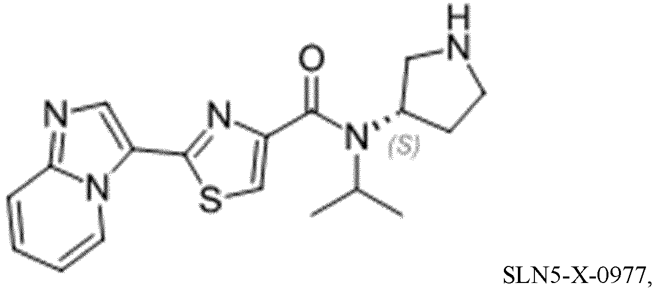 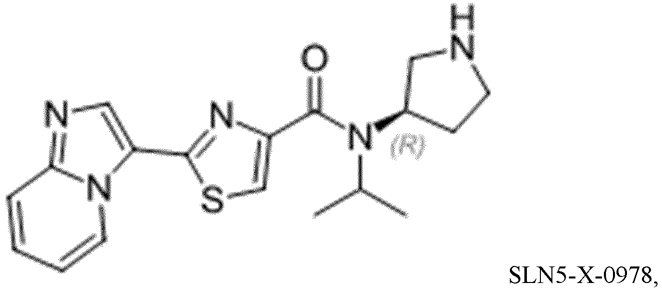 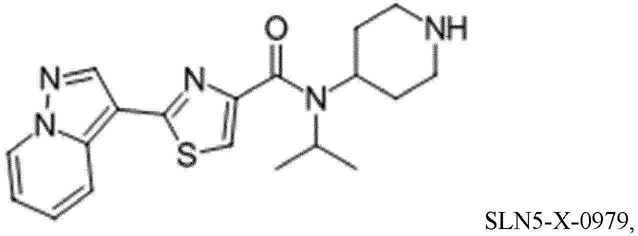 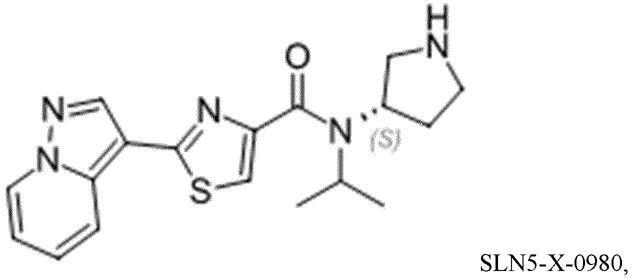 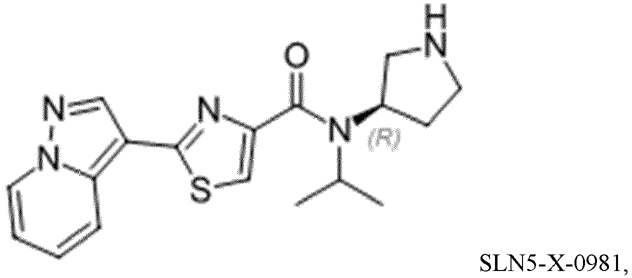 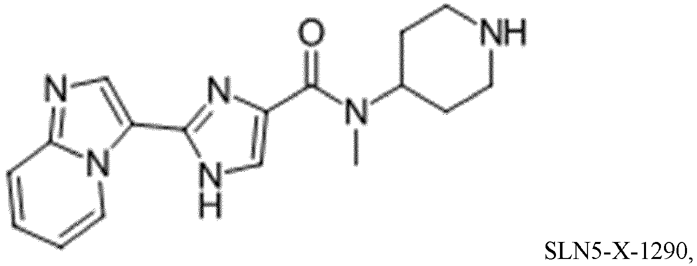 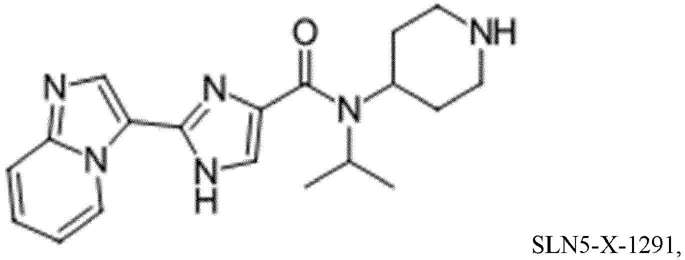 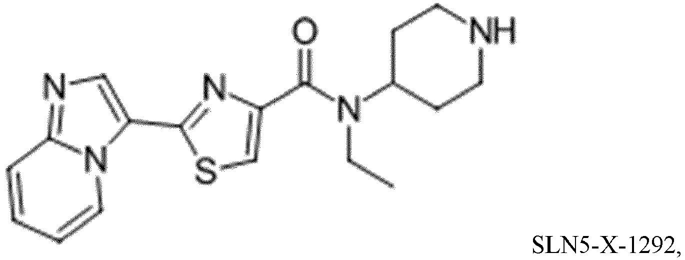 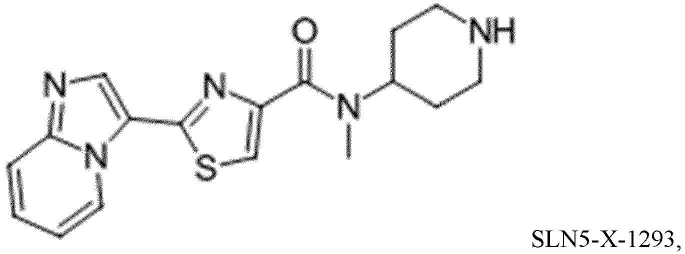 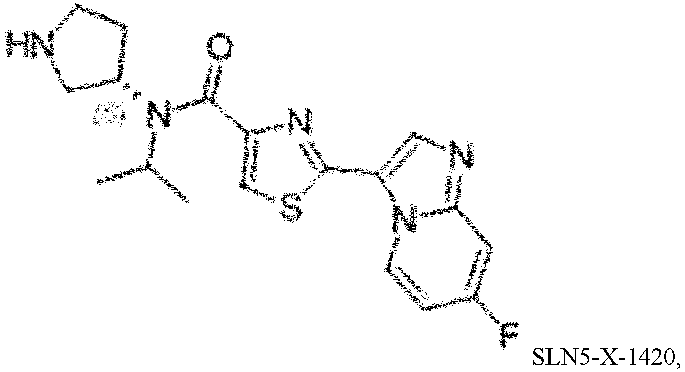 and
a physiologically acceptable salt, a solvate, a hydrate, an enantiomer or a polymorph thereof.

The term "pharmaceutically acceptable salt" refers to a pharmaceutically acceptable organic or inorganic salt of the compound of the invention. This may include addition salts of inorganic acids such as hydrochloride, hydrobromide, hydroiodide, sulphate, phosphate, diphosphate and nitrate or of organic acids such as acetate, maleate, fumarate, tartrate, succinate, citrate, lactate, methanesulphonate, p-toluenesulphonate, palmoate and stearate. Exemplary salts also include oxalate, chloride, bromide, iodide, bisulphate, acid phosphate, isonicotinate, salicylate, acid citrate, oleate, tannate, pantothenate, bitartrate, ascorbate, gentisinate, gluconate, glucuronate, saccharate, formate, benzoate, glutamate, ethanesulfonate, and benzenesulfonate salts. For other examples of pharmaceutically acceptable salts, reference can be made to Gould (1986, Int J Pharm 33: 201-217).

According to a further aspect of the invention, there is a provided a method for producing a compound according to the present invention, comprising the steps according to any one of general experimental procedures as disclosed herein and below.

According to a further aspect of the invention, there is a provided a pharmaceutical composition comprising a pharmaceutically effective amount of the compound according to the present invention, and a pharmaceutically or therapeutically acceptable excipient or carrier.

The term "pharmaceutically or therapeutically acceptable excipient or carrier" refers to a solid or liquid filler, diluent or encapsulating substance which does not interfere with the effectiveness or the biological activity of the active ingredients and which is not toxic to the host, which may be either humans or animals, to which it is administered. Depending upon the particular route of administration, a variety of pharmaceutically-acceptable carriers such as those well known in the art may be used. Non-limiting examples include sugars, starches, cellulose and its derivatives, malt, gelatin, talc, calcium sulfate, vegetable oils, synthetic oils, polyols, alginic acid, phosphate buffered solutions, emulsifiers, isotonic saline, and pyrogen-free water. Pharmaceutically acceptable carriers or excipients also include diluents (fillers, bulking agents, e.g. lactose, microcrystalline cellulose), disintegrants (e.g. sodium starch glycolate, croscarmellose sodium), binders (e.g. PVP, HPMC), lubricants (e.g. magnesium stearate), glidants (e.g. colloidal Si0₂), solvents/co-solvents (e.g. aqueous vehicle, Propylene glycol, glycerol), buffering agents (e.g. citrate, gluconates, lactates), preservatives (e.g. Na benzoate, parabens (Me, Pr and Bu), BKC), anti -oxidants (e.g. BHT, BHA, Ascorbic acid), wetting agents (e.g. polysorbates, sorbitan esters), thickening agents (e.g. methylcellulose or hydroxyethylcellulose), sweetening agents (e.g. sorbitol, saccharin, aspartame, acesulfame), flavoring agents (e.g. peppermint, lemon oils, butterscotch, etc.), humectants (e.g. propylene, glycol, glycerol, sorbitol). Other suitable pharmaceutically acceptable excipients are inter alia described in Remington's Pharmaceutical Sciences, 15th Ed., Mack Publishing Co., New Jersey (1991) and Bauer et al., Pharmazeutische Technologic, 5th Ed., Govi-Verlag Frankfurt (1997). The person skilled in the art knows suitable formulations for the compounds according to the present invention, and will readily be able to choose suitable pharmaceutically acceptable carriers or excipients, depending, e.g., on the formulation and administration route of the pharmaceutical composition.

All suitable modes of administration are contemplated according to the invention. For example, administration of the medicament may be via oral, subcutaneous, direct intravenous, slow intravenous infusion, continuous intravenous infusion, intravenous or epidural patient controlled analgesia (PCA and PCEA), intramuscular, intrathecal, epidural, intracistemal, intraperitoneal, transdermal, topical, buccal, sublingual, transmucosal, inhalation, intra-atricular, intranasal, rectal or ocular routes, abuse deterrent and abuse resistant formulations, sterile solutions suspensions and depots for parenteral use, and the like, administered as immediate release, sustained release, delayed release, controlled release, extended release and the like. The medicament may be formulated in discrete dosage units and can be prepared by any of the methods well known in the art of pharmacy. The pharmaceutical composition of the present invention can be formulated using methods known in the art to provide rapid, sustained or delayed release of the active ingredient after administration to a mammal. In general, at least one compound according to the present invention is admixed with at least one pharmaceutically acceptable carrier and/or excipient.

In addition to the aforementioned compounds of the invention, the pharmaceutical composition can contain two or more compounds according to the present invention and also other therapeutically active substances.

The dosage of the pharmaceutical composition according to the present invention can be appropriately selected according to the route of administration, the subject to be administered, the target disease and its severity, age, sex weight, individual differences and disease state. Dosage may be repeated several times a day.

According to the present invention, a mammalian subject can be preferably selected from a mouse, rat, cat, dog, rabbit, goat, sheep, horse, camel, lama, cow, monkey, a farm animal, a sport animal, and a pet, and a human.

Further provided is a compound of the invention as defined herein for use in the prevention and/or treatment of conditions and/or diseases in a mammalian subject, such as a human. A condition and/or disease suitable for treatment according to the relevant aspects of the invention is one which is characterized by defective or insufficient autophagy or which would benefit from modulation such as induction of autophagy.

The invention further encompasses the use of a compound of the invention as an autophagy inducer. The use may be a cosmetic use and/or in vitro, for example in an in vitro assay.

US 9,138,400, for example, relates to a cosmetic process for detoxifying the skin and/or for combating cutaneous aging, comprising the topical application on the skin of a composition that comprises at least one activator of the autophagy of cells of the skin. Eckhart L, Tschachler E, and Gruber F (in: Autophagic Control of Skin Aging. Front Cell Dev Biol. 2019;7:143. Published 2019 Jul 30. doi:10.3389/fcell.2019.00143) review the evidence for cell type-specific roles of autophagy in the skin and their differential contributions to aging.

Autophagy inhibition plays a key role in the pathogenesis of inherited autophagic vacuolar myopathies (including Danon disease, X- linked myopathy with excessive autophagy, and infantile autophagic vacuolar myopathy), all of which are characterized by lysosomal defects and an accumulation of autophagic vacuoles. Autophagic vacuolar myopathies and cardiomyopathies can also be secondary to treatment with autophagy-inhibiting drugs (chloroquine, hydroxychloroquine and colchicine), which are used experimentally to interrogate autophagic flux and clinically to treat malaria, rheumatological diseases, and gout.

Autophagy impairment has also been implicated in the pathogenesis of inclusion body myositis, an age- associated inflammatory myopathy that is currently refractory to any form of treatment, along with other muscular dystrophies such as tibial muscular dystrophy.

Modified basal autophagy levels are seen in rheumatoid arthritis and osteoarthritis. Other aspects of the immune response associated with dysfunctional autophagy are seen in neutrophils from patients with familial Mediterranean fever and in monocytes from patients with TNF receptor-associated periodic syndrome, both of which are autoinflammatory disorders. Moreover, autophagy regulates an important neutrophil function, the generation of neutrophil extracellular traps (NETs). The important role of autophagy in the induction of NET formation has been studied in several neutrophil-associated disorders such as gout, sepsis, and lung fibrosis. Furthermore, there is a relationship between autophagy and the secretory pathway in mammalian macrophages for the release of IL1B, demonstrating a possible alternative role of autophagy for protein trafficking. This role has also been implied in neutrophils through exposure of protein epitopes on NETs by acidified LC3-positive vacuoles in sepsis and antineutrophil cytoplasmic antibody associated vasculitis. Patients with chronic kidney disease also have impaired autophagy activation in leukocytes, which is closely related to their cardiac abnormalities. There is also evidence for altered autophagy in pancreatic beta cells and in adipocytes of patients with type 2 diabetes.

A crucial role for therapy-induced autophagy in cancer cells has recently emerged, in modulating the interface of cancer cells and the immune system; primarily, by affecting the nature of danger signaling (i.e., the signaling cascade that facilitates the exposure and/or release of danger signals) associated with immunogenic cell death (ICD).

Therefore, compounds according to the present invention are for use in the prevention and/or treatment of an autophagy-related disease or condition in a mammalian subject, such as a human.

By "treatment" or "treating" is meant any treatment of a disease or disorder, in a mammal, including: preventing or protecting against the disease or disorder, that is, causing, the clinical symptoms of the disease not to develop; inhibiting the disease, that is, arresting or suppressing the development of clinical symptoms; and/or relieving the disease, that is, causing the regression of clinical symptoms. By "amelioration" is meant the prevention, reduction or palliation of a state, or improvement of the state of a subject; the amelioration of a stress is the counteracting of the negative aspects of a stress. Amelioration includes, but does not require complete recovery or complete prevention of a stress.

Preferred is a compound for use according to the present invention, wherein said autophagy- related disease or condition is selected from the group consisting of systemic lupus erythematosus, cancer, liver diseases, al antitrypsin deficiency, Charcot Marie Tooth syndrome, Rett Syndrome, Sickle Cell disease, Wilson Disease, amyloidosis, Gaucher's diseases, lysosomal and glycogen storage disorders, cystic fibrosis, viral infection and diseases, human cytomegalovirus (HCMV) infection, hepatitis B, human immunodeficiency virus infection, Zika virus infection, coronavirus infection, HCoV-229E, HCoV-NL63, betacoronavirus infection, such as HCoV-OC43, SARS-CoV-1, HCoV-HKUl, MERS-CoV or SARS-CoV-2, bacterial infections, metabolic disorders, diabetes, fibrosis, wound healing disorders, Niemann-Pick type C (NPC) disease, fibrinogen storage disease (FSB), inclusion body disease (IBD), muscular dystrophy, Duchenne muscular dystrophy, Limb-girdle muscular dystrophy, myopathy, myofibrillar myopathy, hereditary myopathy, diabetic cardiomyopathy, anti-inflammatory disorders, autoimmune diseases, multiple sclerosis, rheumatoid arthritis, irritable bowel syndrome, Crohn's disease, vascular disorders, coronary artery diseases, myocardial infarction, unstable angina pectoris, atherosclerosis or vasculitis, Behcet's syndrome, giant cell arteritis, polymyalgia rheumatica, Wegener's granulomatosis, Churg- Strauss syndrome, vasculitis, Henoch-Schonlein purpura, Kawasaki disease, viral infection or replication, pox virus infection, herpes virus infection, asthma, allergic rhinitis, COPD, osteoporosis, organ transplant rejection, psoriasis, hypertrophic scarring (keloid formation), adhesion formations following general or gynecological surgery, lung fibrosis, liver fibrosis, kidney fibrosis, disorders caused by intracellular parasites, malaria, tuberculosis, neuropathic pain, post-operative phantom limb pain or postherpetic neuralgia, allergies, antigen induced recall response, immune response suppression, muscle degeneration and atrophy, frailty in aging, spinal cord injury, and diseases and conditions involving misfolded and/or nonfolded proteins that can be effectively treated with compounds that are effective when provided systemically as well as age-related forms of the diseases and conditions (such as cancer, and metabolic syndrome). Particularly preferred is Charcot Marie Tooth syndrome.

Cheon SY, Kim H, Rubinsztein DC, and Lee JE. (in: Autophagy, Cellular Aging and Age-related Human Diseases. Exp Neurobiol 2019;28:643-657. https://doi.org/10.5607/en.2019.28.6.643) disclose that during aging, cellular factors suggested as the cause of aging have been reported to be associated with progressively compromised autophagy. Dysfunctional autophagy may contribute to age-related diseases, such as neurodegenerative disease, cancer, and metabolic syndrome, in the elderly. Therefore, restoration of impaired autophagy to normal may help to prevent age-related disease and extend lifespan and longevity. They provide an overview of the mechanisms of autophagy underlying cellular aging and the consequent disease.

Similarly, Leidal, A.M., Levine, B. & Debnath, J. (in: Autophagy and the cell biology of age-related disease. Nat Cell Biol 20, 1338-1348 (2018). https://doi.org/10.1038/s41556-018-0235-8) review that autophagy declines with age and that impaired autophagy predisposes individuals to age-related diseases, whereas interventions that stimulate autophagy often promote longevity.

Also, Vaccaro Maria Ines, De Tata Vincenzo, and Gonzalez Claudio Daniel (in: Editorial: Autophagy in Endocrine-Metabolic Diseases Associated With Aging; Frontiers in Endocrinology, 11, 2020, pp 572, doi=10.3389/fendo.2020.00572) present a special issue containing a collection of 12 articles covering a broad range of key topics on the interplay of the different types of autophagy alterations with aging, endocrine-metabolic, and degenerative diseases.

Further preferred is the compound for use according to the present invention, wherein said prevention and/or treatment comprises a combination of at least two compounds for use according to the present invention, and/or a combination with at least one additional pharmaceutically active substance for said autophagy-related disease or condition.

It is to be understood that the present compound and/or a pharmaceutical composition comprising the present compound is for use to be administered to a human patient. The term "administering" means administration of a sole therapeutic agent or in combination with another therapeutic agent. It is thus envisaged that the pharmaceutical composition of the present invention are employed in co-therapy approaches, i.e. in co-administration with other medicaments or drugs and/or any other therapeutic agent which might be beneficial in the context of the methods of the present invention. Nevertheless, the other medicaments or drugs and/or any other therapeutic agent can be administered separately from the compound for use, if required, as long as they act in combination (i.e. directly and/or indirectly, preferably synergistically) with the present compound(s) (for use).

In another aspect of the compound for use according to the present invention the prevention and/or treatment further comprises detecting and/or monitoring in said subject a response of at least one autophagy-biomarker. The biomarker is preferably selected from the group consisting of BECN1, ATG8/LC3 family, including LC3A, LC3B, LC3C), LC3-II, ULK1, p62, NBR1, ATG5 and ATG7.

This monitoring usually is performed on a biologically sample taken from the mammalian subject, and comprises commonly known tests, for example antibody based, PCR based, and the like. The tests are repeated over time, and can be compared to control samples and/or samples taken earlier from the mammalian subject. The results help the attending physician to maintain or modify the course of a treatment, usually based on the severity of the clinical symptoms of the autophagy-related disease and/or condition as treated.

In another aspect thereof, the present invention provides methods for preventing and/or treating an autophagy-related disease and/or condition in a mammalian subject, such as a human, comprising administering to said mammal an effective amount of a compound or a pharmaceutical composition according to the present invention.

Preferred is the method according to the present invention, wherein said autophagy-related disease or condition is selected from the group consisting of systemic lupus erythematosus, cancer, liver diseases, al antitrypsin deficiency, Charcot Marie Tooth syndrome, Rett Syndrome, Sickle Cell disease, Wilson Disease, amyloidosis, Gaucher's diseases, lysosomal and glycogen storage disorders, cystic fibrosis; viral infection and diseases, human cytomegalovirus (HCMV) infection, hepatitis B, human immunodeficiency virus infection, Zika virus infection, coronavirus infection, HCoV-229E, HCoV-NL63, betacoronavirus infection, such as HCoV-OC43, SARS-CoV-1, HCoV-HKUl, MERS-CoV or SARS-CoV-2, bacterial infections, metabolic disorders, diabetes, fibrosis, wound healing disorders, Niemann- Pick type C (NPC) disease, fibrinogen storage disease (FSB), inclusion body disease (IBD), muscular dystrophy, Duchenne muscular dystrophy, Limb -girdle muscular dystrophy, myopathy, myofibrillar myopathy, hereditary myopathy, diabetic cardiomyopathy, antiinflammatory disorders, autoimmune diseases, multiple sclerosis, rheumatoid arthritis, irritable bowel syndrome, Crohn's disease, vascular disorders, coronary artery diseases, myocardial infarction, unstable angina pectoris, atherosclerosis or vasculitis, Behcet's syndrome, giant cell arteritis, polymyalgia rheumatica, Wegener's granulomatosis, Churg-Strauss syndrome, vasculitis, Henoch-Schonlein purpura, Kawasaki disease, viral infection or replication, pox virus infection, herpes virus infection, asthma, allergic rhinitis, COPD, osteoporosis, organ transplant rejection, psoriasis, hypertrophic scarring (keloid formation), adhesion formations following general or gynecological surgery, lung fibrosis, liver fibrosis, kidney fibrosis, disorders caused by intracellular parasites, malaria, tuberculosis, neuropathic pain, postoperative phantom limb pain or postherpetic neuralgia, allergies, antigen induced recall response, immune response suppression, muscle degeneration and atrophy, frailty in aging, spinal cord injury, and diseases and conditions involving misfolded and/or nonfolded proteins that can be effectively treated with compounds that are effective when provided systemically. Particularly preferred is Charcot Marie Tooth syndrome.

The dosage of the pharmaceutical composition to be administered according to the present invention can be appropriately selected according to the route of administration, the subject to be administered, the target disease and its severity, age, sex weight, individual differences and disease state. Dosage may be repeated several times a day.

According to the present invention, a mammalian subject can be preferably selected from a mouse, rat, cat, dog, rabbit, goat, sheep, horse, camel, lama, cow, monkey, a farm animal, a sport animal, and a pet, and a human.

In addition to the aforementioned compounds of the invention, the pharmaceutical composition as administered can contain two or more compounds according to the present invention and also other therapeutically active substances. In the method, the compound for use can be provided and/or is administered as a suitable pharmaceutical composition as discussed above. The compounds can be administered alone or in combination with other active compounds - for example with medicaments already known for the treatment of the aforementioned conditions and/or diseases, whereby in the latter case a favorable additive, amplifying or preferably synergistically effect is noticed.

In another aspect of the method according to the present invention the prevention and/or treatment further comprises detecting and/or monitoring in said subject a response of at least one autophagy-biomarker. The biomarker is preferably selected from the group of BECN1, and the ATG8/LC3 family, including LC3A, LC3B, LC3C), LC3-II, ULK1, p62, NBR1, ATG5 and ATG7.

This monitoring usually is performed on a biologically sample taken from the mammalian subject, and comprises commonly known tests, for example antibody based, PCR based, and the like. The tests are repeated over time, and can be compared to control samples and/or samples taken earlier from the mammalian subject. The results help the attending physician to maintain or modify the course of a treatment, usually based on the severity of the clinical symptoms of the autophagy-related disease and/or condition as treated.

It was surprisingly found that the compounds according to Formula I, as an autophagy inducer, were effective and exhibits many advantages compared to other autophagy-related treatment options, particularly as a combination treatment. In comparison with prior art compounds as tested (including data not shown), an improvement of the present invention lies in the unexpected observation that the compounds described herein are highly effective autophagy inducers (see Examples below).

The present invention relates to the following items.

Item 1. A compound according to the general Formula (I) 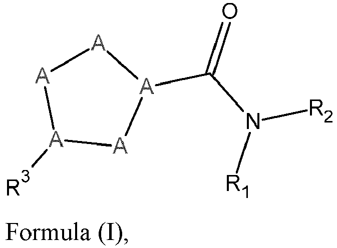 wherein
R¹ is selected from methyl, ethyl, and isopropyl,
R² is a chemically possible ring of 5 or 6 atoms selected from saturated C and N and comprising at least one N, wherein the ring is optionally substituted with at least one C₁ to C₄ alkyl or halo,
A is selected from chemically possible saturated or unsaturated C, N, O, and S, comprising at least one N,
R³ is selected from 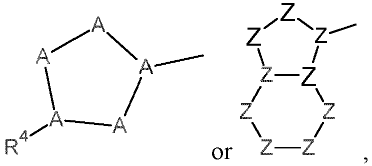 optionally substituted with - CN, halo or methyl, wherein A is as above, and Z is independently selected from chemically possible combinations of C, CH, N and NH
R⁴ is selected from hexyl, -C(CH₃)₃, or 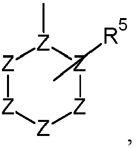
wherein Z is above and R⁵ is independently selected from H, straight or branched C₂ to C₆ alkyl, straight or branched C₁ to C₄ hydroxyalkyl, such as, for example, -CH₂-CH₂-OH, straight or branched C₁ to C₄ hydroxyalkoxy, such as, for example, -O-CH₂-CH₂-OH, halo, trifluoromethyl, cyclopropyl, or cyano, and
a physiologically acceptable salt, a solvate, a hydrate, an enantiomer or a polymorph thereof.

Item 2. A compound according to Item 1 according to the general Formula (II), 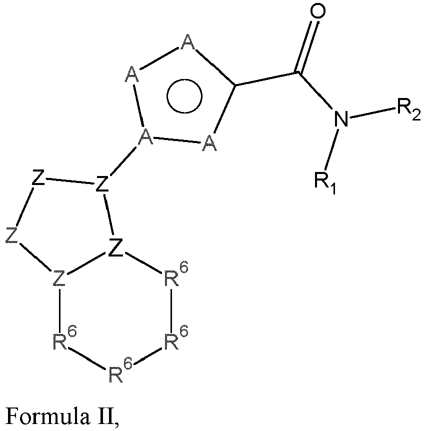
wherein R1, R2, Z and A are as above, R6 is independently selected from chemically possible combinations of C, CH and CH2 and wherein the rings are optionally substituted with -CN, halo or methyl,
a physiologically acceptable salt, a solvate, a hydrate, an enantiomer or a polymorph thereof.

Item 3. A compound according to Item 1 according to the general Formula (III), 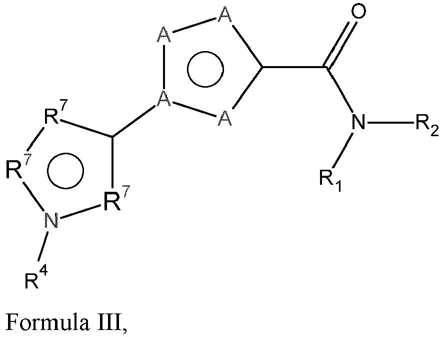
wherein R1, R2, R4 and A are as above, wherein the rings are optionally substituted with -CN, halo or methyl,
R7 is independently selected from chemically possible combinations of C, CH, N and NH, optionally substituted with cyano, and
a physiologically acceptable salt, a solvate, a hydrate, an enantiomer or a polymorph thereof.

Item 4. A compound selected from the following group 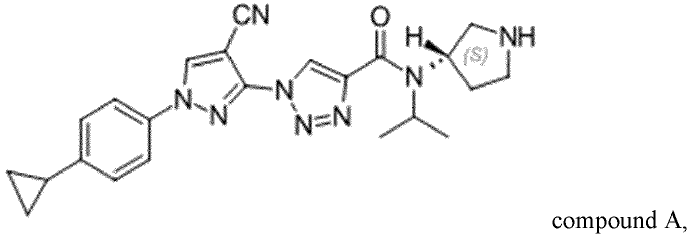 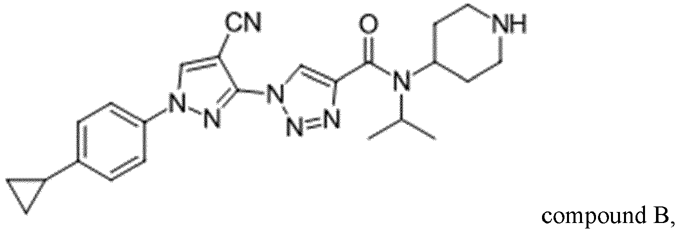 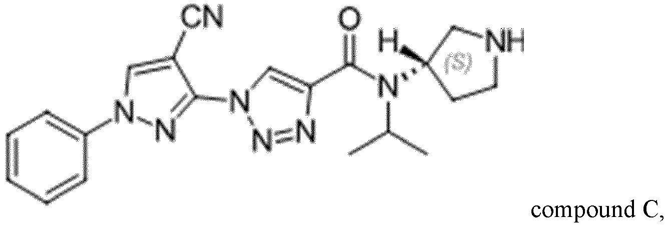 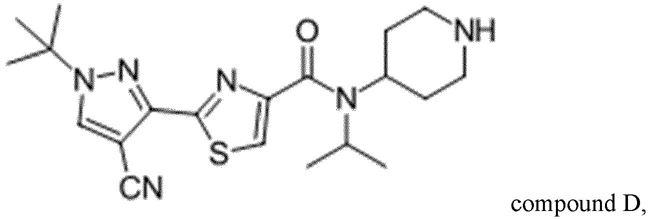 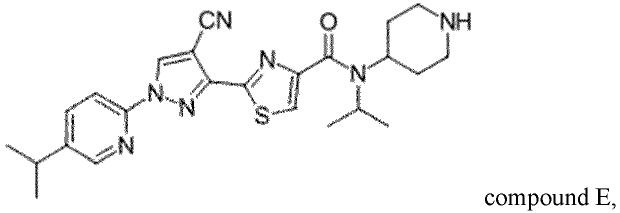 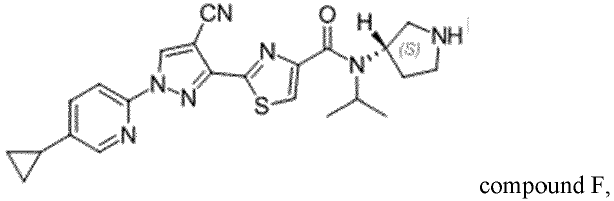 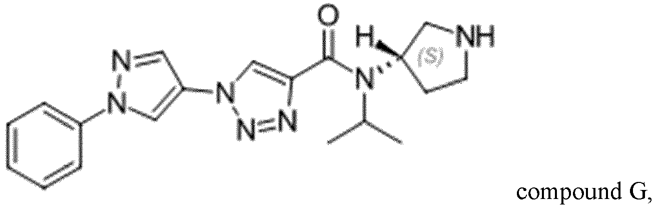 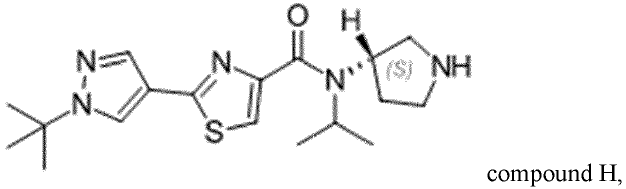 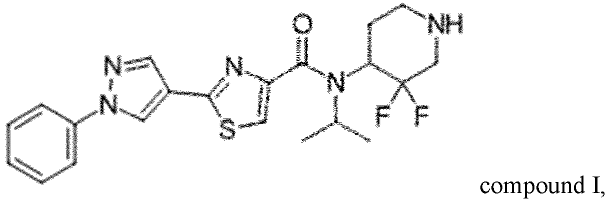 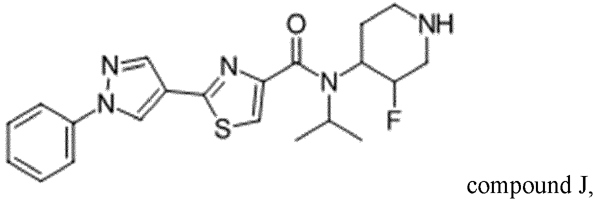 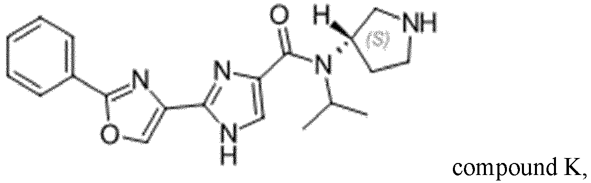 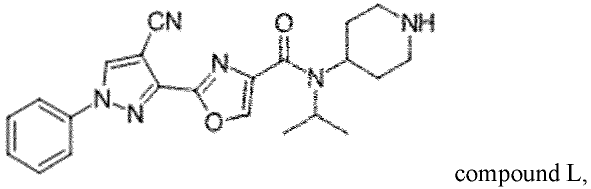 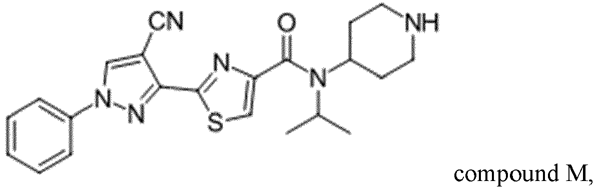 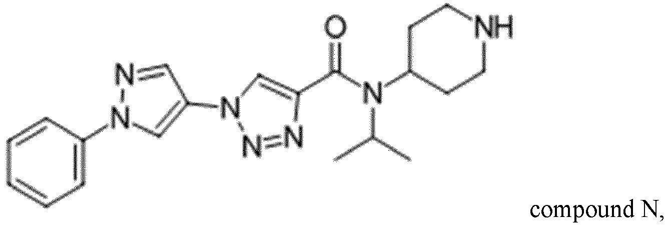 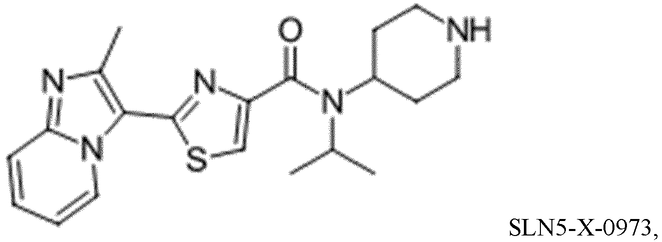 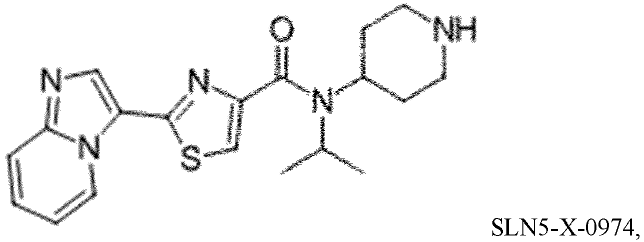 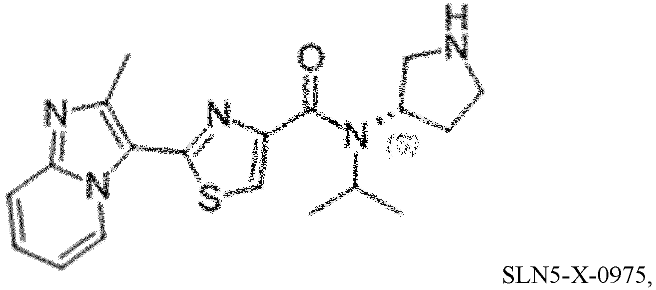 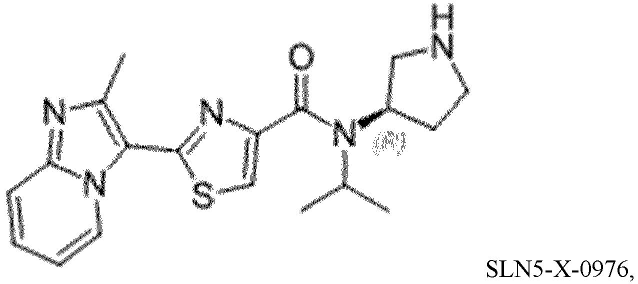 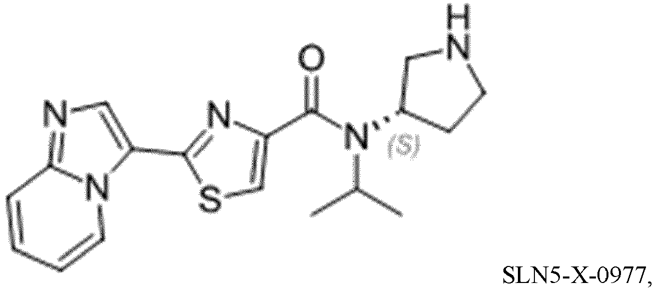 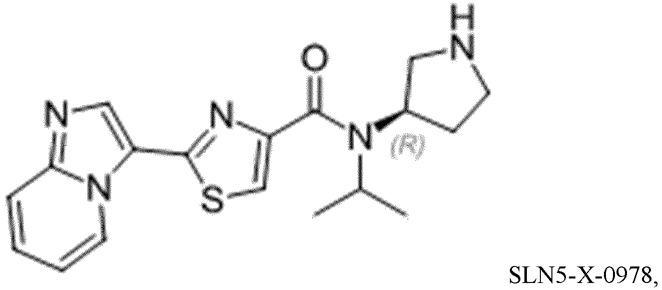 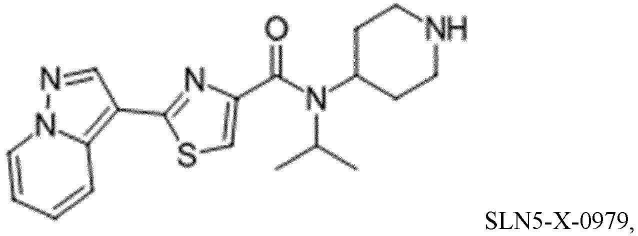 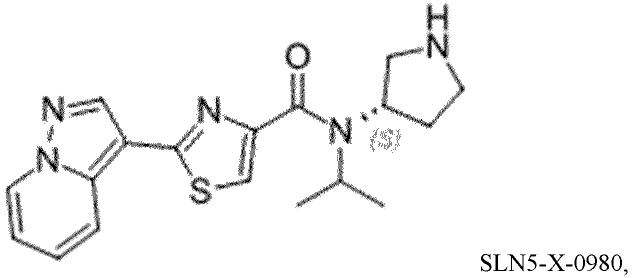 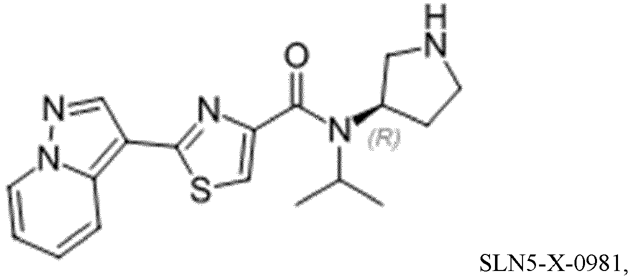 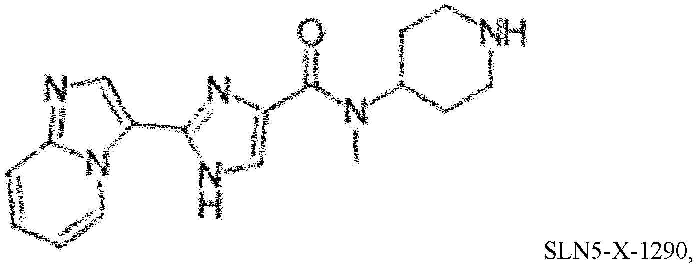 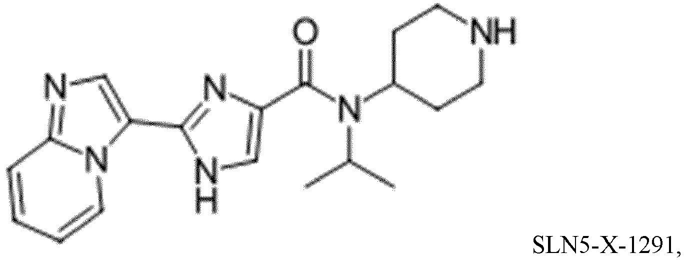 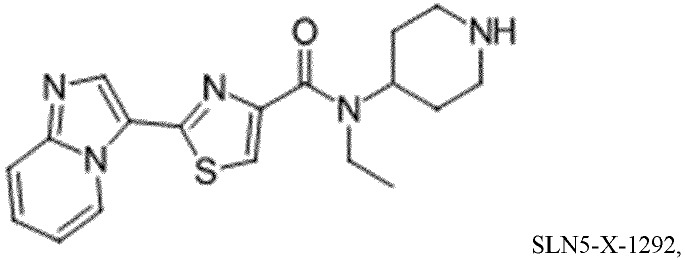 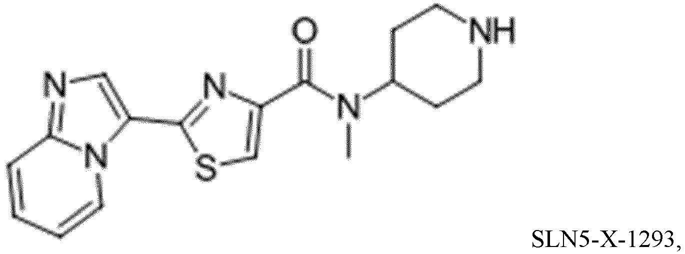 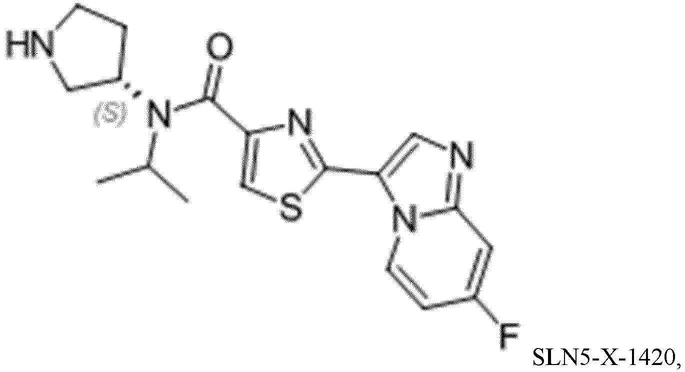 and
a physiologically acceptable salt, a solvate, a hydrate, an enantiomer or a polymorph thereof.

Item 5. A pharmaceutical composition, comprising a pharmaceutically effective amount of the compound according to any one of Items 1 to 4, and a pharmaceutically acceptable carrier.

Item 6. The compound according to any one of Items 1 to 4 or the pharmaceutical composition according to Item 5 for use in the prevention and/or treatment of diseases in a mammalian subject, such as a human.

Item 7. The compound according to any one of Items 1 to 4 or the pharmaceutical composition according to Item 5 for use as an autophagy inducer, wherein preferably said use is cosmetic and/or in vitro.

Item 8. The compound according to any one of Items 1 to 4 or the pharmaceutical composition according to Item 5 for use in the prevention and/or treatment of an autophagy-related disease or condition in a mammalian subject, such as a human.

Item 9. The compound for use according to Item 8, wherein said autophagy-related disease or condition is selected from the group consisting of systemic lupus erythematosus, cancer, liver diseases, al antitrypsin deficiency, Charcot Marie Tooth syndrome, Rett Syndrome, Sickle Cell disease, Wilson Disease, amyloidosis, Gaucher's diseases, lysosomal and glycogen storage disorders, cystic fibrosis; viral infection and diseases, human cytomegalovirus (HCMV) infection, hepatitis B, human immunodeficiency virus infection, Zika virus infection, coronavirus infection, HCoV-229E, HCoV-NL63, betacoronavirus infection, such as HCoV-OC43, SARS-CoV-1, HCoV-HKUl, MERS-CoV or SARS-CoV-2, bacterial infections, metabolic disorders, diabetes, fibrosis, wound healing disorders, Niemann-Pick type C (NPC) disease, fibrinogen storage disease (FSB), inclusion body disease (IBD), muscular dystrophy, Duchenne muscular dystrophy, Limb-girdle muscular dystrophy, myopathy, myofibrillar myopathy, hereditary myopathy, diabetic cardiomyopathy, anti-inflammatory disorders, autoimmune diseases, multiple sclerosis, rheumatoid arthritis, irritable bowel syndrome, Crohn's disease, vascular disorders, coronary artery diseases, myocardial infarction, unstable angina pectoris, atherosclerosis or vasculitis, Behcet's syndrome, giant cell arteritis, polymyalgia rheumatica, Wegener's granulomatosis, Churg- Strauss syndrome, vasculitis, Henoch-Schonlein purpura, Kawasaki disease, viral infection or replication, pox virus infection, herpes virus infection, asthma, allergic rhinitis, COPD, osteoporosis, organ transplant rejection, psoriasis, hypertrophic scarring (keloid formation), adhesion formations following general or gynecological surgery, lung fibrosis, liver fibrosis, kidney fibrosis, disorders caused by intracellular parasites, malaria, tuberculosis, neuropathic pain, post-operative phantom limb pain or postherpetic neuralgia, allergies, antigen induced recall response, immune response suppression, muscle degeneration and atrophy, frailty in aging, spinal cord injury, and diseases and conditions involving misfolded and/or nonfolded proteins that can be effectively treated with compounds that are effective when provided systemically as well as age-related forms of the diseases and conditions (such as cancer, and metabolic syndrome).

Item 10. The compound for use according to Item 8 or 9, wherein said prevention and/or treatment comprises a combination of at least two compounds for use according to Item 8 or 9, and/or a combination with at least one additional pharmaceutically active substance for said autophagy-related disease or condition.

Item 11. The compound for use according to any one of Items 8 to 10, wherein said prevention and/or treatment further comprises detecting and/or monitoring in said subject a response of at least one autophagy-biomarker, preferably selected from the group of BECN1, and the ATG8/LC3 family, including LC3A, LC3B, LC3C), LC3-II, ULK1, p62, NBR1, ATG5 and ATG7.

Item 12. A method for preventing and/or treating an autophagy-related disease or condition in a mammalian subject, such as a human, comprising administering to said mammal an effective amount of at least one compound according to any one of Items 1 to 4 or a pharmaceutical composition according to Item 5.

Item 13. The method according to Item 12, wherein said autophagy-related disease or condition is selected from the group consisting of systemic lupus erythematosus, cancer, liver diseases, al antitrypsin deficiency, Charcot Marie Tooth syndrome, Rett Syndrome, Sickle Cell disease, Wilson Disease, amyloidosis, Gaucher's diseases, lysosomal and glycogen storage disorders, cystic fibrosis; viral infection and diseases, human cytomegalovirus (HCMV) infection, hepatitis B, human immunodeficiency virus infection, Zika virus infection, coronavirus infection, HCoV-229E, HCoV-NL63, betacoronavirus infection, such as HCoV-OC43, SARS-CoV-1, HCoV-HKUl, MERS-CoV or SARS-CoV-2, bacterial infections, metabolic disorders, diabetes, fibrosis, wound healing disorders, Niemann-Pick type C (NPC) disease, fibrinogen storage disease (FSB), inclusion body disease (IBD), muscular dystrophy, Duchenne muscular dystrophy, Limb-girdle muscular dystrophy, myopathy, myofibrillar myopathy, hereditary myopathy, diabetic cardiomyopathy, anti-inflammatory disorders, autoimmune diseases, multiple sclerosis, rheumatoid arthritis, irritable bowel syndrome, Crohn's disease, vascular disorders, coronary artery diseases, myocardial infarction, unstable angina pectoris, atherosclerosis or vasculitis, Behcet's syndrome, giant cell arteritis, polymyalgia rheumatica, Wegener's granulomatosis, Churg- Strauss syndrome, vasculitis, Henoch-Schonlein purpura, Kawasaki disease, viral infection or replication, pox virus infection, herpes virus infection, asthma, allergic rhinitis, COPD, osteoporosis, organ transplant rejection, psoriasis, hypertrophic scarring (keloid formation), adhesion formations following general or gynecological surgery, lung fibrosis, liver fibrosis, kidney fibrosis, disorders caused by intracellular parasites, malaria, tuberculosis, neuropathic pain, post-operative phantom limb pain or postherpetic neuralgia, allergies, antigen induced recall response, immune response suppression, muscle degeneration and atrophy, frailty in aging, spinal cord injury, and diseases and conditions involving misfolded and/or nonfolded proteins that can be effectively treated with compounds that are effective when provided systemically as well as age-related forms of the diseases and conditions (such as cancer, and metabolic syndrome).

Item 14. The method according to Item 12 or 13, further comprising detecting and/or monitoring in said subject a response of at least one autophagy-biomarker, preferably selected from the group of BECN1, and the ATG8/LC3 family, including LC3A, LC3B, LC3C), LC3-II, ULK1, p62, NBR1, ATG5 and ATG7.

The present invention will now be described further in the following examples, nevertheless, without being limited thereto. For the purposes of the present invention, all references as cited herein are incorporated by reference in their entireties.

### EXAMPLES

The following examples have been performed in part using preferred compounds as disclosed. Nevertheless, it should be understood that the present invention is not in any way limited thereto, and the person of skill is readily able to adjust the conditions as described to other compounds according to the present invention.

The compounds of the present invention were synthesized from commercially available starting materials, in preferably by following the relevant general experimental compounds of the present invention were prepared according to the following synthetic procedures and Examples and are further exemplified by the following specific examples. Unless otherwise indicated in the following, the starting materials are obtained from commercial suppliers, which is indicated for example by the CAS numbers, and used without further purification. However, it is to be understood that in case the CAS number is not indicated below a starting material, this does not mean that the starting material was not obtained from commercial suppliers. Further, it is to be understood that the starting materials for the examples are either commercially available or are readily prepared by standard methods from known materials.

### Abbreviations used:

- Boc: tertiary-butoxycarbonyl
- tBu: tertiary butyl
- DCM: dichloromethane
- DIPEA: N,N-Diisopropylethylamine
- DMF: dimethylformamide
- DMSO: dimethylsulfoxide
- dppf: 1, 1'-bis(diphenylphosphino)ferrocene
- eq: equivalents
- ESI: electrospray ionisation
- Hz: hertz
- h: hours
- HATU: 1-[Bis(dimethylamino)methylene]-1H-1,2,3-triazolo[4,5-b]pyridinium 3-oxide hexafluorophosphate
- HPLC: high performance liquid chromatography
- *J*: coupling constant in Hz
- LCMS: liquid chromatography-mass spectrometry
- min: minutes
- NMR: nuclear magnetic resonance
- N: normal (equivalents per litre)
- Ph: phenyl
- RT: retention time
- rt: room temperature
- RuPhos: X-Phos
- SFC: supercritical fluid chromatography
- THF: tetrahydrofuran
- TLC: thin-layer chromatography
- vol: volumes
- X-Phos: 2-Dicyclohexylphosphin-2',4',6'-triisopropylbiphenyl
- X-Phos Pd G2: Second generation XPhos Precatalyst (X-Phos aminobiphenyl palladium chloride pre-catalyst); Chloro(2-dicyclohexylphosphino-2',4',6'-triisopropyl-1,1'-biphenyl)[2-(2'-amino-1, 1 '-biphenyl)]palladium(II)

### Standard set-up:

***Column details*** X-BRIDGE BEH C18 (2.1 x 50 mm), 2.5µm
***Machine details*** Column temperature: 35°C, Auto sampler temperature: 5°C, Mobile Phase A: 0.1 % Formic acid in Milli Q water (pH= 2.70), Mobile Phase B: 0.1% Formic acid in Milli Q water: Acetonitrile (10:90).
***Mobile phase gradient details*** T = 0 min (97% A, 3% B) flow: 0.8 mL/min; T = 0.75 min (97% A, 3% B) flow : 0.8 mL/min; gradient to T = 2.7 min (2% A, 98% B) flow : 0.8 mL/min; gradient to T = 3 min (0% A, 100% B) flow : 1 mL/min; T = 3.5 min (0% A, 100% B) flow : 1 mL/min; gradient to T= 3.51 min (97% A, 3% B) flow: 0.8 mL/min; end of run at T = 4 min (97% A, 3% B), Flow rate: 0.8 mL/min, Run Time: 4 min, UV Detection Method: PDA.
***Mass parameter*** Probe: -ESI, Mode of Ionisation: Positive and Negative, Cone voltage : 30 V and 10 V, capillary voltage: 0.8 KV, Extractor Voltage: 1 V, Rf Lens: 0.1 V, Temperature of source: 120°C,Temperature of Probe: 600°C Cone Gas Flow: 50 L/Hr , Desolvation Gas flow: 850 L/Hr.

### High-performance liquid chromatography (HPLC)

For most compounds HPLC was carried out using a Waters Alliance e2695 equipped with a 2998 Photodiode Array (PDA) detector.
Standard set-up for HPLC using a Waters Alliance e2695 equipped with a 2998 Photodiode Array (PDA) detector:
***Column details*** X-Bridge C18 (150 x 4.6 mm), 3.5µm
***Column temperature*** 25°C
***Auto sampler temperature*** 25°C
***Mobile Phase A*** 0.1% Formic acid in HPLC water
***Mobile Phase B*** 100% Acetonitrile
***Mobile phase gradient details*** T = 0 min (10% A, 90% B) flow : 1 mL/min; T = 7 min (90%A, 10% B) flow : 1 mL/min; gradient to T = 9 min (100% A, 0% B) flow : 1 mL/min; gradient to T = 14 min (100% A, 0% B) flow : 1 mL/min; T = 14.01 min (10% A, 90% B) flow : 1 mL/min; gradient to T= 17 min (10% A, 90% B) flow : 1 mL/min; end of run at T = 17 min (10% A, 90% B), Flow rate: 1 mL/min, Run Time: 17 min, UV Detection Method: PDA.

For compounds SLN5-X-0978, Compound I, Compound J, Compound M, Compound N, Compound L, Compound E, Compound F, Compound C, Compound A, Compound B, and Compound K HPLC was carried out using a SHIMADZU i-Series LC-2050C 3D system with a Photodiode Array (PDA) detector.

Standard set-up for HPLC using a SHIMADZU i-Series LC-2050C 3D system with a Photodiode Array (PDA) detector:
***Column details*** Sunfire C18 (150 x 4.6mm), 3.5µm
***Column temperature*** 25°C
***Auto sampler temperature*** 25°C
***Mobile Phase A*** 0.05% Trifluoroacetic acid in HPLC water
***Mobile Phase B*** 100% Acetonitrile
***Mobile phase gradient details*** T = 0 min (90% A, 10% B) flow : 1 mL/min; T = 7 min (10%A, 90% B) flow : 1 mL/min; gradient to T = 9 min (0% A, 100% B) flow : 1 mL/min; gradient to T = 14 min (0% A, 100% B) flow : 1 mL/min; T = 14.01 min (90% A, 10% B) flow : 1 mL/min; gradient to T= 17 min (90% A, 10% B) flow : 1 mL/min; end of run at T = 17 min (90% A, 10% B), Flow rate: 1 mL/min, Run Time: 17 min, UV Detection Method: PDA.

For compounds SLN5-X-0975, SLNS-X-0976, SLNS-X-1292, Compound H, and Compound D, HPLC was carried out using a Agilent Infinity II G6125C system with a Photodiode Array (PDA) detector.

Standard set-up for HPLC using a Agilent Infinity II G6125C system with a Photodiode Array (PDA) detector:
***Column details*** Sunfire C18 (150 x 4.6mm), 3.5µm
***Column temperature*** 25°C
***Auto sampler temperature*** 25°C
***Mobile Phase A*** 0.1% Formic acid in HPLC water
***Mobile Phase B*** 100% Acetonitrile
***Mobile phase gradient details*** T = 0 min (100% A, 00% B) T = 7 min (50%A, 50% B) T = 9 min (0% A, 100% B) T = 14 min (0% A, 100% B) T = 14.01 min (100% A, 00% B) gradient to T= 17 min (100% A, 00% B), Flowrate: 1 mL/min, Runtime: 17min, UV Detection Method: PDA.

### Chiral high-performance liquid chromatography (chiral HPLC)

For most compounds Chiral HPLC was carried out using a Waters SFC Investigator system with 2998 PDA detector.

Standard set-up for Chiral HPLC using a Waters SFC Investigator system with 2998 PDA detector:
***Column details*** CHIRALPAK IG (250 x 4.6mm), 5 µm
***Column temperature*** 40°C
***Auto sampler temperature*** 25°C
***Mobile Phase A*** Liq. CO2
***Mobile Phase B*** 0.1% Di-ethyl amine in 2-Propanol
***Mobile phase gradient details*** Isocratic Method (50% A, 50% B); Flow rate: 4 mL/min; Runtime:15 min; Back Pressure: 100 bar

For compounds Compound J, Compound H, Compound F, Compound C, Compound A and Compound G, an alternative standard setup was used for chiral HPLC using a Waters SFC Investigator system with 2998 PDA detector:
***Column details*** CHIRALPAK IG (250 x 4.6mm), 5 µm (Compound J, Compound H,
Compound C) or CHIRALPAK IB-N (250 x 4.6mm), 5mm (Compound F) or CHIRALPAK IH (250 x 4.6mm) 5mm (Compound A, Compound G)
***Column temperature*** 40°C
***Auto sampler temperature*** 25°C
***Mobile Phase A*** Liq. CO2
***Mobile Phase B*** 0.1 Methanolic Ammonia in Methanol:Acetonitrile(50:50) or 0.1% Diethylamine in Methanol:Acetonitrile(50:50) (Compound F)
***Mobile phase gradient*** T = 0 min (95% A, 5% B) T =5 min (50% A, 50% B) T = 12 min (50% A, 50% B); Runtime: 12 min; Back Pressure: 100 Bar
***Mobile phase gradient*** For Compound A and Compound G T = 0 min (95% A, 5% B) T =5 min (50% A, 50% B) T = 10 min (50% A, 50% B); Runtime: 10 min; Back Pressure: 100 Bar
***Mobile phase gradient*** For Compound J T = 0 min (60% A, 40% B) T =20 min (60% A, 40% B); Runtime: 20 min; Back Pressure: 100 Bar

For compound SLN5-X-0978 and Compound K Chiral HPLC was carried using a Waters Acquity UPC2 system with PDA detector with CHIRALPAK IG (100 x 3.0 mm), 3 µm.

Standard set up used for chiral HPLS using a Waters Acquity UPC2 system with PDA detector:
***Column temperature*** 50°C
***Auto sampler temperature*** 25°C
***Mobile Phase A*** Liq. CO2
***Mobile Phase B*** 0.1% Methanolic Ammonia in Methanol: Acetonitrile (50:50)
***Mobile phase gradient details for SLN5-X-0978*** T = 0 min (95% A, 5% B) T = 2 min (50%A, 50% B) gradient to T= 6 min (50% A, 50% B); Flow rate: 2 mL/min; Runtime: 6 min; Back Pressure: 1500 psi
***Mobile phase gradient details for Compound K*** Gradient 0 min> 5% Co-solvent 2 min> 50% Co-solvent 5 min> 50% Co-solvent; Flow rate: 2 mL/min; Runtime: 5 min.

### Synthesis of some non-commercially available materials

### Synthesis of ethyl 2-diazo-3-oxopropanoate

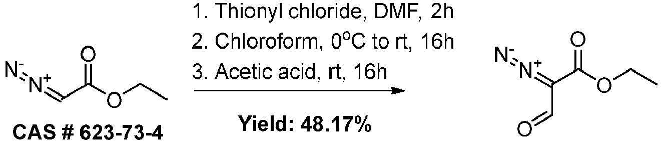

Thionyl chloride (46.0 mL, 0.46 V) was added dropwise to DMF (46.0 mL, 0.46 V) under nitrogen at rt. The reaction mixture was stirred for 2h. After 2h the reaction mixture was concentrated under reduced pressure to generate a highly moisture sensitive white solid which was kept under nitrogen. Chloroform (267 mL, 2.67 V) was added to the solid under nitrogen at rt with stirring. Ethyl 2-diazoacetate (100.0 g, 871.8 mmol) was added dropwise to the mixture at 0⁰C and the reaction mixture stirred at rt for 16 h. The reaction mixture was concentrated under reduced pressure. Acetic acid (267 mL, 2.67 V) was added at rt. The reaction mixture was stirred at rt for 16h. The progress of the reaction was monitored by TLC analysis. After completion of the reaction, the reaction mixture was diluted with sat. NaHCO₃ solution (1 Lit) and extracted with EtOAc (2 x 500 mL). The combined organic fractions were dried over Na₂SO₄ and concentrated under reduced pressure at 35°C to provide ethyl 2-diazo-3-oxopropanoate (60.0 g, 48.17% yield) as a yellow liquid.

**¹H NMR (400 MHz, CDCl₃):** δ 9.61 (s, 1H), 4.31-4.26 (q, *J*=12Hz, 2H), 1.29-1.25 (m, 3H).

### Synthesis of tert-butyl (S)-3-(isopropylamino)pyrrolidine-1-carboxylate

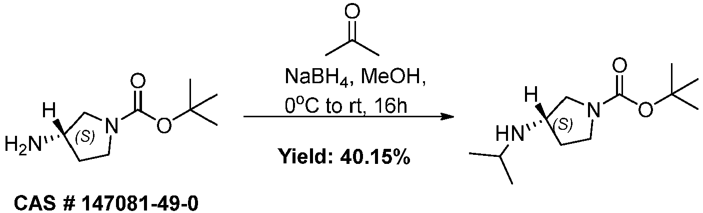

To a stirred solution of tert-butyl (S)-3-aminopyrrolidine-1-carboxylate (40.0 g, 214.0mmol) in MeOH (400mL, 10V) at rt, acetone (240.0 mL, 6V) was added. The reaction mixture was stirred at rt for 3h. Then NaBH₄ (12.2 g, 322.0 mmol) was added portion-wise at 0°C. The progress of the reaction was monitored by TLC analysis. After completion of the reaction, the reaction mixture was concentrated under reduced pressure. The crude material was dissolved in EtOAc (500 mL) and washed with Sat. NaHCO₃ solution (200 x 2 mL). The combined organic fractions were dried over Na₂SO₄ and concentrated under reduced pressure. The crude material was purified by column chromatography (3% MeOH in DCM) to provide tert-butyl (S)-3-(isopropylamino)pyrrolidine-1-carboxylate (2.5g, 40.15% yield) as a light yellow liquid. **¹H NMR (400 MHz, *d*₆-DMSO):** δ 3.42-3.28 (m, 3H), 3.19-3.13 (m, 1H), 2.91-2.86 (m, 1H), 2.78-2.72 (m, 1H), 1.96-1.89 (m, 1H), 1.61-1.53 (m, 1H), 1.39 (s, 9H), 0.98-0.96 (t, 3H)

### Synthesis tert-butyl (R)-3-(isopropylamino) pyrrolidine-1-carboxylate

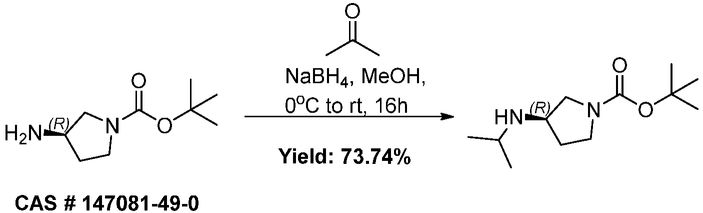

To a stirred solution of tert-butyl (R)-3-aminopyrrolidine-1-carboxylate (5 g, 26.88 mmol) was dissolved in MeOH (50mL, 10V) at room temperature. Then Acetone (11.9 mL, 161.29 mmol) was added into reaction mixture. Then reaction mixture was stirred at room temperature for 3 h. Sodium borohydride (1.52 g, 0.0040 mmol) was added portion-wise at 0 °C. The reaction was stirred at room temperature for 13 h. Progress of reaction was monitored by TLC and LCMS analysis, after completion of reaction, the reaction mixture was concentrated under reduced pressure and poured in water (150mL) and extracted with ethyl acetate (2 x 100mL). Both Organic layers combined dried over anhydrous sodium sulphate and evaporated u/vacuum to get Crude material, which was purified by combi flash column chromatography (product was eluted in 100% hexane using neutral silica) to get tert-butyl (R)-3-(isopropylamino) pyrrolidine-1-carboxylate (4.5g, 73.74% yield) as white sticky solid.

**¹H NMR (400 MHz, DMSO):** δ 3.41 - 3.25 (m, 3H), 3.18 - 3.13 (m, 1H), 2.89 - 2.85 (m, 1H), 2.74 - 2.71 (m, 1H), 1.93 - 1.90 (m, 1H), 1.59 - 1.53 (m, 2H), 1.39 (s, 9H), 0.96 (t, *J* = 6.0 Hz, 6H).

### General experimental procedures

### General experimental procedure 1 (GP1)

### Schematic overview of synthesis. Y = unspecified atom/combination of atoms; R¹, R², R³ = alkyl

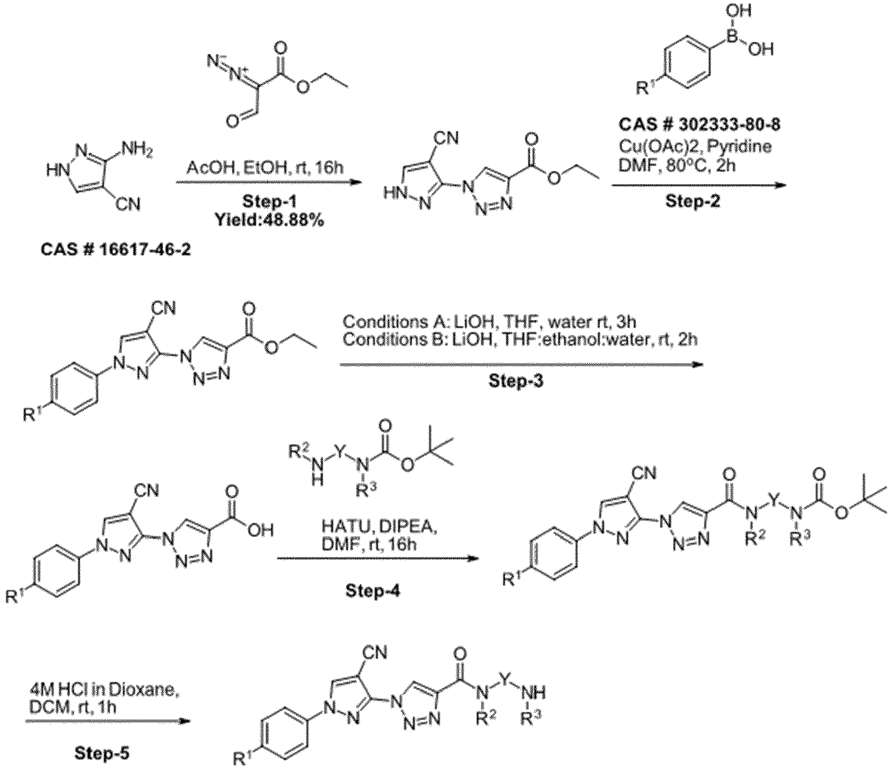

### Typical general conditions

Step-1: To a stirred solution of ethyl 2-diazo-3-oxopropanoate (10.51 g, 74.00 mmol) in EtOH (40 mL, 10V) at rt, acetic acid (4.0 mL, 1.0V) and 3-amino-1H-pyrazole-4-carbonitrile (4.0 g, 37.00 mmol) were added. The reaction mixture was stirred at rt for 16 h. The progress of the reaction was monitored by TLC analysis. After completion of the reaction, the reaction mixture was concentrated under reduced pressure. The crude material was purified by reverse phase column chromatography (product eluted at 38% CH₃CN in water) to provide ethyl 1-(4-cyano-1H-pyrazol-3-yl)-1H-1,2,3-triazole-4-carboxylate (4.0 g, 46.76 % yield) as a yellow fluffy solid.

**LCMS** [ESI, M+1]: 232.9 (RT: 1.187min, Purity: 100%),
Step-2: To a stirred solution of ethyl 1-(4-cyano-1H-pyrazol-3-yl)-1H-1,2,3-triazole-4-carboxylate (1 eq) and appropriate boronic acid (2 eq) in DMF (1.2mL per mmol boronic acid; 10 vol) was added Cu(OAc)₂ (2 eq) and pyridine (2 eq) at rt. The reaction mixture was stirred at 80°C for 2h. The progress of the reaction was monitored by TLC analysis. After completion of the reaction, the reaction mixture was poured into cold water (12 mL per mmol boronic acid) resulting in the formation of a solid precipitate. The solid material was filtered and dried under reduce pressure. The crude material was purified by normal phase column chromatography to yield ester **A.**

### Step-3: Conditions A

To a stirred solution of ester A (1 eq) in THF: Water (2:1 volume ratio, total volume of 5.6 mL per mmol ester A, 10 vol) at rt, LiOH (3 eq) was added, and the reaction mixture was stirred at rt for 3h. The progress of reaction was monitored by TLC analysis. After completion of the reaction, the reaction mixture was acidify using citric acid (pH= 4~5) and extracted with EtOAc (3 x 20 mL per mmol ester A). The combined organic fractions were dried over Na₂SO₄ and concentrated under reduced pressure to provide acid B.

### Conditions B

To a stirred solution of ester A (1.0 g, 3.24 mmol 1 eq) in THF: EtOH: Water (6:3:1 volume ratio, total volume of 3mL per mmol ester A, 10V) was added LiOH (3 eq) at rt. The reaction mixture was stirred at rt for 2h. The progress of the reaction was monitored by TLC analysis. After completion of the reaction, the reaction mixture was concentrated under reduced pressure at the temperature 35°C. The reaction mixture was diluted with water (5 mL per mmol ester A), acidified using citric acid solution (pH=~4-5) and extracted with EtOAc (3 x 10 mL per mmol ester A). The combined organic fractions were dried over Na₂SO₄ and concentrated under reduced pressure to provide acid B.

Step-4: To a stirred solution of acid B (0.2 g, 0.71 mmol 1 eq) in DMF (3 mL per mmol of acid B, 10V) at rt, HATU (1.5 eq) was added. The reaction mixture was stirred at rt for 30 minutes. Amine C (1.2 eq) and DIPEA (3 eq) were added. The reaction mixture was stirred at rt for 6h. The progress of the reaction was monitored by TLC and LCMS analysis. After completion of the reaction, the reaction mixture was diluted with cold water (70 mL pre mmol acid B) and extracted with Ethyl Acetate (3 x 70 mL per mmol of acid B). The combined organic fractions were washed with cold water (100 mL), dried over sodium sulphate and concentrated under reduced pressure. The crude material was purified by column chromatography to yield Boc-protected amide D.

Step-5: To a stirred of Boc-protected amide D (1 eq) in CH₂Cl₂ (5 mL per mmol of Boc-protected amide D) at 0°C, 4M HCl in Dioxane (1.5 mL per mmol of Boc-protected amide D, 3V) was added. The reaction mixture was stirred at rt for 2h. The progress of the reaction was monitored by TLC and LCMS analysis. After completion of the reaction, the reaction mixture was concentrated under reduced pressure and the crude material purified by trituration with diethyl ether to yield the final product.

### Examples of compounds synthesised using the general procedures

### N-[(S)-3-pyrrolidinyl]-N-isopropyl-1-[4-cyano-1-(p-cyclopropylphenyl)-3-pyrazolyl]-1H-1,2,3-triazole-4-carboxamide hydrochloride (Compound A)

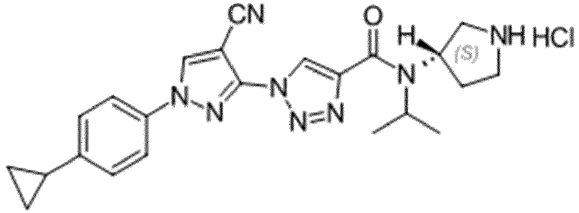

Prepared by GP1 using (4-cyclopropylphenyl) boronic acid (1.39 g, 8.61 mmol) in step 2, conditions A in step 3 and tert-butyl (S)-3-(isopropylamino) pyrrolidine-1-carboxylate (0.29 g, 1.31 mmol) in step 4. The title compound was isolated as a white solid (0.31 g, 76.42 % yield for final step).
**LCMS [ESI, M+1]:** 431.3 (RT: 1.459 min, Purity: 99.52%),
**HPLC Purity:** RT: 6.868min, Purity: 99.19%,
**Chiral HPLC Purity:** RT: 5.82min, Purity: 99.63%,
**¹H NMR (400 MHz, CD₃OD):** δ 9.14 (s, 1H) 8.99 (s, 1H), 7.76(d, *J* = 4.8 Hz, 2H), 7.29 (d, *J* = 4.8 Hz, 2H), 5.07-5.04 (m, 1H), 4.50-4.47 (m, 1H), 3.70 (t, *J* = 11.2 Hz, 1H), 3.52 (d, *J* = 9.2 Hz, 1H), 3.50-3.47 (m, 1H), 3.32-3.22 (m, 1H), 2.53-2.50 (m, 2H), 2.04-2.00 (m, 1H), 1.40-1.36 (m, 6H), 1.10-1.05 (m, 2H), 0.79-0.75 (m, 2H).

### N-isopropyl-N-4-piperidyl-1-[4-cyano-1-(p-cyclopropylphenyl)-3-pyrazolyl]-1H-1,2,3-triazole-4-carboxamide hydrochloride (Compound B)

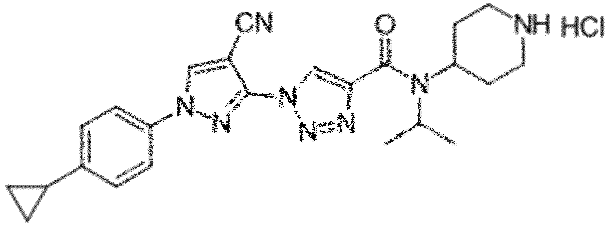

Prepared by GP1 using (4-cyclopropylphenyl) boronic acid (1.39 g, 8.61 mmol) in step-2, conditions A in step-3 and tert-butyl 4-(isopropylamino) piperidine-1-carboxylate (0.31 g, 1.31 mmol) in step-4. The title compound was isolated as a white solid (0.37 g, 90.67 % yield for final step).
**LCMS [ESI, M+1]:** 445.2 (RT: 1.441 min, Purity: 98.59%),
**HPLC Purity:** RT: 6.940min, Purity: 99.09%,
**¹H NMR (400 MHz, CD₃OD):** δ 9.14 (s, 1H) 8.91 (s, 1H), 7.76 (d, *J* = 8.8 Hz, 2H), 7.29 (d, *J* = 10 Hz, 2H), 4.78 (bs, 1H), 3.69 (bs, 1H), 3.52 (d, *J* = 11.6 Hz, 2H), 3.21-3.10 (m, 4H), 2.24 (bs, 1H), 2.04-2.00 (m, 1H), 1.99-1.92 (m, 1H) 1.57 (bs, 1H), 1.39-1.38 (m, 5H), 1.09-1.04 (m, 2H), 0.81-0.72 (m, 2H).

### N-[(S)-3-pyrrolidinyl]-N-isopropyl-1-(4-cyano-1-phenyl-3-pyrazolyl)-1H-1,2,3-triazole-4-carboxamide hydrochloride (Compound C)

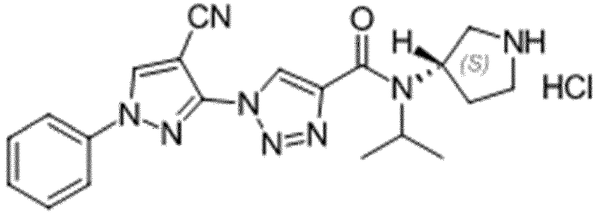

Prepared by GP1 using phenyl boronic acid (1.0 g, 8.61 mmol) in step-2, conditions B in step-3 and tert-butyl (S)-3-(isopropyl amino) pyrrolidine-1-carboxylate (0.19 g, 0.85mmol) ins tep-4. The title compound was isolated as an off-white solid (0.26 g, 85.51 % yield for final step).
**LCMS [ESI, M+1]:** 391.01 (RT: 1.291 min, Purity: 100%),
**HPLC Purity:** RT: 3.818 min, Purity: 100%,
**Chiral HPLC:** RT: 8.69 min, Purity: 100%.
**¹H NMR (400 MHz, CD₃OD)** δ 9.23 (s, 1H), 9.02 (s, 1H), 7.92 (d, *J* = 8.0 Hz, 2H), 7.61 (t, *J* = 8.4 Hz, 2H), 7.51 (t, J = 7.2 Hz, 1H), 5.06 (t, *J* = 9.2 Hz, 1H), 4.49 (s, 1H), 3.84 (s, 1H), 3.70 (d, *J* = 10.8 Hz, 1H), 3.51 (s, 1H), 3.32 (s, 1H), 2.43 - 2.53 (m, 2H), 1.36 - 1.40 (m, 6H).

### Full synthetic details for a representative set of compounds

### Synthesis of N-isopropyl-N-4-piperidyl-2-[1-(tert-butyl)-4-cyano-3-pyrazolyl]-1,3-thiazole-4-carboxamide hydrochloride (Compound D)

### Overall synthetic scheme

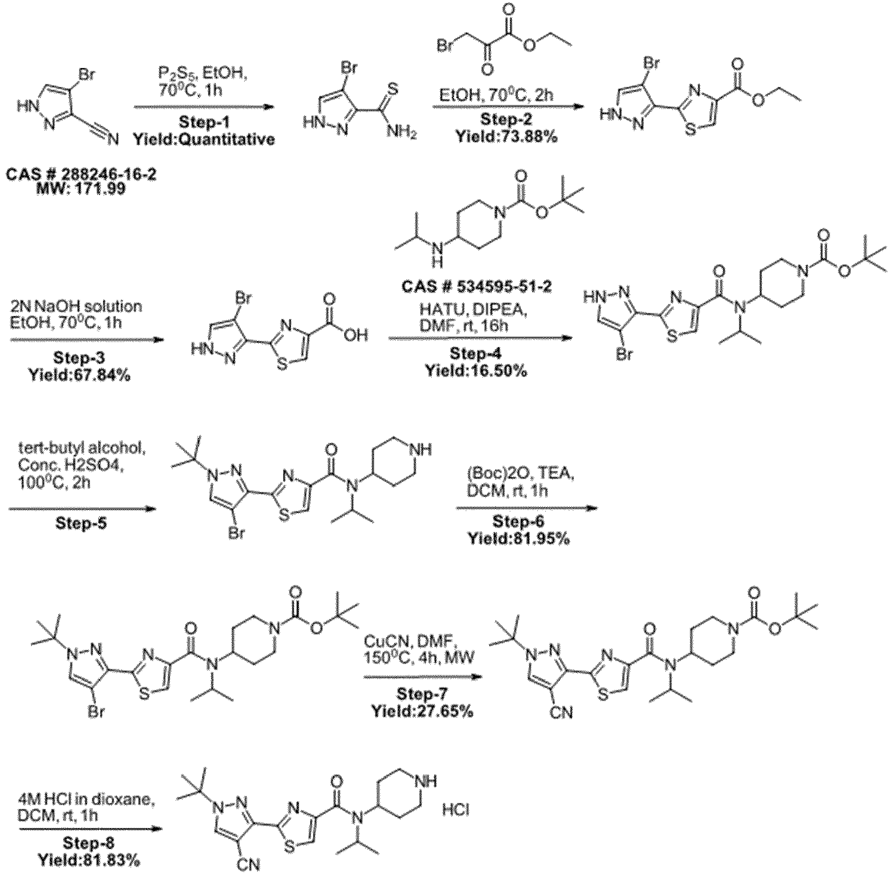

### Synthetic procedures

### Step-1: Synthesis of 4-bromo-1H-pyrazole-3-carbothioamide

To a stirred solution of 4-bromo-1H-pyrazole-3-carbonitrile (10.0 g, 58.4mmol) in EtOH (100mL, 10V) at 0⁰C, P₂S₅ (25.99g, 116.9mmol) was added. The reaction mixture was stirred at 70⁰C for 1h. The progress of the reaction was monitored by TLC and LCMS analysis. After completion of the reaction, the reaction mixture was concentrated under reduced pressure, poured into sat. NaHCO₃ solution (500mL) and extracted with EtOAc (2 x 500mL). The combined organic fractions were dried over Na₂SO₄ and concentrated under reduced pressure to provide 4-bromo-1H-pyrazole-3-carbothioamide (12.0 g, Quantitative yield) as a yellow solid, which was used directly in the next step of the synthesis without further purification.

**LCMS [ESI, M, M-2]:** 203.8, 205.9 (RT: 1.047 min, Purity: 94.82%).

### Step-2: Synthesis of ethyl 2-(4-bromo-1H-pyrazol-3-yl) thiazole-4-carboxylate

To a stirred solution of 4-bromo-1H-pyrazole-3-carbothioamide (12.0 g, 58.0 mmol) in EtOH (120mL) at rt, ethyl 3-bromo-2-oxopropanoate (13.63 g, 69.9 mmol) was added. The reaction mixture was heated at 70°C for 2 h. The progress of the reaction was monitored by TLC and LCMS analysis. After completion of the reaction, the reaction mixture was concentrated under reduced pressure, poured into water (700mL) and extracted with CH₂Cl₂ (2 x 300 mL). The combined organic fractions were dried over Na₂SO₄ and concentrated under reduced pressure. The crude material was purified by trituration using diethyl ether (2 x 100mL) to provide ethyl 2-(4-bromo-1H-pyrazol-3-yl) thiazole-4-carboxylate (13.0 g, 73.88 %) as an off-white solid.

**LCMS [ESI, M, M+2]:** 302.0, 304.1 (RT: 1.872min, Purity: 94.77%),

### Step-3: Synthesis of 2-(4-bromo-1H-pyrazol-3-yl) thiazole-4-carboxylic acid

To a stirred solution of ethyl 2-(4-bromo-1H-pyrazol-3-yl) thiazole-4-carboxylate (13.0 g, 43.0 mmol) in EtOH (13.0 mL, 10V) at rt, 2N NaOH solution (65.0 mL, 10V) was added. The reaction mixture was stirred at 70°C for 1h. The progress of the reaction was monitored by TLC and LCMS analysis. After completion of the reaction, the reaction mixture was cooled to rt and concentrated under reduced pressure. The crude material was dissolved in water (100 mL). The aqueous layer was separated and acidified using citric acid solution (pH=~4) to form a solid precipitate. The solid material was filtered and dried under reduced pressure to provide 2-(4-bromo-1H-pyrazol-3-yl) thiazole-4-carboxylic acid (8.0 g, 67.84 % yield) as an off-white solid.

**LCMS [ESI, M+1]:** 275.6 (RT: 1.165min, Purity: 100%).

**¹H NMR (400 MHz, *d*₆-DMSO):** δ 13.95 (s, 1H), 8.30 (s, 1H), 8.08 (s, 1H).

### Step-4: Synthesis of tert-butyl 4-(2-(4-bromo-1H-pyrazol-3-yl)-N-isopropylthiazole-4-carboxamido) piperidine-1-carboxylate

To a stirred solution of 2-(4-bromo-1H-pyrazol-3-yl) thiazole-4-carboxylic acid (0.5 g, 1.82 mmol) in DMF (5 mL) at rt, added HATU (1.03 g, 2.73 mmol) was added. After 30min, tert-butyl 4-(isopropyl amino) piperidine-1-carboxylate (0.44 g, 1.82 mmol) and DIPEA (0.93 mL, 5.46 mmol) were added at rt and the reaction mixture was stirred at rt for 16h. The progress of the reaction was monitored by TLC and LCMS analysis. After completion of the reaction, the reaction mixture was poured into ice cold water (500 mL) and extracted with EtOAc (3 x 400 mL). The combined organic fractions were dried over Na₂SO₄ and concentrated under reduced pressure. The crude material was purified by reverse phase chromatography (67% MeCN in water) to provide tert-butyl 4-(2-(4-bromo-1H-pyrazol-3-yl)-N-isopropylthiazole-4-carboxamido) piperidine-1-carboxylate (0.9 g, 16.50 % yield) as a light orange sticky solid.
**LCMS [ESI, M-55]:** 443.82 (RT: 1.770 min, Purity: 97.03 %),

### Step-5: Synthesis of 2-(4-bromo-1-(tert-butyl)-1H-pyrazol-3-yl)-N-Isopropyl-N-(piperidin-4-yl) thiazole-4-carboxamide

To a stirred solution of tert-butyl 4-(2-(4-bromo-1H-pyrazol-3-yl)-N-isopropylthiazole-4-carboxamido) piperidine-1-carboxylate (0.3 g, 0.60 mmol) in tert-butyl alcohol (10 V) at 0 °C, Conc. H₂SO₄ (0.06 g, 0.60 mmol) was added. The reaction mixture was stirred at 100 °C for 2 h. The progress of the reaction was monitored by TLC and LCMS analysis. After completion of the reaction, the reaction mixture was poured into sat. NaHCO₃ solution (200 mL) and extracted with CH₂Cl₂ (2 x 100mL). The combined organic fractions were dried over Na₂SO₄ and concentrated under reduced pressure to provide **2-**(4-bromo-1-(tert-butyl)-1H-pyrazol-3-yl)-N-Isopropyl-N-(piperidin-4-yl) thiazole-4-carboxamide as a colourless liquid.
**LCMS [ESI, M, M+2]:** 454.4, 456.4 (RT: 1.919 min, Purity: 77.42 %),

### Step-6: Synthesis of tert-butyl 4-(2-(4-bromo-1-(tert-butyl)-1H-pyrazol-3-yl)-N-isopropylthiazole-4-carboxamido) piperidine-1-carboxylate

To a stirred solution of 2-(4-bromo-1-(tert-butyl)-1H-pyrazol-3-yl)-N-Isopropyl-N-(piperidin-4-yl) thiazole-4-carboxamide (0.3 g, 0.66 mmol) in CH₂Cl₂ (3.0 mL, 10V) at 0°C, TEA (0.19 g, 1.98 mmol) and Di-tert-butyl dicarbonate (0.09 g, 0.79 mmol) were added. The reaction mixture was stirred at rt for 1h. The progress of the reaction was monitored by TLC and LCMS analysis. After completion of the reaction, the reaction mixture was poured into water (70 mL) and extracted with CH₂Cl₂ (2 x 80 mL). The combined organic fractions were dried over Na₂SO₄ and concentrated under reduced pressure to provide tert-butyl 4-(2-(4-bromo-1-(tert-butyl)-1H-pyrazol-3-yl)-N-isopropylthiazole-4-carboxamido) piperidine-1-carboxylate (0.3 g, 81.95% yield) as a light green solid.
**LCMS [ESI, M, M+2]:** 554.4, 556.5 (RT: 2.716 min, Purity: 95.93%),

### Step-7: Synthesis of tert-butyl 4-(2-(1-(tert-butyl)-4-cyano-1H-pyrazol-3-yl)-N-isopropylthiazole-4-carboxamido) piperidine-1-carboxylate

To a stirred solution of tert-butyl 4-(2-(4-bromo-1-(tert-butyl)-1H-pyrazol-3-yl)-N-isopropylthiazole-4-carboxamido)piperidine-1-carboxylate (0.3 g, 0.54 mmol) in DMF (3mL) at rt, CuCN (0.14 g, 1.62 mmol) was added. The reaction mixture was stirred at 150 °C for 4 h under microwave irradiation. The progress of the reaction was monitored by TLC and LCMS analysis. After completion of the reaction, the reaction mixture was poured into ice cold water (100 mL) and extracted with EtOAc (2 x 50 mL). The combined organic fractions were dried over Na₂SO₄ and concentrated under reduced pressure. The crude material was purified by normal phase chromatography (67% EtOAc in Hexane) to provide tert-butyl 4-(2-(1-(tert-butyl)-4-cyano-1H-pyrazol-3-yl)-N-isopropylthiazole-4-carboxamido) piperidine-1-carboxylate (0.075 g, 27.65 % yield) as an off-white solid.

**¹H NMR (400 MHz, CD₃OD):** δ 8.57 (s, 1H), 7.97 (d, *J* = 8.0 Hz, 1H), 4.55 (t, *J* = 13.2 Hz, 1H), 4.19-4.08 (m, 3H), 3.68-3.65 (m, 1H), 3.44-3.33 (m, 1H), 2.87-2.67 (m, 4H), 2.05 (d, *J* = 12 Hz, 1H), 1.82-1.79 (m, 1H), 1.67 (s, 9H), 1.59 (s, 2H), 1.59-1.52 (m, 9H), 1.49 (m, 3H).

### Step-8: Synthesis of 2-(1-(tert-butyl)-4-cyano-1H-pyrazol-3-yl)-N-Isopropyl-N-(piperidin-4-yl) thiazole-4-carboxamide hydrochloride (Compound D)

To a stirred solution of tert-butyl 4-(2-(1-(tert-butyl)-4-cyano-1H-pyrazol-3-yl)-N-isopropylthiazole-4-carboxamido) piperidine-1-carboxylate (0.07 g, 0.12 mmol) in CH₂Cl₂ (0.7 mL, 10V) at 0 °C, 4M HCl in Dioxane (0.35 mL, 5V) was added. The reaction mixture was stirred at rt for 1 h. The progress of the reaction was monitored by TLC. After completion of the reaction, the reaction mixture was concentrated under reduced pressure. The crude material was dissolved in water (5 mL) and washed with EtOAc (2 x 2 mL). The aqueous layer was isolated and lyophilized to provide 2-(1-(tert-butyl)-4-cyano-1H-pyrazol-3-yl)-N-Isopropyl-N-(piperidin-4-yl) thiazole-4-carboxamide hydrochloride (Compound D; 0.05 g, 81.83 %) as an off white solid.
**LCMS [ESI, M+1]:** 401.1 (RT: 1.332 min, Purity: 95.57 %),
**HPLC Purity:** RT: 7.091 min, Purity: 100 %
**¹H NMR (400 MHz, CD₃OD):** δ 8.58 (s, 1H), 8.08-8.01 (m, 1H), 4.61-4.46 (m, 1H), 3.66-3.61 (m, 1H), 3.57-3.42 (m, 2H), 3.20-3.02 (m, 3H), 2.35 (d, *J* = 12 Hz, 1H), 2.11-2.08 (m, 1H), 1.92 (d, *J* = 12.8 Hz, 1H), 1.65 (s, 9H), 1.59-1.53 (m, 2H), 1.35-1.29 (m, 4H).

### Synthesis of N-isopropyl-N-4-piperidyl-2-[4-cyano-1-(5-isopropyl-2-pyridyl)-3-pyrazolyl]-1,3-thiazole-4-carboxamide hydrochloride (Compound E)

### Overall synthetic scheme

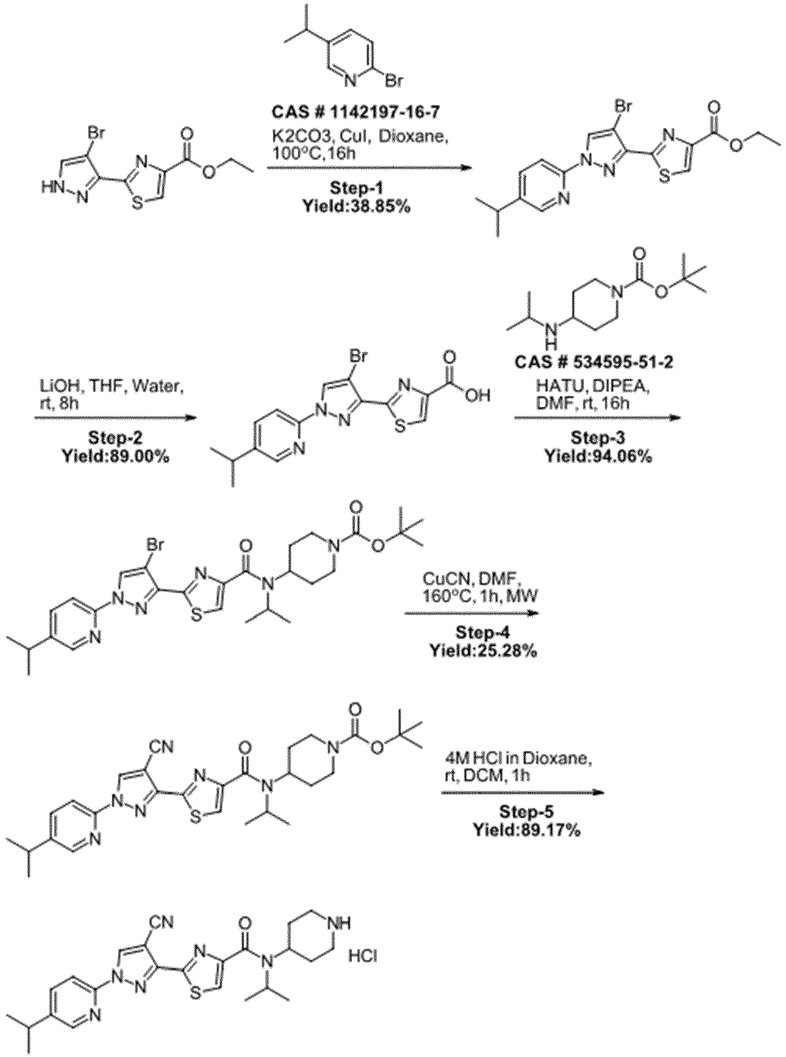

### Synthetic steps

### Step-1: Synthesis of ethyl 2-(4-bromo-1-(5-isopropylpyridin-2-yl)-1H-pyrazol-3-yl) thiazole-4-carboxylate

To a stirred solution of ethyl 2-(4-bromo-1H-pyrazol-3-yl) thiazole-4-carboxylate* (0.6 g, 1.98 mmol) in 1,4-Dioxane (9.0 mL, 15V) at rt, 2-bromo-5-isopropylpyridine (0.59 g, 2.97 mmol) and K₂CO₃ (0.82 g, 5.95 mmol) were added. The reaction vessel was purged with nitrogen gas for 15min, then CuI (0.037 g, 0.19 mmol) and 2-bromo-5-propan-2-ylpyridine (0.14 g, 0.99 mmol) were added at rt. The reaction mixture was heated at 100°C for 16 h. The progress of the reaction was monitored by TLC analysis. After completion of the reaction, the reaction mixture was diluted with EtOAc (50 mL) and filtered through Celite^{®}. Water (50 mL was added) and the mixture extracted with EtOAc (3 x 50 mL). The combined organic fractions were dried over Na₂SO₄ and concentrated under reduced pressure. The crude material was purified by normal phase column chromatography (10% EtOAc in hexane) to provide ethyl 2-(4-bromo-1-(5-isopropylpyridin-2-yl)-1H-pyrazol-3-yl)thiazole-4-carboxylate (0.65 g, 38.85 % yield) as an off white solid Note that two batches of this reaction were carried out in parallel (on the same scale as described here) and the product yield quoted here is based on the combination of these two batches.
**LCMS [ESI, M, M+2]:** 420.9, 422.8 (RT: 2.489 min, Purity: 100%),
*Note: ethyl 2-(4-bromo-1H-pyrazol-3-yl) thiazole-4-carboxylate was synthesized as described in the synthesis of N-isopropyl-N-4-piperidyl-2-[1-(tert-butyl)-4-cyano-3-pyrazolyl]-1,3-thiazole-4-carboxamide hydrochloride (Compound D)

### Step-2: Synthesis of 2-(4-bromo-1-(5-isopropylpyridin-2-yl)-1H-pyrazol-3-yl) thiazole-4-carboxylic acid

To a stirred solution of ethyl 2-(4-bromo-1-(5-isopropylpyridin-2-yl)-1H-pyrazol-3-yl) thiazole-4-carboxylate (0.325 g, 0.77 mmol) in THF:Water at rt, LiOH was added. Thew reaction mixture as stirred at rt for 8 h. The progress of the reaction was monitored by TLC analysis. After completion of the reaction, the reaction mixture was distilled out. Water (20 mL) was added, and the solution acidified with 1N HCl until pH=~3. The reaction mixture was extracted with 10% MeOH in CH₂Cl₂ (2 x 100mL). The combined organic fractions were dried over Na₂SO₄ and concentrated under reduced pressure to provide ethyl 2-(4-bromo-1-(5-isopropylpyridin-2-yl)-1H-pyrazol-3-yl) thiazole-4-carboxylate (0.54 g, 89.00% yield) as an off-white solid. Note that two batches of this reaction were carried out in parallel (on the same scale as described here) and the product yield quoted here is based on the combination of these two batches.

**LCMS [ESI, M, M+2]:** 392.8, 394.8 (RT: 1.981 min, Purity: 98.20 %).

### Step-3: Synthesis of tert-butyl 4-(2-(4-bromo-1-(5-isopropylpyridin-2-yl)-1H-pyrazol-3-yl)-N-isopropylthiazole-4-carboxamido) piperidine-1-carboxylate

To a stirred solution 2-(4-bromo-1-(5-isopropylpyridin-2-yl)-1H-pyrazol-3-yl) thiazole-4-carboxylic acid (0.22 g, 0.55 mmol) in DMF (20.0 mL) at 0 °C, HATU (0.42 g, 1.11 mmol) was added. After 20 min, DIPEA (0.28 g, 2.23 mmol), tert-butyl 4-(isopropylamino) piperidine-1-carboxylate (0.17 g, 0.72 mmol) were added at rt and the reaction mixture was stirred at rt for 16 h. The progress of the reaction was monitored by TLC and LCMS analysis. After completion of the reaction, the reaction mixture was poured into ice cold water (600 mL). The precipitate that formed was filtered out and dried under reduced pressure. The crude material was purified by normal phase chromatography (3% MeOH in CH₂Cl₂) to provide tert-butyl 4-(2-(4-bromo-1-(5-isopropylpyridin-2-yl)-1H-pyrazol-3-yl)-N-isopropylthiazole-4-carboxamido) piperidine-1-carboxylate (0.65 g, 94.06 % yield) as a brown solid.

Note that two batches of this reaction were carried out in parallel (on the same scale as described here) and the product yield quoted here is based on the combination of these two batches.
**LCMS [ESI, M-100]:** 517.0 (RT: 2.844 min, Purity: 95.57%),

### Step-4: Synthesis of tert-butyl 4-(2-(4-cyano-1-(5-isopropylpyridin-2-yl)-1H-pyrazol-3-yl)-N-isopropylthiazole-4-carboxamido) piperidine-1-carboxylate

To a stirred solution of tert-butyl 4-(2-(4-bromo-1-(5-isopropylpyridin-2-yl)-1H-pyrazol-3-yl)-N-isopropylthiazole-4-carboxamido) piperidine-1-carboxylate (0.32 g, 0.52 mmol) in DMF (3.2 mL, 10V) at rt, CuCN (0.14 g, 1.57 mmol) was added. The reaction mixture was heated at 160 °C for 1 h under microwave irradiation. The progress of the reaction was monitored by TLC analysis. After completion of the reaction, the reaction mixture was concentrated under reduced pressure. The crude material was purified by normal phase column chromatography (35% EtOAc in hexane) to provide tert-butyl 4-(2-(4-cyano-1-(5-isopropylpyridin-2-yl)-1H-pyrazol-3-yl)-N-isopropylthiazole-4-carboxamido) piperidine-1-carboxylate (0.15 g, 25.28 %) as a yellow oil. Note that two batches of this reaction were carried out in parallel (on the same scale as described here) and the product yield quoted here is based on the combination of these two batches.
**LCMS [ESI, M-100]:** 464.1 (RT: 2.445 min, Purity: 93.66 %)

### Step-5: Synthesis of 2-(4-cyano-1-(5-isopropylpyridin-2-yl)-1H-pyrazol-3-yl)-N-Isopropyl-N-(piperidin-4-yl) thiazole-4-carboxamide hydrochloride (Compound E)

To a stirred solution of tert-butyl 4-(2-(4-cyano-1-(5-isopropylpyridin-2-yl)-1H-pyrazol-3-yl)-N-isopropylthiazole-4-carboxamido) piperidine-1-carboxylate (0.15 g, 0.26 mmol) in CH₂Cl₂ (1.5 mL) at 0 °C, 4M HCl in Dioxane (0.75 mL) was added. The reaction mixture was stirred at rt for 1h. The progress of the reaction was monitored by TLC analysis. After completion of the reaction, the reaction mixture was concentrated under reduced pressure. The crude material was triturated with diethyl ether (20 mL), filtered and dried under reduced pressure to provide 2-(4-cyano-1-(5-isopropylpyridin-2-yl)-1H-pyrazol-3-yl)-N-isopropyl-N-(piperidin-4-yl) thiazole-4-carboxamide hydrochloride (Compound E; 0.11 g, 89.17% yield) as a white solid.
**LCMS [ESI, M+1]:** 464.1 (RT: 1.657 min, Purity: 98.69%),
**HPLC Purity:** RT: 5.907 min, Purity: 96.82%,
**¹H NMR (400 MHz, CD₃OD)** δ 9.34 (s, 1H), 8.44 (d, *J* = 1.6 Hz, 1H), 8.18-8.12 (m, 1H), 8.04 (d, *J* = 8.0 Hz, 1H), 7.99 (dd, *J* = 8.8, 2.0 Hz, 1H), 4.64-4.59 (m, 1H), 3.67-3.65 (m, 1H), 3.50-3.44 (m, 2H), 3.22-3.06 (m, 4H), 2.38 (d, *J* = 12.4 Hz, 1H), 2.11 (bs, 1H), 2.00-1.92 (m, 1H), 1.58 (d, *J* = 6.0 Hz, 2H), 1.62-1.58 (m, 2H), 1.37-1.19 (m, 10H).

### Synthesis of N-[(S)-3-pyrrolidinyl]-N-isopropyl-2-[4-cyano-1-(5-cyclopropyl-2-pyridyl)-3-pyrazolyl]-1,3-thiazole-4-carboxamide hydrochloride (Compound F)

### Overall synthetic scheme

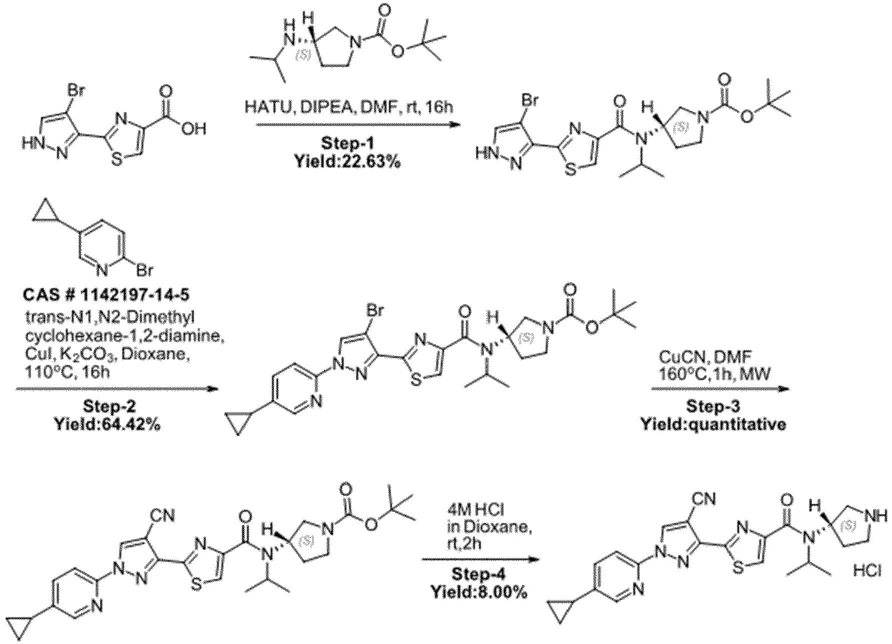

### Synthetic procedures

### Step-1: Synthesis of tert-butyl (S)-3-(2-(4-bromo-1H-pyrazol-3-yl)-N-isopropylthiazole-4-carboxamido) pyrrolidine-1-carboxylate

To a stirred solution of 2-(4-bromo-1H-pyrazol-3-yl)thiazole-4-carboxylic acid* (2.0 g, 7.29 mmol) in DMF (40 mL, 20V) at 0 °C, HATU (4.1 6 g, 10.9 mmol) was added. After 30 min, tert-butyl (S)-3-(isopropylamino)pyrrolidine-1-carboxylate (1.66 g, 7.29 mmol) and DIPEA (3.76 mL, 21.8 mmol) were added at 0 °C. The reaction mixture was stirred at rt for 16 h. The progress of the reaction was monitored by TLC analysis. After completion of the reaction, the reaction mixture was poured into ice-cold water (200 mL) and extracted with 10% MeOH in CH₂Cl₂ (3 x 100 mL). The combined organic fractions were dried over Na₂SO₄ and concentrated under reduced pressure. The crude material was purified by reverse phase column chromatography (54% CH₃CN in water) to provide tert-butyl (S)-3-(2-(4-bromo-1H-pyrazol-3-yl)-N-isopropylthiazole-4-carboxamido)pyrrolidine-1-carboxylate (0.8 g, 22.63 % yield) as a yellow solid. Note that two batches of this reaction were carried out in parallel (on the same scale as described here) and the product yield quoted here is based on the combination of these two batches.
**LCMS [ESI, M, M-2]:** 484.0, 482.1 (RT: 1.733 min, Purity: 95.25%)
*Note: 2-(4-bromo-1H-pyrazol-3-yl)thiazole-4-carboxylic acid was synthesized as described in the synthesis of N-isopropyl-N-4-piperidyl-2-[1-(tert-butyl)-4-cyano-3-pyrazolyl]-1,3-thiazole-4-carboxamide hydrochloride (Compound D)

### Step-2: Synthesis of tert-butyl (S)-3-(2-(4-bromo-1-(5-cyclopropylpyridin-2-yl)-1H-pyrazol-3-yl)-N-isopropylthiazole-4-carboxamido)pyrrolidine-1-carboxylate

To a stirred solution of tert-butyl (S)-3-(2-(4-bromo-1H-pyrazol-3-yl)-N-isopropylthiazole-4-carboxamido)pyrrolidine-1-carboxylate (0.25 g, 0.51 mmol) in dioxane (2.5 mL) at rt, 2-bromo-5-cyclopropylpyridine (0.2 g, 1.63 mmol), K₂CO₃ (0.21 g, 1.54 mmol) and CuI (0.049 g, 0.25 mmol) were added. The reaction mixture was purged with nitrogen gas for 10 min and trans-N,N'-Dimethylcyclohexane-1,2-diamine (0.007 g, 0.05 mmol) was added at rt. The reaction mixture was heated at 110 °C for 16 h. The progress of the reaction was monitored by TLC analysis. After completion of the reaction, the reaction mixture was diluted with water (200 mL) and extracted with EtOAc (2 x 200 mL). The combined organic fractions were dried over Na₂SO₄ and concentrated under reduced pressure. The crude material was purified by normal phase column chromatography (33% EtOAc in Hexane) to provide tert-butyl (S)-3-(2-(4-bromo-1-(5-cyclopropylpyridin-2-yl)-1H-pyrazol-3-yl)-N-isopropylthiazole-4-carboxamido) pyrroled ine-1-carboxylate (0.2 g, 21.47 %) as a white solid. Note that three batches of this reaction were carried out in parallel (on the same scale as described here) and the product yield quoted here is based on the combination of these three batches.
**LCMS [ESI, M-100]:** 502.78 (RT: 2.71 min, Purity: 90.43%),

### Step-3: Synthesis of tert-butyl (S)-3-(2-(4-cyano-1-(5-cyclopropylpyridin-2-yl)-1H-pyrazol-3-yl)-N-isopropylthiazole-4-carboxamido)pyrrolidine-1-carboxylate

To a stirred solution of tert-butyl (S)-3-(2-(4-bromo-1-(5-cyclopropylpyridin-2-yl)-1H-pyrazol-3-yl)-N-isopropylthiazole-4-carboxamido) pyrrolidine-1-carboxylate (0.1 g, 0.16 mmol) in DMF (1 mL, 10V), Copper Cyanide (0.0 44 g, 0.49 mmol) was added. The reaction mixture was stirred at 160 °C under microwave irradiation for 1 h. The progress of the reaction was monitored by TLC and LCMS analysis. After completion of the reaction, the reaction mixture was concentrated under reduced pressure to provide crude tert-butyl (S)-3-(2-(4-cyano-1-(5-cyclopropylpyridin-2-yl)-1H-pyrazol-3-yl)-N-isopropylthiazole-4-carboxamido) pyrrolidine-1-carboxylate (0.26 g, quantitative yield) as a light yellow liquid, which was used in the next step of the synthesis without any further purification.

**LCMS [ESI, M-100]:** 448.1 (RT: 2.398 min, Purity: 22.78%).

### Step-4: Synthesis of (S)-2-(4-cyano-1-(5-cyclopropylpyridin-2-yl)-1H-pyrazol-3-yl)-N-Isopropyl-N-(pyrrolidin-3-yl) thiazole-4-carboxamide hydrochloride (Compound F)

To a stirred solution tert-butyl (S)-3-(2-(4-cyano-1-(5-cyclopropylpyridin-2-yl)-1H-pyrazol-3-yl)-N-isopropylthiazole-4-carboxamido) pyrrolidine-1-carboxylate (0.26 g, 0.47 mmol) in CH₂Cl₂ (2.6 mL, 10V) at 0 °C, 4M HCl in Dioxane (1.3 mL) was added. The reaction mixture was stirred at rt for 2h. The progress of the reaction was monitored by TLC analysis. After completion of the reaction, the reaction mixture was concentrated under reduced pressure and the crude material purified by preparative HPLC to provide (S)-2-(4-cyano-1-(5-cyclopropylpyridin-2-yl)-1H-pyrazol-3-yl)-N-isopropyl-N-(pyrrolidin-3-yl)thiazole-4-carboxamide hydrochloride (Compound F; 0.017 g, 8.00 % yield) as a white solid.
**LCMS [ESI, M+1]:** 448.17 (RT: 1.57 min, Purity: 98.86%),
**Chiral HPLC Purity:** RT: 6.40 min, Purity: 97.92%,
**HPLC Purity:** RT: 4.47 min, Purity: 92.41%,
**¹H NMR (400 MHz, CD₃OD)** δ 9.33 (s, 1H), 8.37-8.36 (d, *J*=2.4 Hz, 1H), 8.29 (s,1H), 7.99-7.97 (d, *J*=8.4 Hz, 1H), 7.71-7.69, 7.71-7.68 (q, *J*=8.4, 8.4 Hz, 1H), 5.08-4.90 (m, 1H), 4.47-4.45 (m, 1H), 3.84-3.70 (m, 1H), 3.68-3.52 (m, 1H), 3.50-3.33 (m, 1H), 3.33-3.24(m, 2H), 2.52-2.49 (m, 2H), 2.09-2.05 (m, 1H), 1.39-1.28 (m, 6H), 1.16-1.11 (m, 2H), 0.86-0.82 (m, 2H).

### Synthesis of N-[(S)-3-pyrrolidinyl]-N-isopropyl-1-(1-phenyl-4-pyrazolyl)-1H-1,2,3-triazole-4-carboxamide hydrochloride (Compound G)

### Overall synthetic scheme

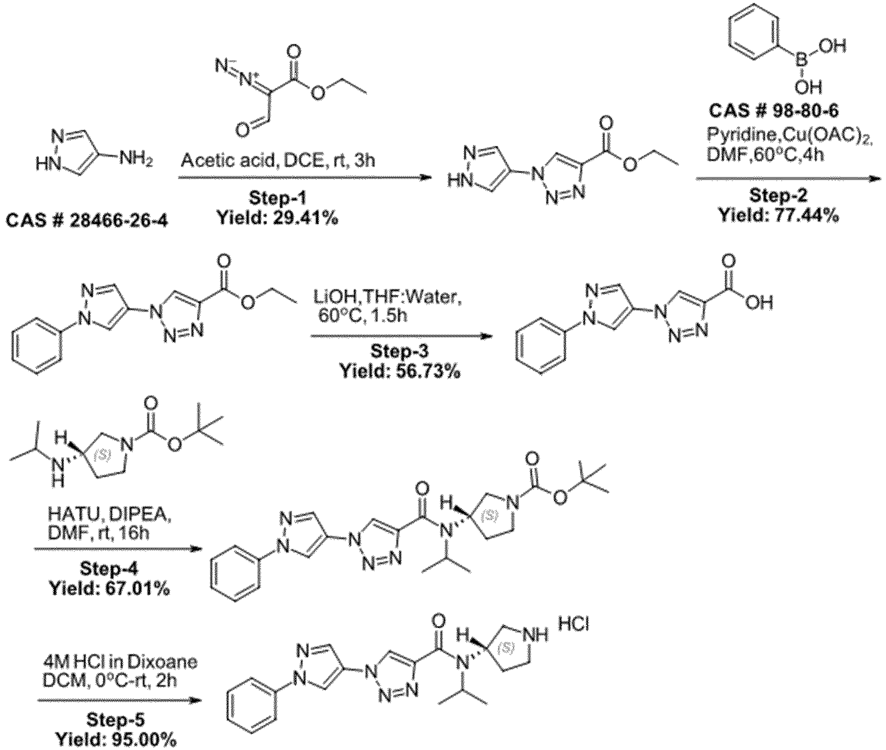

### Synthetic procedures

### Step-1: Synthesis of ethyl 1-(1H-pyrazol-4-yl)-1H-1,2,3-triazole-4-carboxylate

To a stirred solution of ethyl 2-diazo-3-oxopropanoate (30.0 g, 211.0 mmol) in DCE (450 mL, 15V) at rt, 1H-pyrazol-4-amine (15.43 g, 185.0 mmol) and acetic acid (30.0 mL, 1V) were added. The reaction mixture was stirred at rt for 3h. The progress of the reaction was monitored by TLC analysis. After completion of the reaction, the reaction mixture was concentrated under reduced pressure. Ice cold water (150 mL) was added to reaction mixture to form a solid precipitate, which was filtered and dried under reduced pressure to provide ethyl 1-(1H-pyrazol-4-yl)-1H-1,2,3-triazole-4-carboxylate (22.0 g, 29.41 % yield) as a purple solid.
**LCMS [ESI, M+1]:** 208.12 (RT: 1.51min, Purity: 100%),

### Step-2: Synthesis of ethyl 1-(1-phenyl-1H-pyrazol-4-yl)-1H-1,2,3-triazole-4-carboxylate

To a stirred solution of phenylboronic acid (8.5 g, 69.7mmol) in DMF (85.0 mL, 10V) at rt, pyridine (11.25 mL, 139.0 mmol), ethyl 1-(1H-pyrazol-4-yl)-1H-1,2,3-triazole-4-carboxylate (14.44 g, 69.7mmol) and Copper(II)acetate (27.83 g, 139.0 mmol) were added. The reaction mixture was heated at 60°C for 3h. The progress of the reaction was monitored by TLC. After completion of the reaction, the reaction mixture was cooled to rt, diluted with EtOAc (150 mL) and filtered through Celite^{®} using a Büchner funnel. The filtrate was concentrated under reduced pressure and the crude material purified by column chromatography (25% EtOAc in Hexane) to provide ethyl 1-(1-phenyl-1H-pyrazol-4-yl)-1H-1,2,3-triazole-4-carboxylate (9.0 g, 77.44 % yield) as a light brown solid.
**LCMS [ESI, M+1]:** 284.1 (RT:2.08 min, Purity: 97.73%)

### Step-3: Synthesis of 1-(1-phenyl-1H-pyrazol-4-yl)-1H-1,2,3-triazole-4-carboxylic acid

To a stirred solution of ethyl 1-(1-phenyl-1H-pyrazol-4-yl)-1H-1,2,3-triazole-4-carboxylate (9.0 g, 31.70 mmol) in THF:Water (72:18 mL, 8:2 V) at rt, LiOH (3.99g, 95.0 mmol) was added. The reaction mixture was stirred at 60°C for 1.5 h. The progress of the reaction was monitored by TLC analysis. After completion of the reaction, the reaction mixture was concentrated under reduced pressure. The residual mass was poured into water and acidified using 1N HCl acid solution until solid precipitate formed (pH of approx. 4.0). The precipitate was filtered and dried under reduced pressure to provide 1-(1-phenyl-1H-pyrazol-4-yl)-1H-1,2,3-triazole-4-carboxylic acid (4.6 g, 56.73 % yield) as off-white solid.

**¹H NMR (400 MHz, *d*₆-DMSO):** δ 13.36 (s, 1H), 9.29 (s, 1H), 9.24-9.23 (m, 1H), 8.40-8.39 (m, 1H), 7.91-7.89 (d, *J*=8.4 Hz, 2H), 7.59-7.55 (m, 1H), 7.42-7.39 (m, 1H). **LCMS [ESI, M+1]:** 256.20 (RT:1.78 min, Purity: 100%).

### Step-4: Synthesis of tert-butyl (S)-3-(N-isopropyl-1-(1-phenyl-1H-pyrazol-4-yl)-1H-1,2,3-triazole-4-carboxamido)pyrrolidine-1-carboxylate

To a stirred solution of 1-(1-phenyl-1H-pyrazol-4-yl)-1H-1,2,3-triazole-4-carboxylic acid (4.5 g, 17.6 mmol) in DMF (45 mL, 10V) at 0°C, HATU (10.07 g, 26.4 mmol) was added. The reaction mixture was stirred at 0 °C for 20 min. DIPEA (9.09 mL, 52.0 mmol) and tert-butyl (S)-3-(isopropylamino)pyrrolidine-1-carboxylate (4.83 g, 21.10 mmol) were added at 0°C and the reaction mixture was stirred at rt for 16h. The progress of the reaction was monitored by TLC analysis. After completion of the reaction, ice cold water (20mL) was added to the reaction mixture to form a solid precipitate, which was filtered and dried under reduced pressure to provide tert-butyl (S)-3-(N-isopropyl-1-(1-phenyl-1H-pyrazol-4-yl)-1H-1,2,3-triazole-4-carboxamido)pyrrolidine-1-carboxylate (5.5 g, 67.01 % yield) as off-white solid.

**LCMS [ESI, M-100]:** 366.1 (RT: 2.43 min, Purity: 86.61%).

### Step-6: Synthesis of (S)-N-isopropyl-1-(1-phenyl-1H-pyrazol-4-yl)-N-(pyrrolidin-3-yl)-1H-1,2,3-triazole-4-carboxamide hydrochloride (Compound G)

To a stirred solution of tert-butyl (S)-3-(N-isopropyl-1-(1-phenyl-1H-pyrazol-4-yl)-1H-1,2,3-triazole-4-carboxamido)pyrrolidine-1-carboxylate (5.5 g, 11.8 mmol) in CH₂Cl₂ (55 mL, 10V) at 0 °C, 4M HCl in Dioxane (27.5mL, 5V) was added. The reaction mixture was stirred at rt for 2 h. The progress of the reaction was monitored by TLC analysis. After completion of the reaction, the reaction mixture was concentrated under reduced pressure and the crude material triturated using diethyl ether (3 x 30 mL) to provide (S)-N-isopropyl-1-(1-phenyl-1H-pyrazol-4-yl)-N-(pyrrolidin-3-yl)-1H-1,2,3-triazole-4-carboxamide hydrochloride (Compound G; 4.5 g, 95.00 % yield) as an off-white solid.
**LCMS [ESI, M-100]:** 366.30 (RT: 1.67min, Purity: 96.93%)
**HPLC Purity:** (RT: 5.93min, Purity: 95.02%)
**Chiral HPLC Purity:** (RT: 4.87min, Purity: 96.05%)
**¹H NMR (400 MHz, *d*₆-DMSO):** δ 9.30 (s, 1H), 9.02 (s, 1H), 8.39 (s, 1H), 7.92-7.90 (d, J = 8.0 Hz, 2H), 7.58 (t, 2H), 7.43-7.41 (m, 1H), 4.87 (bs, 1H), 4.33 (bs, 1H), 3.60 (bs, 2H), 3.47 (bs, 1H), 3.16-3.09 (m, 1H), 2.27-2.26 (m, 1H), 1.27 (s, 6H).

### Synthesis of N-[(S)-3-pyrrolidinyl]-N-isopropyl-2-[1-(tert-butyl)-4-pyrazolyl]-1,3-thiazole-4-carboxamide hydrochloride (Compound H)

### Overall synthetic scheme

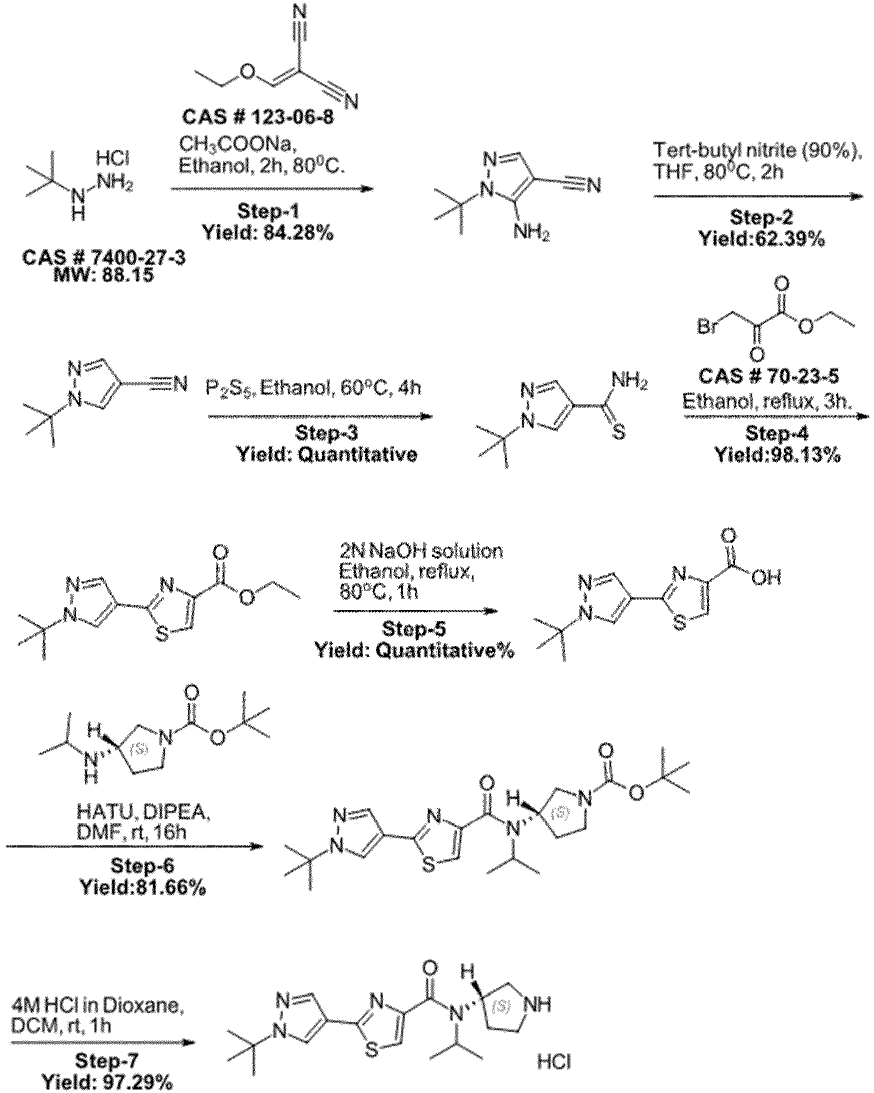

### Synthetic procedures

### Step-1: Synthesis of 5-amino-1-(tert-butyl)-1H-pyrazole-4-carbonitrile

To a stirred solution of tert-Butylhydrazine hydrochloride (10 g, 8.02 mmol) in Ethanol (100 mL) at rt, sodium acetate (13.15 g, 16.04 mmol) and 2-(ethoxymethylene)malononitrile (10.7 g, 8.82 mmol) were added. The reaction mixture was refluxed at 800C for 2h. The progress of the reaction was monitored by TLC and LCMS analysis. After completion of the reaction, the reaction mixture was concentrated under reduced pressure. The crude material was poured into ice cold water to form a solid precipitate. The precipitate was filtered, washed with diethyl ether (2 x 50 mL) and dried under reduced pressure to provide 5-amino-1-(tert-butyl)-1H-pyrazole-4-carbonitrile (15.7 g, 84.28 % yield) as a yellow solid.
**LCMS [ESI, M+1]:** 164.9 (RT: 1.22 min, Purity: 97.46%)

### Step-2: Synthesis of 1-(tert-butyl)-1H-pyrazole-4-carbonitrile

To a stirred solution of 5-amino-1-(tert-butyl)-1H-pyrazole-4-carbonitrile (15.7 g, 95.73 mmol) in THF (157mL, 10V) at rt, tert-butyl nitrite (90 %) (23 mL, 191.46 mmol) was added. The reaction mixture was refluxed at 80 °C for 2 h. The progress of the reaction was monitored by TLC and LCMS analysis. After completion of the reaction, the reaction mixture was concentrated under reduced pressure. The crude material was poured into ice cold water to form a solid precipitate. The solid was filtered out, washed with diethyl ether (100 mL) and dried under reduced pressure to provide 1-(tert-butyl)-1H-pyrazole-4-carbonitrile (8.9 g, 62.39 % yield) as yellow solid.
**LCMS [ESI, M-1]:** 149.9 (RT: 1.500 min, Purity: 90.27 %)

### Step-3: Synthesis of 1-(tert-butyl)-1H-pyrazole-4-carbothioamide

To a stirred solution of 1-(tert-butyl)-1H-pyrazole-4-carbonitrile (8.9g, 59.73mmol) in ethanol (89mL, 10V) at rt, P₂S₅ (26.55 g, 119.46 mmol) was added. The reaction mixture was refluxed at 60 °C for 4 h. The progress of the reaction was monitored by TLC and LCMS analysis. After completion of the reaction, the reaction mixture was distilled under vacuum. The crude reaction mixture was washed with sodium bicarbonate solution (200 mL) until neutralization of the pH and then extracted with ethyl acetate (3 x 200 mL). The combined organic fractions were dried over sodium sulphate, concentrated under reduced pressure, and triturated with diethyl ether to provide 1-(tert-butyl)-1H-pyrazole-4-carbothioamide (11.9 g, Quantitative% yield) as a yellow solid.
**LCMS [ESI, M+1]:** 183.9 (RT: 1.226 min, Purity: 98.30 %)

### Step-4: Synthesis of ethyl 2-(1-(tert-butyl)-1H-pyrazol-4-yl)thiazole-4-carboxylate

To a stirred solution of 1-(tert-butyl)-1H-pyrazole-4-carbothioamide (11.9 g, 64.93 mmol) in ethanol (119 mL) at rt, ethyl 3-bromo-2-oxopropanoate (13.92 g, 71.42 mmol) was added. The reaction mixture was refluxed at 60 °C for 1 h. The progress of the reaction was monitored by TLC and LCMS analysis. After completion of the reaction, the reaction mixture was cooled to 0 °C which led to the formation of a solid precipitate. The precipitate was filtered, dried under reduced pressure and triturated with diethyl ether (2 x 75 mL) to provide ethyl 2-(1-(tert-butyl)-1H-pyrazol-4-yl)thiazole-4-carboxylate (17.8 g, 98.13% yield) as a yellow solid.
**LCMS [ESI, M+1]:** 279.8 (RT: 1.794 min, Purity: 85.27 %)

### Step-5: Synthesis of 2-(1-(tert-butyl)-1H-pyrazol-4-yl)thiazole-4-carboxylic

To a stirred solution of ethyl 2-(1-(tert-butyl)-1H-pyrazol-4-yl)thiazole-4-carboxylate (17.8 g, 63.71 mmol) in ethanol (178 mL, 10V) at rt, 2 N NaOH solution (178 mL, 10V) was added. The reaction mixture was heated at 80 °C for 1 h. The progress of the reaction was monitored by TLC and LCMS analysis. After completion of the reaction, the reaction mixture was distilled under vacuum. The reaction mixture was poured into water (150 mL) and washed with ethyl acetate (2 x 70 mL). The aqueous layer was separated and acidified with 2M HCl solution until a pH of approximately 2-3 was reached. A solid precipitate was formed which was filtered out and dried under reduced pressure to provide 2-(1-(tert-butyl)-1H-pyrazol-4-yl)thiazole-4-carboxylic acid (21.3 g, Quantitative yield) as a brown solid.
**LCMS [ESI, M+1]:** 251.8 (RT: 1.352min, Purity: 95.40%)

### Step-6: Synthesis of tert-butyl (S)-3-(2-(1-(tert-butyl)-1H-pyrazol-4-yl)-N-isopropylthiazole-4-carboxamido) pyrrolidine-1-carboxylate

To a stirred solution of 2-(1-(tert-butyl)-1H-pyrazol-4-yl) thiazole-4-carboxylic acid (1.0 g, 3.98 mmol) in DMF (10 mL, 10V) at rt, HATU (2.26 g, 5.97 mmol) was added. After 1h, tert-butyl (S)-3-(isopropyl amino) pyrrolidine-1-carboxylate (1.0 g, 4.77 mmol) and DIPEA (2.0 mL, 11.94 mmol) were added at rt. The reaction mixture was stirred at rt for 16h. The progress of the reaction was monitored by TLC and LCMS analysis. After completion of the reaction, the reaction mixture was poured into cold water (200 mL) and extracted with EtOAc (2 x 200 mL). The combined organic fractions were washed with cold water (3 x 20 mL), dried over Na₂SO₄, and concentrated under reduced pressure. The crude material was purified by column chromatography (18% EtOAc in hexane) to provide tert-butyl (S)-3-(2-(1-(tert-butyl)-1H-pyrazol-4-yl)-N-isopropylthiazole-4-carboxamido) pyrrolidine-1-carboxylate (1.5 g, 81.66 % yield) as a pale yellow sticky solid.
**LCMS [ESI, M-56]:** 406 (RT: 2.249 min, Purity: 100%)

### Step-7: Synthesis of (S)-2-(1-(tert-butyl)-1H-pyrazol-4-yl)-N-Isopropyl-N-(pyrrolidin-3-yl) thiazole-4-carboxamide hydrochloride (Compound H):

To a stirred solution of tert-butyl (S)-3-(2-(1-(tert-butyl)-1H-pyrazol-4-yl)-N-isopropylthiazole-4-carboxamido) pyrrolidine-1-carboxylate (1.5 g, 3.25 mmol) in CH₂Cl₂ (15 mL, 10V) at 0 °C, 4M HCl in Dioxane (7.5 mL, 5V) was added. The reaction mixture was stirred at rt for 1h. The progress of the reaction was monitored by TLC and LCMS analysis. After completion of the reaction, the reaction mixture was concentrated under reduced pressure. The crude material was purified by trituration using diethyl ether (40 mL) and pentane (20 mL) to provide (S)-2-(1-(tert-butyl)-1H-pyrazol-4-yl)-N-Isopropyl-N-(pyrrolidin-3-yl) thiazole-4-carboxamide hydrochloride (Compound H; 1.3 g, 97.29 %) as a light orange solid.
**LCMS [ESI, M+1]:** 362.1 (RT: 1.208 min, Purity: 97.87%)
**HPLC Purity:** RT: 5.135min, Purity: 97.56%
**CHIRAL HPLC Purity:** RT: 6.66min, Purity: 98.42%
**¹H NMR (400 MHz, CD₃OD):** δ 8.43 (s, 1H), 8.06 (s, 1H), 7.90 (s, 1H), 4.46-4.38 (m, 2H), 3.84 (t, *J*=9.2 Hz, 1H), 3.68 (d, *J*=11.1 Hz, 1H), 3.54-3.49 (m, 1H), 3.33-3.26 (m, 1H), 2.53-2.49 (m, 1H), 2.47-2.40 (m, 1H), 1.66 (s, 9H), 1.34-1.30 (m, 6H).

### Synthesis of N-(3,3-difluoro-4-piperidyl)-N-isopropyl-2-(1-phenyl-4-pyrazolyl)-1,3-thiazole-4-carboxamide hydrochloride (Compound I)

### Overall synthetic scheme

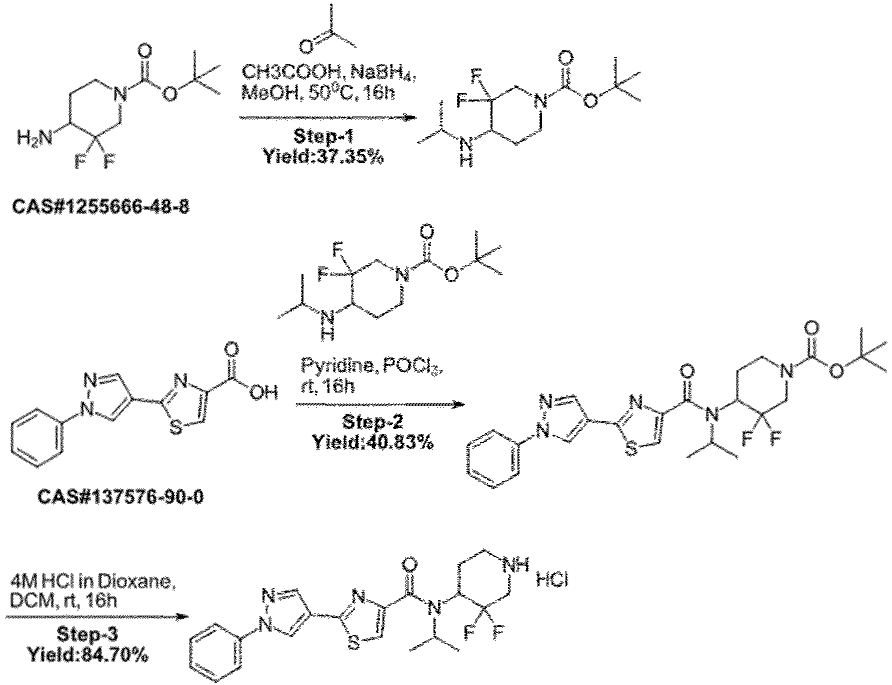

### Synthetic procedures

### Step-1: Synthesis of tert-butyl 3,3-difluoro-4-(isopropylamino)piperidine-1-carboxylate

To a stirred solution of tert-butyl 4-amino-3,3-difluoropiperidine-1-carboxylate (0.5 g, 2.11 mmol) in MeOH (5 mL, 10V) at rt, acetone (1.2 g, 21.1 mmol) and glacial acetic acid (0.3 g,5.29 mmol) were added. The reaction mixture was stirred at 50 °C for 1h. Then NaBH₄ (0.39 g, 10.59 mmol) was added portion wise at 0 °C and the reaction was heated at 50 °C for 16 h. The progress of reaction was monitored by TLC and LCMS analysis. After completion of reaction, the reaction mixture was concentrated under reduced pressure. Water (70mL) as added, and the mixture extracted with EtOAc (3 x 50 mL). The combined organic fractions were dried over Na₂SO₄ and concentrated under reduced pressure. The crude material was purified by flash column chromatography (1% in EtOAc in hexane) to provide tert-butyl 3,3-difluoro-4-(isopropylamino)piperidine-1-carboxylate (0.22 g, 37.35% yield) as a white sticky liquid.

**LCMS [ESI, M+1]:** 279.04 (RT: 0.99 min, Purity: 100%),
**¹H NMR (400 MHz, CD₃OD)** δ 3.91 (bs, 1H), 3.69 (d, *J*=13.2 Hz, 1H), 3.31-3.19 (m, 1H), 3.03-2.99 (m, 2H), 2.94-2.88 (m, 1H), 1.82-1.77 (m, 1H), 1.52 (d, *J*=9.2 Hz, 1H), 1.39 (s, 9H), 0.99 (d, *J*=6.4 Hz, 6H).

### Step-2: Synthesis of tert-butyl 3,3-difluoro-4-(N-isopropyl-2-(1-phenyl-1H-pyrazol-4-yl)thiazole-4-carboxamido)piperidine-1-carboxylate

To a stirred solution of 2-(1-phenyl-1H-pyrazol-4-yl)thiazole-4-carboxylic acid (0.2 g, 0.73 mmol) in pyridine (2.0mL, 10V) at rt, tert-butyl 3,3-difluoro-4-(isopropylamino)piperidine-1-carboxylate (0.24 g, 0.88 mmol) was added. After 15 min, POCl₃ (1.6 g, 11.07 mmol) was added at 0°C and the resulting reaction was stirred at rt for 16 h. The progress of the reaction was monitored by TLC and LCMS analysis. After completion of the reaction, then reaction mixture was quenched with sat. citric acid solution (30mL) and extracted with CH₂Cl₂ (2 x 30 mL). The combined organic fractions were dried over Na₂SO₄ and concentrated under reduced pressure. The crude material was purified by column chromatography (29% EtOAc in hexane) to provide tert-butyl 3,3-difluoro-4-(N-isopropyl-2-(1-phenyl-1H-pyrazol-4-yl)thiazole-4-carboxamido)piperidine-1-carboxylate (0.16 g, 40.83 % yield) as an yellowish solid. **LCMS [ESI, M-56]: 476** (RT: 2.571min, Purity: 99.39%),

### Step-3: Synthesis of N-(3,3-difluoropiperidin-4-yl)-N-isopropyl-2-(1-phenyl-1H-pyrazol-4-yl)thiazole-4-carboxamide hydrochloride (Compound I)

To a stirred solution of tert-butyl 3,3-difluoro-4-(N-isopropyl-2-(1-phenyl-1H-pyrazol-4-yl) thiazole-4-carboxamido) piperidine-1-carboxylate (0.16 g, 0.3 mmol) in CH₂Cl₂ (1.6 mL, 10V) at 0 °C, 4M HCl in Dioxane (0.8 mL, 5V) was added. The reaction mixture was stirred at rt for 16 h. After completion of the reaction, the reaction mixture was concentrated under reduced pressure. The crude material was purified by trituration using diethyl ether to provide N-(3,3-difluoropiperidin-4-yl)-N-isopropyl-2-(1-phenyl-1H-pyrazol-4-yl) thiazole-4-carboxamide hydrochloride (0.11 g, 84.70 % yield) as an off-white solid.
**LCMS [ESI, M+1]:** 432.0 (RT: 1.408 min, Purity: 95.50%),
**HPLC Purity:** RT: 5.588min, Purity: 95.43%,
**¹H NMR (400 MHz, CD₃OD)** δ 8.92 (s, 1H), 8.25 (s, 1H), 7.93 (s, 1H), 7.87 (dd, *J*=8.8, 4.4 Hz, 2H), 7.57 (t, *J*=3.8 Hz, 2H), 7.41 (t, *J*=7.4 Hz, 1H), 4.5-4.12 (m, 1H), 3.88-3.73 (m, 2H), 3.64 (d, *J*=12.8 Hz, 1H), 3.60-3.45 (m, 1H), 3.26-3.24 (m, 1H), 2.55 (bs, 2H), 1.57 (d, *J*=18.6, 6H).

### Synthesis of N-(3-fluoro-4-piperidyl)-N-isopropyl-2-(1-phenyl-4-pyrazolyl)-1,3-thiazole-4-carboxamide hydrochloride (Compound J)

### Overall synthetic scheme

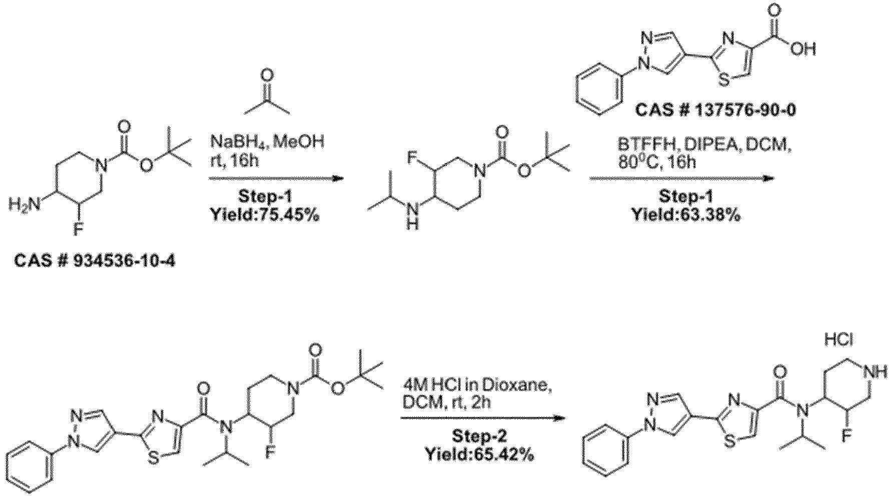

### Synthetic procedures

### Step-1: Synthesis of tert-butyl 3-fluoro-4-(isopropylamino) piperidine-1-carboxylate

To a stirred solution of tert-butyl 4-amino-3-fluoropiperidine-1-carboxylate (0.5 g, 2.29 mmol) in MeOH (5 mL, 10V) at rt, acetone (3 mL, 6V) was added. After 1 h, sodium borohydride (0.12 g, 3.44 mmol) was added portion wise at 0 °C. The reaction was stirred rt for 16 h. The progress of the reaction was monitored by TLC and LCMS analysis. After completion of the reaction, the reaction mixture was concentrated under reduced. The residue was poured into water (200 mL) and extracted with EtOAc (2 x 300 mL). The combined organic fractions were dried over Na₂SO₄ and concentrated under reduced pressure. The crude material was purified by column chromatography (100% hexane) to provide tert-butyl 3-fluoro-4-(isopropylamino) piperidine-1-carboxylate (1.8 g, 75.45 % yield) as a yellow sticky liquid. Note that four batches of this reaction were carried out in parallel (on the same scale as described here) and the product yield quoted here is based on the combination of all these four batches.
**LCMS [ESI, M+1]:** 260.9 (RT: 0.889 min, Purity: 41.00%), (RT: 0.952min, Purity: 47.52%)

### Step-2: Synthesis of tert-butyl 3-fluoro-4-(N-isopropyl-2-(1-phenyl-1H-pyrazol-4-yl)thiazole-4-carboxamido)piperidine-1-carboxylate

To a stirred solution of 2-(1-phenyl-1H-pyrazol-4-yl) thiazole-4-carboxylic acid (0.5 g, 1.84 mmol) in CH₂Cl₂ (5mL, 10V) at rt, BTFFH (2.9g, 9.22mmol) and DIPEA (1.7 mL, 9.22 mmol) were added. After 1h, tert-butyl 3-fluoro-4-(isopropylamino) piperidine-1-carboxylate (0.47 g, 1.84 mmol) was added at rt and the reaction was stirred at 80⁰C for 16h. The progress of the reaction was monitored by TLC and LCMS analysis. After completion of the reaction, the reaction mixture was poured in water (200mL) and extracted with CH₂Cl₂ (2 x 200 mL). The combined organic fractions were dried over Na₂SO₄ and concentrated under reduced pressure. The crude material was purified by flash column chromatography (25% EtOAc in Hexane) to provide tert-butyl 3-fluoro-4-(N-isopropyl-2-(1-phenyl-1H-pyrazol-4-yl)thiazole-4-carboxamido)piperidine-1-carboxylate (1.8 g, 63.38 % yield) as a white sticky solid. Note that three batches of this reaction were carried out in parallel (on the same scale as described here) and the product yield quoted here is based on the combination of all these three batches. **LCMS [ESI, M-56]:** 458.0 (RT: 2.416min, Purity: 19.09%), (RT: 2.435min, Purity: 75.27%)

### Step-3: Synthesis of N-(3-fluoropiperidin-4-yl)-N-isopropyl-2-(1-phenyl-1H-pyrazol-4-yl)thiazole-4-carboxamide hydrochloride (Compound J)

To a stirred solution of tert-butyl 3-fluoro-4-(N-isopropyl-2-(1-phenyl-1H-pyrazol-4-yl)thiazole-4-carboxamido)piperidine-1-carboxylate (1.5 g, 2.92 mmol) in CH₂Cl₂ (15 mL, 10V) at 0 °C, 4M HCl in Dioxane (0.75 mL, 5V) was added. The reaction mixture was stirred at rt for 2h. After completion of the reaction, the reaction mixture was concentrated under reduced pressure. The crude material triturated with diethyl ether then into water (150 mL) and extracted with EtOAc (3 x 150 mL). The aqueous layer was lyophilized to provide N-(3-fluoropiperidin-4-yl)-N-isopropyl-2-(1-phenyl-1H-pyrazol-4-yl) thiazole-4-carboxamide hydrochloride (Compound J; 0.75 g, 65.42 % yield) as a white solid, obtained as a stereoisomeric mixture of all possible stereoisomers.
**LCMS [ESI, M+1]:** 414.0 (**1.** RT: 1.330 min, Purity: 24.13%; **2.** RT: 1.453 min, Purity: 75.87%),
**HPLC Purity: 1.** RT: 4.511min, Purity: 22.43%, **2.** RT: 4.789 min, Purity: 77.57%, **Chiral HPLC Purity: 1.** RT: 05.51min, Purity: 11.36%; **2.** RT: 09.71min, Purity: 38.37%; **3.** RT: 10.86min, Purity: 06.62%; **4.** RT: 11.44min, Purity: 42.68%,
**¹H NMR (400 MHz, CD₃OD):** δ 8.91-8-88 (m, 1H), 8.22 (s, 1H), 7.87-7.84 (m, 3H), 7.55 (t, *J*=7.6 Hz, 2H), 7.41 (t, *J*=7.2 Hz, 1H), 5.91-5.79 (m, 1H), 4.39-4.36 (m, 1H), 3.79 (t, *J*=11.2 Hz, 2H), 3.50-3.49 (m, 1H), 3.15 (t, *J*=1.6 Hz, 2H), 3.04-3.01 (m, 1H), 2.10-2.07 (m, 1H), 1.57 (dd, *J*=6.4, 6.5 Hz, 2H), 1.33 (t, *J*=12.5, 6.4 Hz, 4H)

### Synthesis of N-[(S)-3-pyrrolidinyl]-N-isopropyl-2-(2-phenyl-1,3-oxazol-4-yl)-4-imidazolecarboxamide hydrochloride (Compound K)

### Overall synthetic scheme

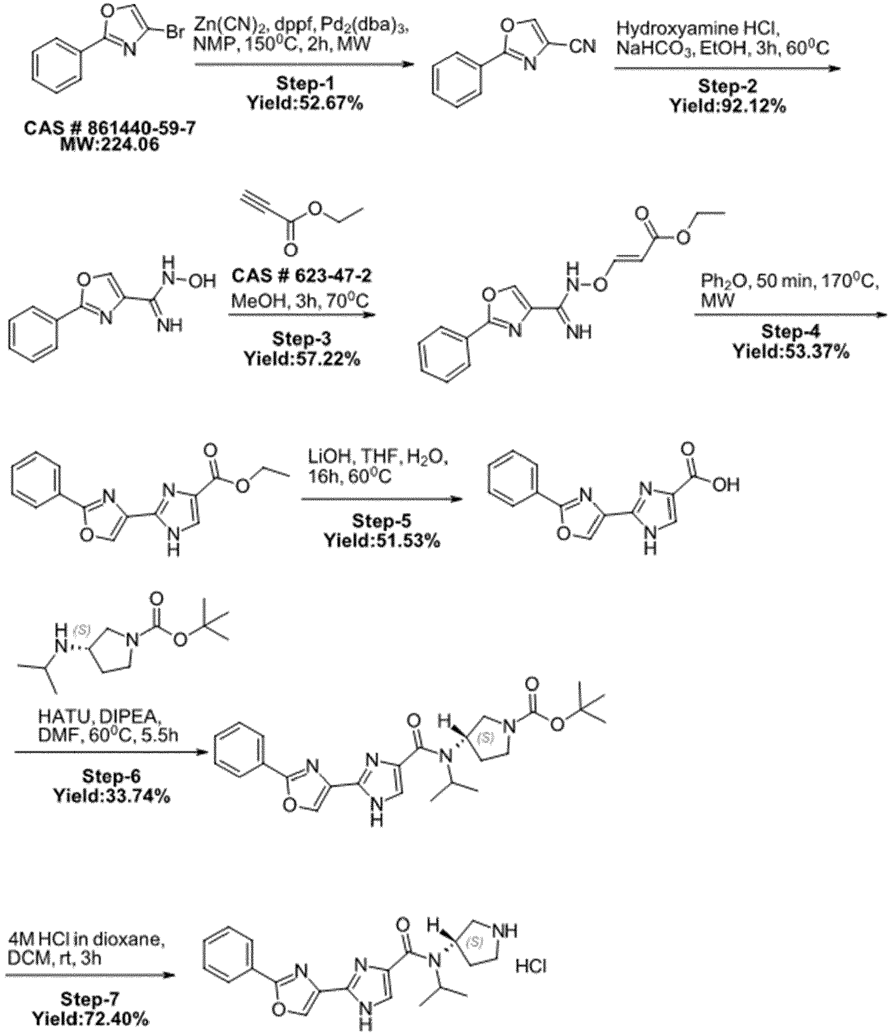

### Synthetic procedures

### Step-1: Synthesis of 2-phenyloxazole-4-carbonitrile

To a stirred solution of 4-bromo-2-phenyloxazole (0.5 g, 2.23 mmol) in NMP (5mL, 10V) at rt, Zn(CN)₂ (0.52 g, 4.46 mmol) and 1,1'-Bis(diphenylphosphino)ferrocene (0.12 g, 0.22 mmol) were added. The reaction mixture was purged with N₂ gas for 10 min. After 10 min Tris(dibenzylideneacetone)dipalladium(0) (0.2 g, 0.22 mmol) was added to the reaction mixture, which was purged again for 10 amin with N₂ gas. The reaction mixture was stirred at 150 °C for 2 h under microwave irradiation. The progress of the reaction was monitored by TLC analysis. After completion of the reaction, the reaction mixture was poured into water (250 mL). A brown precipitate was formed which was isolated by filtration through a Büchner funnel, washing on the filter with DCM (700 mL). The filtrate was concentrated under reduced pressure. The crude material was purified by column chromatography using neutral alumina (2% ethyl acetate in hexane) to provide 2-phenyloxazole-4-carbonitrile (0.6 g, 52.67 % yield) as dark yellow solid. Note that three batches of this reaction were carried out in parallel (on the same scale as described here) and the product yield quoted here is based on the combination of all these three batches. **¹H NMR (400 MHz, *d*₆-DMSO):** δ 9.23 (s, 1H), 8.3 (dd, J = 3.2 Hz 2H), 7.64 - 7.59 (m, 3H).

### Step-2: Synthesis of N-hydroxy-2-phenyloxazole-4-carboximidamide

To a stirred solution of Hydroxylamine HCl (0.61 g, 8.81 mmol) in Water (3 mL, 5V) at 0⁰C, NaHCO₃ (0.74 g, 8.81 mmol) was added and the reaction stirred for 15 min. Then a solution of 2-phenyloxazole-4-carbonitrile (0.6g, 3.52mmol) in EtOH (6mL, 10V) was added. The reaction mixture was stirred at 60 °C for 3h. The progress of the reaction was monitored by TLC analysis. The reaction mixture was concentrated under reduced pressure, then ice cold water (30mL) was added to reaction mixture. A precipitate formed which was filtered through a Büchner funnel and dried under reduced pressure to provide N-hydroxy-2-phenyloxazole-4-carboximidamide (0.66 g, 92.12 % yield) as a yellow solid.
**LCMS [ESI, M+1]:** 203.8 (RT: 1.106 min, Purity: 98.34 %)

### Step-3: Synthesis of ethyl (E)-3-((2-phenyloxazole-4-carboximidamido) oxy) acrylate:

To a stirred solution N-hydroxy-2-phenyloxazole-4-carboximidamide (0.66 g, 3.24 mmol) in MeOH (6.6 mL, 10V) at rt, ethyl propiolate (1.11 g, 11.36 mmol) was added. The reaction mixture was stirred at 70 °C for 3h. The progress of the reaction was monitored by TLC analysis. The reaction mixture was concentrated under reduced pressure and the crude material purified by column chromatography (8% EtOAc in hexane) to provide ethyl (E)-3-((2-phenyloxazole-4-carboximidamido) oxy) acrylate (0.56 g, 57.22 % yield) as a yellow solid.
**LCMS [ESI, M+1]:** 301.8 (RT: 2.360 min, Purity:100%)

### Step-4: Synthesis of ethyl 2-(2-phenyloxazol-4-yl)-1H-imidazole-4-carboxylate

A stirred solution of ethyl (E)-3-((2-phenyloxazole-4-carboximidamido)oxy)acrylate (0.28 g, 0.92 mmol) in Diphenyl ether (1.4 mL, 5V) was heated at 170 °C for 50 min under microwave irradiation. The progress of the reaction was monitored by TLC analysis. After completion of the reaction the reaction mixture was directly bound to 230-400 mesh Silica gel and purified by column chromatography (25% EtOAc in hexane) to provide ethyl 2-(2-phenyloxazol-4-yl)-1H-imidazole-4-carboxylate (0.28 g, 53.37 % yield) as a yellow sticky solid. Note that two batches of this reaction were carried out in parallel (on the same scale as described here) and the product yield quoted here is based on the combination of these two batches.
**LCMS [ESI, M+1]:** 283.7 (RT: 1.478, Purity: 98.13%),

### Step-5: Synthesis of 2-(2-phenyloxazol-4-yl)-1H-imidazole-4-carboxylic acid

To a stirred solution of ethyl 2-(2-phenyloxazol-4-yl)-1H-imidazole-4-carboxylate (0.28 g, 0.98 mmol) in THF (1.68 mL, 6V) at rt, a solution of LiOH (0.25 g, 5.93 mmol) in H₂O (1.12 mL, 4V) was added. The reaction mixture was stirred for 16 h at 60 °C. The progress of the reaction was monitored by TLC analysis. The reaction mixture was concentrated under reduced pressure, then diluted with water (3.0mL) and acidified by the addition of a saturated solution of citric acid (25.0 mL). The precipitate was filtered out and dried under reduced pressure to provide 2-(2-phenyloxazol-4-yl)-1H-imidazole-4-carboxylic acid (0.13 g, 51.53 % yield) as an off-white solid.
**LCMS [ESI, M+1]:** 255.7 (RT: 1.184 min, Purity: 100%)

### Step-6: Synthesis of tert-butyl (S)-3-(N-isopropyl-2-(2-phenyloxazol-4-yl)-1H-imidazole-4-carboxamido) pyrrolidine-1-carboxylate

To a stirred solution of 2-(2-phenyloxazol-4-yl)-1H-imidazole-4-carboxylic acid (0.13 g, 0.5 mmol) in DMF (1.3 mL, 10V) at rt, HATU (0.29 g, 0.76 mmol) was added. The reaction was stirred at rt for 30 min. Tert-butyl (S)-3-(isopropylamino) pyrrolidine-1-carboxylate (0.16 g, 0.50 mmol) and DIPEA (0.2mL, 1.52mmol) were added and the reaction mixture was stirred for 5.5 h at 60 °C. The progress of the reaction was monitored by TLC analysis. After completion of the reaction, ice cold water (100 mL) was added to reaction mixture. A precipitate was formed which was filtered through a Büchner funnel and dried under reduced pressure. The crude material was purified by column chromatography (50% ethyl acetate in hexane) to provide tert-butyl(S)-3-(N-isopropyl-2-(2-phenyloxazol-4-yl)-1H-imidazole-4-carboxamido) pyrrolidine-1-carboxylate (0.08 g, 33.74 % yield) as a white sticky solid.
**LCMS [ESI, M, M-100]:** 366.4 (RT: 2.239 min, Purity: 97.82%)

### Step-7: Synthesis of (S)-N-isopropyl-2-(2-phenyloxazol-4-yl)-N-(pyrrolidin-3-yl)-1H-imidazole-4-carbox amide hydrochloride (Compound K):

To a stirred solution of get tert-butyl(S)-3-(N-isopropyl-2-(2-phenyloxazol-4-yl)-1H-imidazole-4-carboxamido) pyrrolidine-1-carboxylate (0.08 g, 0.17 mmol) in CH₂Cl₂ (0.8 mL, 10V) at 0 °C, 4M HCl in Dioxane (0.4 mL, 5V) was added. The reaction mixture was stirred at room temperature for 3 h. The progress of the reaction was monitored by TLC analysis. After completion of the reaction, the reaction mixture was concentrated under reduced pressure and azeotroped using CH₂Cl₂ (3 x 13 mL) to provide crude material, which was triturated using CH₂Cl₂ (1 mL) and diethyl ether (2 x 17 mL) to provide (S)-N-isopropyl-2-(2-phenyloxazol-4-yl)-N-(pyrrolidin-3-yl)-1H-imidazole-4-carboxamide hydrochloride (Compound K; 0.05 g, 72.40 % yield) as off-white solid.
**LCMS [ESI, M+1]:** 366.1 (RT:1.216 min, Purity: 98.24%),
**HPLC Purity:** RT: 3.458 min, Purity: 96.17%,
**Chiral HPLC:** RT: 2.746 min, Purity: 98.02
**¹H NMR (400 MHz, CD₃OD):** δ 8.83 (s, 1H), 8.20 (dd, J = 7.8, 1.7 Hz, 2H), 8.04 (s, 1H), 7.64 - 7.58 (m, 3H), 4.61 - 4.49 (m, 2H), 3.83 (t, J = 8.9 Hz, 1H), 3.69 (dd, J = 9.9 Hz, 1H), 3.53 (t, J = 10 Hz, 1H), 3.31 - 3.21 (m, 1H), 2.533 - 2.479 (m, 1H), 2.441 - 2.371 (m, 1H), 1.44-1.28 (dd, J = 9.4, 6.6 Hz, 6H).

### Synthesis of N-isopropyl-N-4-piperidyl-2-(4-cyano-1-phenyl-3-pyrazolyl)-1,3-oxazole-4-carboxamide hydrochloride (Compound L)

### Overall synthetic scheme

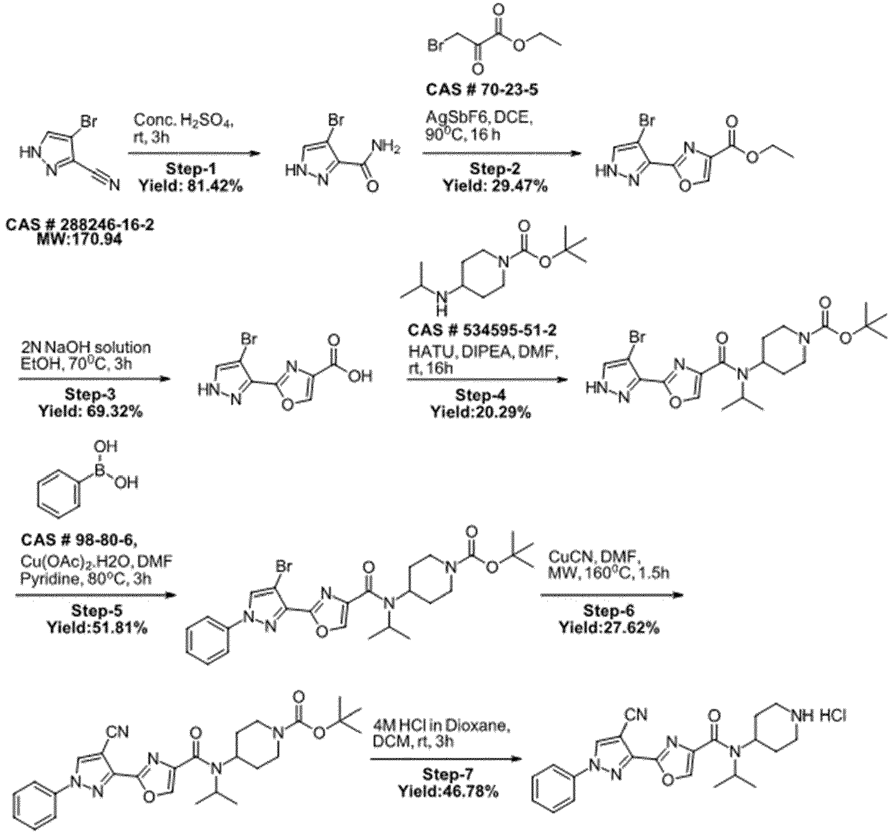

### Synthetic procedures

### Step-1: Synthesis of 4-bromo-1H-pyrazole-3-carboxamide

Conc. H₂SO₄ (30 mL, 6V) was added dropwise with stirring to 4-bromo-1H-pyrazole-3-carbonitrile (5.0 g, 29.0 mmol) at 0 °C. The reaction mixture was stirred at rt for 3 h. The progress of the reaction was monitored by TLC analysis. After completion of the reaction, the reaction mixture was quenched by the addition of ice cold water (150 mL) and neutralized using an aq. NH₄OH solution (pH approximately of 7) resulting in the formation of a solid precipitate. The solid material was filtered and dried under reduced pressure to provide 4-bromo-1H-pyrazole-3-carboxamide (9.0 g, 81.42 % yield) as a white solid. Note that two batches of this reaction were carried out in parallel (on the same scale as described here) and the product yield quoted here is based on the combination of these two batches.
**LCMS [ESI, M, M+2]:** 189.8, 191.8 (RT: 0.749 min, Purity: 99.17%),
**¹H NMR (400 MHz, *d*₆-DMSO):** δ 12.63 (bs, 1H), 8.00 (s, 1H), 7.48 (bs, 1H), 7.34 (bs, 1H).

### Step-2: Synthesis of ethyl 2-(4-bromo-1H-pyrazol-3-yl) oxazole-4-carboxylate

To a stirred solution of 4-bromo-1H-pyrazole-3-carboxamide (4.5 g, 23.8 mmol) in DCE (90 mL, 20V) at rt, ethyl 3-bromo-2-oxopropanoate (18.6 g, 95.2 mmol) and AgSbF₆ (8.19 g, 23.8 mmol) were added. The reaction mixture was stirred at 90 °C for 16 h. The progress of the reaction was monitored by TLC analysis. After completion of the reaction, the reaction mixture was cooled to rt and filtered through Celite^{®}. The filtrate was extracted with CH₂Cl₂ (3 x 100 mL). The combined organic fractions were dried over Na₂SO₄, and concentrated under reduced pressure. The crude material was purified by normal phase column chromatography (neutral alumina; 28% CH₂Cl₂: MeOH: NH₄OH (70:30:0.1%)) to provide ethyl 2-(4-bromo-1H-pyrazol-3-yl) oxazole-4-carboxylate (4.0 g, 29.47 % yield) as a yellow solid. Note that two batches of this reaction were carried out in parallel (on the same scale as described here) and the product yield quoted here is based on the combination of these two batches.
**LCMS [ESI, M, M+2]:** 285.7, 287.6 (RT: 1.332 min, Purity: 89.95%),

### Step-3: Synthesis of 2-(4-bromo-1H-pyrazol-3-yl) oxazole-4-carboxylic acid

To a stirred solution of ethyl 2-(4-bromo-1H-pyrazol-3-yl)oxazole-4-carboxylate (4.0 g, 14.0 mmol) in EtOH (40mL, 10V) at rt, 2N NaOH solution (20 mL, 5V) was added. The reaction mixture was stirred at 70 °C for 3 h. The progress of the reaction was monitored by TLC analysis. After completion of the reaction, the reaction mixture was cooled to rt and concentrated under reduced pressure. The crude material was acidified with sat. citric acid solution (to a pH of approximately 4) resulting in the formation of a solid precipitate. The solid material was filtered and dried under reduced pressure to provide 2-(4-bromo-1H-pyrazol-3-yl) oxazole-4-carboxylic acid (2.5 g, 69.32 % yield) as a white solid.
**LCMS [ESI, M, M+2]:** 257.6, 259.7 (RT: 1.07 min, Purity: 100%),
**¹H NMR (400 MHz, *d*₆-DMSO):** δ 14.2 (bs, 1H), 8.15 (s, 1H), 7.98 (s, 1H).

### Step-4: Synthesis of tert-butyl 4-(2-(4-bromo-1H-pyrazol-3-yl)-N-isopropyloxazole-4-carboxamido) piperidine-1-carboxylate

To a stirred solution of 2-(4-bromo-1H-pyrazol-3-yl) oxazole-4-carboxylic acid (0.5 g, 1.95 mmol) in DMF (5mL, 10V) at 0°C, HATU (1.48 g, 3.90 mmol) was added. After 15 min, tert-butyl 4-(isopropylamino) piperidine-1-carboxylate (0.56 g, 2.34 mmol) and DIPEA (1.4mL, 7.8mmol) were added at 0°C. The reaction mixture was stirred at rt 16h. The progress of the reaction was monitored by TLC analysis. After completion of the reaction, the reaction mixture was diluted with water (50 mL) and extracted with EtOAc (2 x 50 mL). The combined organic fractions were dried over Na₂SO₄, filtered and concentrated under reduced pressure. The crude material was purified by reverse phase column chromatography (52% CH₃CN in H₂O) to provide tert-butyl 4-(2-(4-bromo-1H-pyrazol-3-yl)-N-isopropyloxazole-4-carboxamido) piperidine-1-carboxylate (0.38 g, 20.29 % yield) as a yellow solid. Note that two batches of this reaction were carried out in parallel (on the same scale as described here) and the product yield quoted here is based on the combination of these two batches.
**LCMS [ESI, M-1, M+1]:** 480.1, 482.1 (RT: 1.714 min, Purity: 86.08%),

### Step-5: Synthesis of tert-butyl 4-(2-(4-bromo-1-phenyl-1H-pyrazol-3-yl)-N-isopropyloxazole-4-carboxamido) piperidine-1-carboxylate

To a stirred solution of tert-butyl 4-(2-(4-bromo-1H-pyrazol-3-yl)-N-isopropyloxazole-4-carboxamido) piperidine-1-carboxylate (0.2 g, 0.41 mmol) in DMF (2.0 mL, 10V) at rt, phenylboronic acid (0.08 g, 0.7 mmol), Cu(OAc)₂.H₂O (0.15 g, 0.83 mmol) and Pyridine (0.32 g, 0.83 mmol) were added. The reaction mixture was stirred at 80°C for 3 h. The progress of the reaction was monitored by TLC analysis. After completion of the reaction, the reaction mixture was cooled to rt, diluted with water (50 mL) and extracted with EtOAc (2 x 30 mL). The combined organic fractions were dried over Na₂SO₄ and concentrated under reduced pressure. The crude material was purified by normal phase column chromatography (25% EtOAc in Hexane) to provide tert-butyl 4-(2-(4-bromo-1-phenyl-1H-pyrazol-3-yl)-N-isopropyloxazole-4-carboxamido) piperidine-1-carboxylate (0.12 g, 51.81% yield) as a sticky yellow solid.
**LCMS [ESI, M-100]:** 457.9 (RT: 2.243 min, Purity: 89.78%),

### Step-6: Synthesis of tert-butyl 4-(2-(4-cyano-1-phenyl-1H-pyrazol-3-yl)-N-isopropyloxazole-4-carboxamido) piperidine-1-carboxylate

To a stirred solution of tert-butyl 4-(2-(4-bromo-1-phenyl-1H-pyrazol-3-yl)-N-isopropyloxazole-4-carboxamido) piperidine-1-carboxylate (0.12 g, 0.2 mmol) in DMF (1.2 mL, 10V) at rt, CuCN (0.05 g, 0.60 mmol) was added. The reaction mixture was stirred at 160°C for 1.5 h under microwave irradiation. The progress of the reaction was monitored by TLC analysis. After completion of the reaction, the reaction mixture was concentrated under reduced pressure. The crude material was purified by normal phase column chromatography (20% EtOAc in hexane) to provide tert-butyl 4-(2-(4-cyano-1-phenyl-1H-pyrazol-3-yl)-N-isopropyloxazole-4-carboxamido) piperidine-1-carboxylate (0.03 g, 27.62 % yield) as a yellow sticky solid.
**LCMS [ESI, M-100]:** 405.0 (RT: 2.079 min, Purity: 73.04%),

### Step-7: Synthesis of 2-(4-cyano-1-phenyl-1H-pyrazol-3-yl)-N-Isopropyl-N-(piperidin-4-yl) oxazole-4-carboxamide hydrochloride (Compound L)

To a stirred solution of tert-butyl 4-(2-(4-cyano-1-phenyl-1H-pyrazol-3-yl)-N-isopropyloxazole-4-carboxamido) piperidine-1-carboxylate (0.05 g, 0.09 mmol) in CH₂Cl₂ (0.5 mL) at 0°C, 4M HCl in Dioxane (0.5 mL) was added. The reaction mixture was stirred at rt for 3h. The progress of the reaction was monitored by TLC analysis. After completion of the reaction, the reaction mixture was concentrated under reduced pressure. The crude material was purified by preparative HPLC purification (Mobile Phase: A-0.05% HCl in H₂O, Mobile Phase: B-Acetonitrile: H₂O (80:20)) to provide 2-(4-cyano-1-phenyl-1H-pyrazol-3-yl)-N-Isopropyl-N-(piperidin-4-yl) oxazole-4-carboxamide hydrochloride (Compound L; 0.03 g, 46.78% yield) as an off-white solid.
**LCMS [ESI, M+1]:** 405.16 (RT: 1.294 min, Purity: 99.21%),
**HPLC Purity:** RT: 6.08 min, Purity: 98.24%,
**¹H NMR (400 MHz, *d₆*-DMSO):** δ 9.57 (s, 1H), 8.71 (s, 2H), 7.94 (d, *J* = 7.6 Hz, 2H), 7.62 (t, *J* = 15.6 Hz, 2H), 7.51 (t, *J* = 14.8 Hz, 1H), 4.63-4.49 (m, 1H), 3.55 (bs, 1H), 3.02-2.88 (m, 4H), 2.06 (bs, 2H), 1.70 (bs, 1H), 1.43(bs, 2H), 1.27-1.23 (m, 6H).

### Synthesis of N-isopropyl-N-4-piperidyl-2-(4-cyano-1-phenyl-3-pyrazolyl)-1,3-thiazole-4-carboxamide hydrochloride (Compound M)

### Overall synthetic scheme

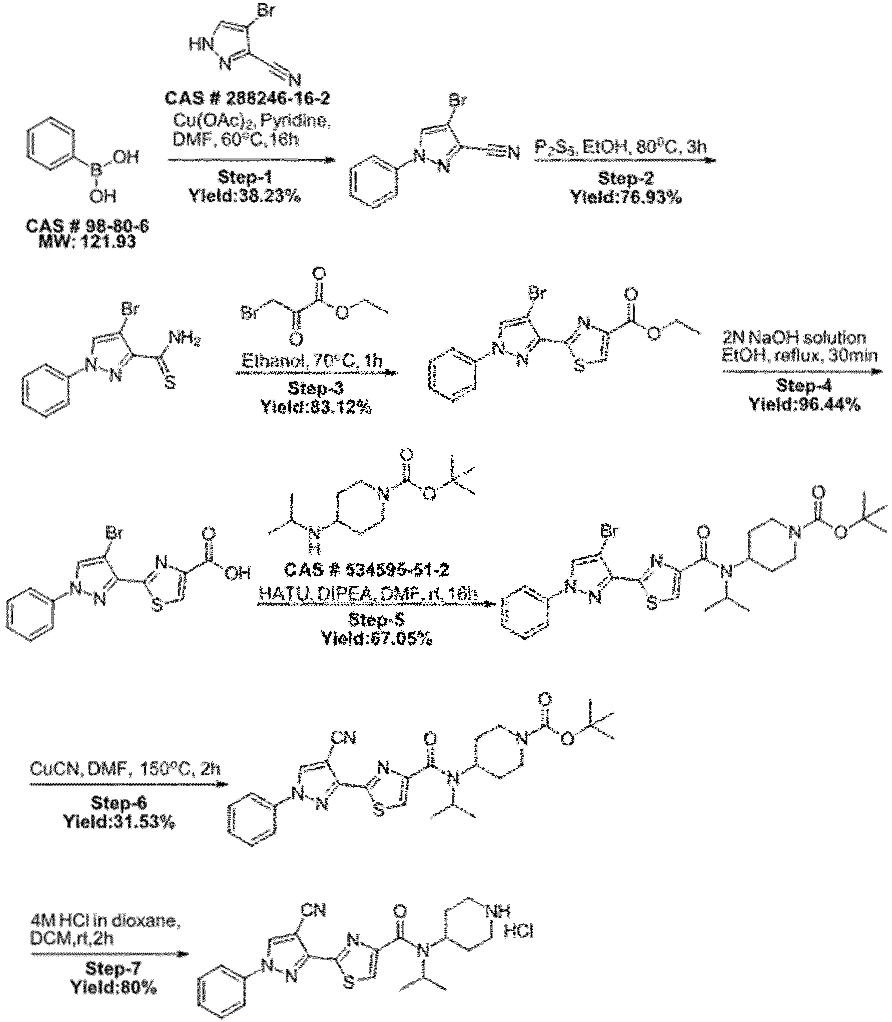

### Synthetic procedures

### Step-1: Synthesis of 4-bromo-1-phenyl-1H-pyrazole-3-carbonitrile

To a stirred solution of phenylboronic acid (1.12 g, 9.18 mmol) in DMF (11.2 mL, 10V) at rt, pyridine (1.46 mL, 18.37 mmol), 4-bromo-1H-pyrazole-3-carbonitrile (1.87 g, 11.02 mmol) and Copper(II) acetate (3.31 g, 18.37 mmol) were added. The reaction mixture was heated at 60 °C for 16 h. The progress of the reaction was monitored by TLC analysis. After completion of the reaction, the reaction mixture was filtered through a Büchner funnel. Water (100 mL) was added to the filtrate, and this was extracted with EtOAc (3 x 100 mL). The combined organic fractions were dried over Na₂SO₄ and concentrated under reduced pressure. The crude material was purified by column chromatography (5% EtOAc in hexane) to provide 4-bromo-1-phenyl-1H-pyrazole-3-carbonitrile (7.0 g, 38.23 % yield) as an off-white solid. Note that eight batches of this reaction were carried out (on the same scale as described here) and the product yield quoted here is based on the combination of these eight batches.

**¹H NMR (400 MHz, CDCl₃):** δ 8.04 (s, 1H), 7.69-7.66 (m, 2H), 7.57-7.52 (m, 2H), 7.48-7.43 (m, 1H).

### Step-2: Synthesis of 4-bromo-1-phenyl-1H-pyrazole-3-carbothioamide

To a stirred solution of 4-bromo-1-phenyl-1H-pyrazole-3-carbonitrile (1.0 g, 4.03 mmol) in EtOH (10 mL, 10V) at 0 °C, P₂S₅ (3.58 g, 8.46 mmol) was added. The reaction mixture was heated at 80 °C for 3 h. The progress of the reaction was monitored by TLC analysis. After completion of the reaction, the reaction mixture was poured into sat. NaHCO₃ solution (100 mL) and extracted with EtOAc (2 x 50 mL). The combined organic fractions were washed with water (100 mL), dried over Na₂SO₄ and concentrated under reduced pressure. The crude material was purified by column chromatography (20% EtOAc in Hexane) to provide 4-bromo-1-phenyl-1H-pyrazole-3-carbothioamide (7.0 g, 76.93 %) as a pale yellow solid. Note that eight batches of this reaction were carried out (on the same scale as described here) and the product yield quoted here is based on the combination of these eight batches.

**LCMS [ESI, M+1]:** 283.7 (RT: 1.753min, Purity: 90.52%).

### Step-3: Synthesis of ethyl 2-(4-bromo-1-phenyl-1H-pyrazol-3-yl)thiazole-4-carboxylate

To a stirred a solution of 4-bromo-1-phenyl-1H-pyrazole-3-carbothioamide (7.0 g, 24.80 mmol) in EtOH (70mL, 10V) at rt, ethyl 3-bromo-2-oxopropanoate (9.68 g, 49.60 mmol) was added. The reaction mixture was stirred at 70°C for 1h. The progress of the reaction was monitored by TLC and LCMS analysis. After completion of the reaction, the reaction mixture was concentrated under reduced pressure. Sat. NaHCO₃ solution (200 mL) was added, and the mixture extracted with EtOAc (2 x 300 mL). The combine organic fractions were dried over Na₂SO₄ and concentrated under reduced pressure. The crude material was purified by trituration using diethyl ether (2 x 50 mL) to provide ethyl 2-(4-bromo-1-phenyl-1H-pyrazol-3-yl)thiazole-4-carboxylate (7.8 g, 83.12 % yield) as a pale yellow solid.

**LCMS [ESI, M+1]:** 379.8 (RT: 2.258min, Purity: 99.73%).

### Step-4: Synthesis of 2-(4-bromo-1-phenyl-1H-pyrazol-3-yl)thiazole-4-carboxylic acid

To a stirred solution of ethyl 2-(4-bromo-1-phenyl-1H-pyrazol-3-yl) thiazole-4-carboxylate (5.6 g, 14.80 mmol) in EtOH (56mL, 10V) at rt, A solution of 2N NaOH solution in water (56 mL, 10V) was added. The reaction mixture was heated at reflux for 30 min. The progress of the reaction was monitored by TLC and LCMS analysis. After completion of the reaction, the reaction mixture was concentrated under reduced pressure Water was added to the crude material and acidified using 6N HCl (to give a pH of approximately 3). A solid precipitate was formed. The solid material was filtered and dried under reduced pressure to provide 2-(4-bromo-1-phenyl-1H-pyrazol-3-yl) thiazole-4-carboxylic acid (5.0 g, 96.44% yield) as an off-white solid.

**LCMS [ESI, M+1]:** 351.7 (RT: 1.760 min, Purity: 96.44%).

### Step-5: Synthesis of tert-butyl 4-(2-(4-bromo-1-phenyl-1H-pyrazol-3-yl)-N-isopropylthiazole-4-carboxamido)piperidine-1-carboxylate

To a stirred solution of 2-(4-bromo-1-phenyl-1H-pyrazol-3-yl)thiazole-4-carboxylic acid (1.0 g, 2.85 mmol) in DMF (10 mL, 10V) at rt, HATU (1.84 g, 4.85mmol) and DIPEA (1.17 g, 9.14 mmol) were added. After 10min, tert-butyl 4-(isopropylamino) piperidine-1-carboxylate (0.82 g, 3.42 mmol) was added at rt and the reaction mixture was stirred at rt for 16h. The progress of the reaction was monitored by TLC and LCMS analysis. After completion of the reaction, the reaction mixture was poured into ice and a solid precipitate was formed. The solid material was filtered, washed with hexane (150 mL) and dried under reduce pressure to provide tert-butyl 4-(2-(4-bromo-1-phenyl-1H-pyrazol-3-yl)-N-isopropylthiazole-4-carboxamido)piperidine-1-carboxylate (1.1 g, 67.05 % yield) as a light brown solid.
**LCMS [ESI, M-56]:** 519.73 (RT: 2.657 min, Purity: 67.05%),

### Step-6: Synthesis of tert-butyl 4-(2-(4-cyano-1-phenyl-1H-pyrazol-3-yl)-N-isopropylthiazole-4-carboxamido) piperidine-1-carboxylate

To a stirred solution of tert-butyl 4-(2-(4-bromo-1-phenyl-1H-pyrazol-3-yl)-N-isopropylthiazole-4-carboxamido) piperidine-1-carboxylate (1.4 g, 2.43 mmol) in DMF (14 mL, 10V) at rt, CuCN (0.54 g, 6.09 mmol) was added. The reaction mixture was stirred at 150 °C for 2 h under microwave irradiation. The progress of the reaction was monitored by TLC and LCMS analysis. After completion of the reaction, the reaction mixture was poured into ice cold water and a solid precipitate was formed. The solid material was filtered, dissolved in CH₂Cl₂ (50 mL) and filtered again. The filtrate was dried over Na₂SO₄ and concentrated under reduced pressure. The crude material was purified by column chromatography (13% EtOAc in hexane) to provide tert-butyl 4-(2-(4-cyano-1-phenyl-1H-pyrazol-3-yl)-N-isopropylthiazole-4-carboxamido) piperidine-1-carboxylate (0.4 g, 31.53 % yield) as a pale yellow solid. Note that four batches of this reaction were carried out (on the same scale as described here) and the product yield quoted here is based on the combination of these four batches.
**LCMS [ESI, M-56]:** 464.97 (RT: 2.479 min, Purity: 89.80%),

### Step-7: Synthesis of 2-(4-cyano-1-phenyl-1H-pyrazol-3-yl)-N-isopropyl-N-(piperidin-4-yl)thiazole-4-carboxamide hydrochloride (Compound M)

To a stirred solution of tert-butyl tert-butyl 4-(2-(4-cyano-1-phenyl-1H-pyrazol-3-yl)-N-isopropylthiazole-4-carboxamido) piperidine-1-carboxylate (0.4 g, 0.76 mmol) in CH₂Cl₂ (4 mL, 10V) at rt, 4M HCl in Dioxane (1.2 mL, 3V) was added. The reaction mixture was stirred at rt for 2 h. The progress of the reaction was monitored by TLC and LCMS analysis. After completion of the reaction, the reaction mixture was concentrated under reduced pressure. The crude material was purified by trituration using diethyl ether (40 mL) to provide 2-(4-cyano-1-phenyl-1H-pyrazol-3-yl)-N-Isopropyl-N-(piperidin-4-yl) thiazole-4-carboxamide hydrochloride (0.27 g, 80% yield) as an off-white solid.
**LCMS [ESI, M+1]:** 421.01 (RT: 1.502 min, Purity: 99.65%),
**HPLC Purity:** RT: 5.589 min, Purity: 96.50%
**¹H NMR (400 MHz, CD₃OD):** δ 9.15 (s, 1H), 8.17-8.10 (m, 1H), 7.92 (d, *J*=7.6 Hz, 2H), 7.61 (t, *J*=16 Hz, 7.6 Hz, 2H), 7.50 (t, *J*=14.8 Hz, 7.2 Hz, 1H), 4.64-4.49 (m, 1H), 3.67-3.65 (m, 1H), 3.52-3.44 (m, 2H), 3.28-3.10 (m, 3H), 2.40-2.37 (m, 1H), 2.13-2.09 (m, 1H), 1.94 (d, *J*=12.4 Hz, 2H), 1.59 (d, *J*=5.2 Hz, 2H), 1.41-1.31 (m, 4H).

### Synthesis of N-isopropyl-N-4-piperidyl-1-(1-phenyl-4-pyrazolyl)-1H-1,2,3-triazole-4-carboxamide hydrochloride (Compound N)

### Overall synthetic scheme

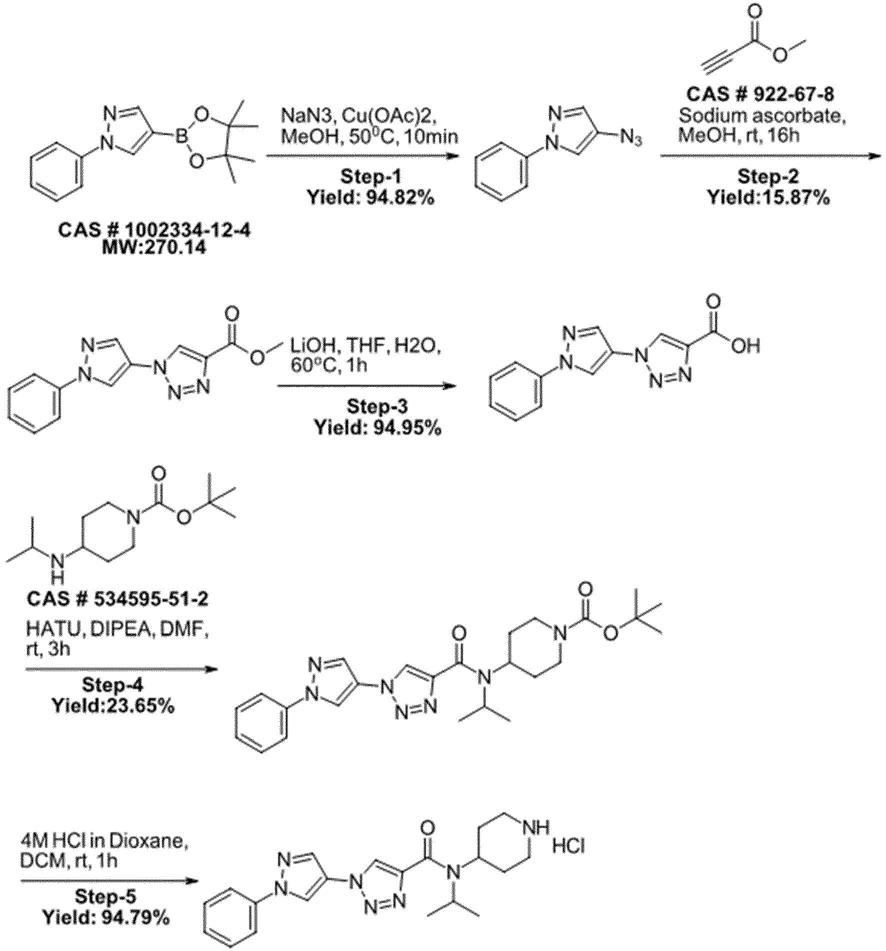

### Synthetic procedures

### Step-1: Synthesis of 4-azido-1-phenyl-1H-pyrazole

To a stirred solution of 1-phenyl-4-(4,4,5,5-tetramethyl-1,3,2-dioxaborolan-2-yl)-1H-pyrazole (2.0 g, 7.40 mmol) in methanol (20mL) at 0⁰C, copper(II) acetate (0.13 g, 0.74 mmol) and NaN₃ (0.69 g, 11.10 mmol) were added. The reaction mixture was refluxed at 50 °C for 10min. The progress of the reaction was monitored by TLC and LCMS analysis. After completion of the reaction, the reaction mixture was concentrated under reduced pressure at 38 °C (water bath temp.). The crude material was purified by column chromatography (2% EtOAc in Hexane) to provide 4-azido-1-phenyl-1H-pyrazole (1.3 g, 94.82 %) as a brown yellow liquid. Note that four batches of this reaction were carried out (on the same scale as described here) and the product yield quoted here is based on the combination of these four batches.
**LCMS [ESI, M+1]:** 186.13 (RT: 2.174 min, Purity: 39.30%)

### Step-2: Synthesis of methyl 1-(1-phenyl-1H-pyrazol-4-yl)-1H-1,2,3-triazole-4-carboxylate

To a stirred solution of 4-azido-1-phenyl-1H-pyrazole (1.3 g, 7.01 mmol) in methanol (13mL) at rt, sodium ascorbate (0.13 g, 0.70 mmol) was added. After 5 min, methyl propionate (1.76 g, 21.05 mmol) was added at rt and the reaction mixture was stirred at rt for 16 h. The progress of the reaction was monitored by TLC and LCMS analysis. After completion of the reaction, the reaction mixture was concentrated under reduced pressure. The crude material was purified by column chromatography (30% EtOAc in Hexane) to provide methyl 1-(1-phenyl-1H-pyrazol-4-yl)-1H-1,2,3-triazole-4-carboxylate (0.3 g, 15.87 % yield) as a white solid.
**LCMS [ESI, M+1]:** 269.84 (RT: 1.585min, Purity: 82.86%)

### Step-3: Synthesis of 1-(1-phenyl-1H-pyrazol-4-yl)-1H-1,2,3-triazole-4-carboxylic acid

To a stirred solution of methyl 1-(1-phenyl-1H-pyrazol-4-yl)-1H-1,2,3-triazole-4-carboxylate (0.3 g, 1.11 mmol) in THF (2mL) at rt, LiOH (0.87 g, 3.34 mmol) in H₂O (1 mL) was added. The reaction mixture was stirred at 60 °C for 1 h. The progress of the reaction was monitored by TLC and LCMS analysis. After completion of the reaction, the reaction mixture was distilled under vacuum. The reaction mixture was poured into water (50 mL) and extracted with EtOAc (10 mL). The aqueous layer was acidified using sat. citric acid (to a pH of approximately 3) leading to the formation of a solid precipitate. The solid material was filtered and dried under reduced pressure to provide 1-(1-phenyl-1H-pyrazol-4-yl)-1H-1,2,3-triazole-4-carboxylic acid (0.27 g, 94.95 % yield) as a light pink solid.
**LCMS [ESI, M+1]:** 255.8 (RT: 1.341min, Purity: 91.46%)

### Step-4: Synthesis of tert-butyl 4-(N-isopropyl-1-(1-phenyl-1H-pyrazol-4-yl)-1H-1,2,3-triazole-4-carboxamido) piperidine-1-carboxylate

To a stirred solution of 1-(1-phenyl-1H-pyrazol-4-yl)-1H-1,2,3-triazole-4-carboxylic acid (0.27 g, 1.05 mmol) in DMF (2.7 mL) at rt, HATU (0.6 g, 1.58 mmol) and DIPEA (0.54 ml, 3.17 mmol) were added. After 1h, tert-butyl 4-(isopropylamino) piperidine-1-carboxylate (0.07 g, 0.28 mmol) was added at rt and the reaction mixture was stirred at rt for 2h. The progress of the reaction was monitored by TLC and LCMS analysis. After completion of the reaction, the reaction mixture was poured into ice-cold water (50 mL) and extracted with EtOAc (3 x 50 mL). The combine organic fractions were washed with cold water (3 x 10 mL), dried over Na₂SO₄ and concentrated under reduced pressure. The crude material was purified by column chromatography (18-20% Ethyl Acetate in Hexane] to provide tert-butyl 4-(N-isopropyl-1-(1-phenyl-1H-pyrazol-4-yl)-1H-1,2,3-triazole-4-carboxamido) piperidine-1-carboxylate (0.12 g, 23.65 % yield) as a brown solid. Note that two batches of this reaction were carried out (on the same scale as described here) and the product yield quoted here is based on the combination of these two batches.
**LCMS [ESI, M-56]:** 424.06 (RT: 2.247min, Purity: 83.41%)

### Step-5: Synthesis of N-isopropyl-1-(1-phenyl-1H-pyrazol-4-yl)-N-(piperidin-4-yl)-1H-1,2,3-triazole-4-carboxamide hydrochloride (Compound N)

To a stirred solution of tert-butyl 4-(N-isopropyl-1-(1-phenyl-1H-pyrazol-4-yl)-1H-1,2,3-triazole-4-carboxamido)piperidine-1-carboxylate (0.12 g, 0.25 mmol) in CH₂Cl₂ (1.2mL, 10V) at 0 °C, 4M HCl in dioxane (0.6 mL, 5V) was added. The reaction mixture was stirred at rt for 1h. The progress of the reaction was monitored by TLC and LCMS analysis. After completion of the reaction, the reaction mixture was concentrated under reduced pressure. The crude material was purified by trituration using diethyl ether (2 x 10 mL) to provide N-isopropyl-1-(1-phenyl-1H-pyrazol-4-yl)-N-(piperidin-4-yl)-1H-1,2,3-triazole-4-carboxamide hydrochloride (Compound N; 0.09 g, 80 % yield) as a white solid.
**LCMS [ESI, M+1]:** 380.06 (RT: 1.329 min, Purity: 95.89%)
**HPLC Purity:** RT: 5.371 min, Purity: 96.55%
**¹H NMR (400 MHz, *d*₆-DMSO):** δ 9.27 (s, 1H), 8.99 (s, 1H), 8.78 (s, 1H), 8.38 (s, 1H), 7.90 (d, *J*=7.7 Hz, 2H), 7.58 (t, *J*=8.0 Hz, 2H), 7.41 (t, *J*=7.4 Hz, 1H), 4.67 (bs, 2H), 3.59-3.57 (m, 1H), 3.04-2.91 (m, 4H), 2.19-2.06 (m, 1H), 1.73-1.70 (m, 2H), 1.44-1.26 (m, 6H).

### Experimental protocol for compound SLN-X-0973

### Synthesis of N-isopropyl-2-(2-methylimidazo[1,2-a] pyridin-3-yl)-N-(piperidin-4-yl) thiazole-4-carboxamide hydrochloride

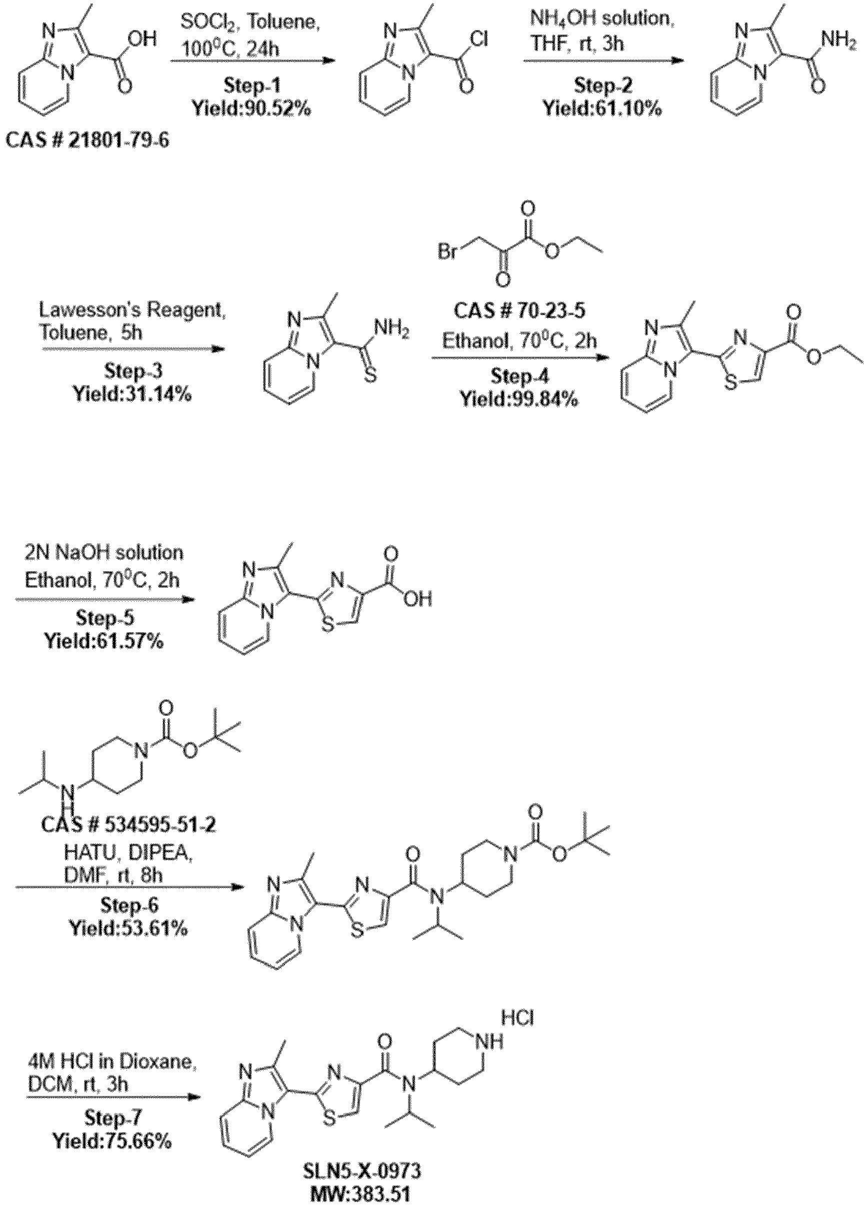

### Step-1: Synthesis of 2-methylimidazo[1,2-a] pyridine-3-carbonyl chloride

To a stirred solution of 2-methylimidazo[1,2-a] pyridine-3-carboxylic acid (2.0 g, 11.35 mmol) in toluene (14 mL, 7V) was added SOCl₂ (8.45 mL, 116.3 mmol) dropwise at 0 °C. The resulting reaction mixture was stirred at 100⁰C for 24h. Progress of the reaction was monitored by TLC analysis. After completion of reaction, the reaction mixture was cooled at rt and evaporated u/vacuum to obtain 2-methylimidazo[1,2-a] pyridine-3-carbonyl chloride (2.0 g, 90.52 %) as a light brown solid. Desired product was confirmed on TLC (using KMnO₄ staining) and forwarded to next step.

### Step-2: Synthesis of 2-methylimidazo[1,2-a] pyridine-3-carboxamide

To a stirred solution of 2-methylimidazo[1,2-a] pyridine-3-carbonyl chloride (0.5 g, 2.5 mmol) in THF (3.75 mL, 7.5V) was added NH₄OH solution (35% in H₂O) (3.75 mL, 7.5V) dropwise at 0⁰C. Than the reaction mixture was stirred at rt for 3 h. Progress of the reaction was monitored by TLC analysis. After completion of reaction, the reaction mixture was evaporated u/vacuum to get crude material, which was purified by normal phase column chromatography (product eluted at 3% MeOH in DCM) to get 2-methylimidazo[1,2-a] pyridine-3-carboxamide (1.1 g, 61.10 %) as a grey solid.

**Note: We have performed 0.5g x 4 batches for this step and yield was combined.**
**LCMS [ESI, M+1]:** 176.1 (RT: 0.396 min, Purity: 99.18%),

### Step-3: Synthesis of 2-methylimidazo[1,2-a] pyridine-3-carbothioamide

To a stirred solution of 2-methylimidazo[1,2-a] pyridine-3-carboxamide (0.5 g, 2.85 mmol) in toluene (5 mL, 10V) was added Lawesson's Reagent (1.73 g, 4.28 mmol) at 0 °C. The resulting reaction mixture was stirred at 100 °C for 5 h. Progress of reaction was monitored by TLC analysis. After completion of reaction, the reaction mixture was diluted with water (30mL) and extracted with EtOAc (3 X 30mL). Combine organic layer dried over Na₂SO₄, filtered and concentrated u/vacuum to get crude material, which was purified by normal phase column chromatography (product eluted at 2% MeOH in DCM) to get 2-methylimidazo[1,2-a] pyridine-3-carbothioamide (0.17 g, 31.14 % yield) as a brown solid.
**LCMS [ESI, M+1]:** 191.9 (RT: 0.861 min, Purity: 100%),

### Step-4: Synthesis of ethyl 2-(2-methylimidazo[1,2-a] pyridin-3-yl) thiazole-4-carboxylate

To a stirred solution of 2-methylimidazo[1,2-a] pyridine-3-carbothioamide (0.3 g, 1.56 mmol) in EtOH (3mL, 10V) was added ethyl 3-bromo-2-oxopropanoate (0.6 g, 3.13 mmol) at rt. The resulting reaction mixture was stirred at 70°C for 2h. Progress of reaction was monitored by TLC analysis. After completion of reaction, the reaction mixture was cooled at rt and solvent was evaporated to get crude material, which was purified by normal phase column chromatography (product eluted at 2% MeOH in DCM) to get ethyl 2-(2-methylimidazo[1,2-a] pyridin-3-yl) thiazole-4-carboxylate (0.45 g, 99.84 %) as a pale yellow solid.
**LCMS [ESI, M+1]:** 288.19 (RT: 1.632 min, Purity: 95.67%)

### Step-5: Synthesis of 2-(2-methylimidazo[1,2-a]pyridin-3-yl)thiazole-4-carboxylic acid

To a stirred solution of ethyl 2-(2-methylimidazo[1,2-a] pyridin-3-yl) thiazole-4-carboxylate (0.45 g, 1.56 mmol) in EtOH (4.5 mL, 10V) was added 2N NaOH solution (4.5 mL, 10V) at rt. The resulting reaction mixture was stirred at 70°C for 2h. Progress of reaction was monitored by TLC analysis. After completion of reaction, the reaction mixture was cooled at rt and solvent was evaporated to get crude material, which was dissolved in water (30mL) and acidify with sat. Citric acid solution until (pH=~4) to get solid precipitated. Solid material was filtered and dried u/vacuum to get 2-(2-methylimidazo[1,2-a] pyridin-3-yl) thiazole-4-carboxylic acid (0.25 g, 61.57 % yield) as a white solid.

**¹H NMR (400 MHz, DMSO):** δ 9.83 (d, *J* = 6.8 Hz, 1H), 8.47 (s, 1H), 7.70 (d, *J* = 9.2 Hz, 1H), 7.51-7.47 (m, 1H), 7.24-7.21 (m, 1H), 2.68 (s, 3H).
**LCMS [ESI, M+1]:** 260.23 (RT: 1.323 min, Purity: 98.28%),

### Step-6: Synthesis of tert-butyl 4-(N-isopropyl-2-(2-methylimidazo[1,2-a] pyridin-3-yl) thiazole-4-carboxamido) piperidine-1-carboxylate

To a stirred solution of 2-(2-methylimidazo[1,2-a] pyridin-3-yl) thiazole-4-carboxylic acid (0.2 g, 0.77 mmol) in DMF (4 mL, 20V) was added HATU (0.58 g, 1.54 mmol) at 0 °C. After 15 min, tert-butyl 4-(isopropylamino) piperidine-1-carboxylate (0.24 g, 1.0 mmol) and DIPEA (0.55 mL, 3.08 mmol) was added at 0°C. The reaction mixture was stirred at rt for 8h. Progress of reaction was monitored by TLC analysis. After completion of reaction, the reaction mixture was diluted with sat. NaHCO₃ solution (50 mL) and extracted with EtOAc (2 x 30 mL). Combine organic layer dried over Na₂SO₄, filtered and concentrated u/vacuum to get crude material, which was purified by normal phase column chromatography (product eluted at 2% MeOH in DCM) to get tert-butyl 4-(N-isopropyl-2-(2-methylimidazo[1,2-a] pyridin-3-yl) thiazole-4-carboxamido) piperidine-1-carboxylate (0.2 g, 53.61 % yield) as a sticky solid.
**LCMS [ESI, M+1]:** 484.38 (RT: 1.520 min, Purity: 69.89%)

### Step-7: Synthesis of N-isopropyl-2-(2-methylimidazo[1,2-a] pyridin-3-yl)-N-(piperidin-4-yl) thiazole-4-carboxamide hydrochloride (SLN5-X-0973)

To a stirred solution of tert-butyl 4-(N-isopropyl-2-(2-methylimidazo[1,2-a] pyridin-3-yl) thiazole-4-carboxamido) piperidine-1-carboxylate (0.2 g, 0.63 mmol) in DCM (2mL) was added 4M HCl in Dioxane (1.0 mL) at 0 °C. The reaction mixture was stirred at rt for 3h. Progress of reaction was monitored by TLC analysis. After completion of reaction, the reaction mixture was concentrated u/vacuum to get crude material, which was purified by reverse phase column chromatography (product eluted at 100% water in ACN) to get N-isopropyl-2-(2-methylimidazo[1,2-a] pyridin-3-yl)-N-(piperidin-4-yl) thiazole-4-carboxamide hydrochloride (0.12 g, 75.66 %) as a yellow solid.
**LCMS [ESI, M+1]:** 384.1 (RT: 0.959 min, Purity: 95.45%),
**HPLC Purity:** RT: 5.32 min, Purity: 95.53%,
**¹H NMR (400 MHz, MeOD):** δ 9.96-9.94 (d, *J*=6.8 Hz, 1H), 8.23 (s, 1H), 8.13 (t, *J*=15.6 Hz, 1H), 8.05-8.03 (d, *J*=8.8 Hz, 1H), 7.68 (t, *J*=13.6 Hz, 1H), 4.34 (bs, 1H), 3.71 (bs, 1H), 3.54-3.51 (d, *J*=10.4 Hz, 2H), 3.22-3.12 (m, 4H), 2.92(s, 3H), 2.0-1.97 (d, *J*=9.6 Hz, 2H), 1.40-1.31 (m, 6H).

### Experimental protocol for compound SLN-X-0974

### Synthesis of 2-(imidazo[1,2-a] pyridin-3-yl)-N-Isopropyl-N-(piperidin-4-yl) thiazole-4-carboxamide hydrochloride

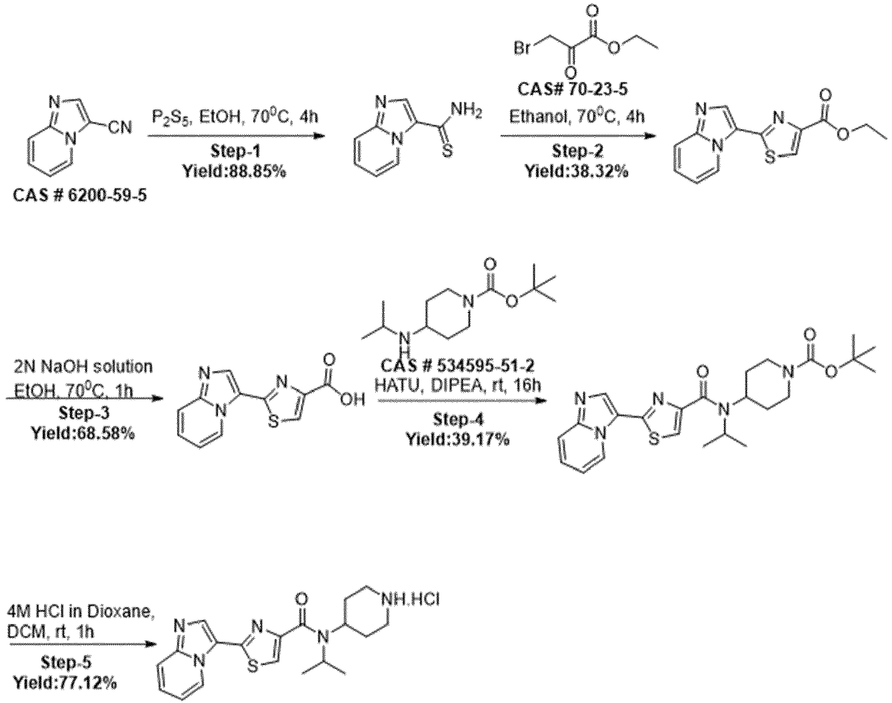

### Step-1: Synthesis of imidazo[1,2-a] pyridine-3-carbothioamide

To a stirred solution of imidazo[1,2-a] pyridine-3-carbonitrile (1.0 g, 6.99 mmol) in DMF (10mL, 10V) was added P₂S₅ (3.1 g, 13.9 mmol) at 0 °C and the reaction mixture was stirred at 70 °C for 4h. Progress of the reaction was monitored by TLC and LCMS analysis. After completion of reaction, the reaction mixture was directly concentrated under vacuum and then diluted with sat. NaHCO₃ solution (300 mL) and extracted with EtOAc (2 X 100 mL). Combined organic layer was dried over Na₂SO₄, filtered and concentrated u/vacuum to get crude material, which was triturated using DCM (20mL) and Diethyl ether (30mL) to get imidazo[1,2-a] pyridine-3-carbothioamide (1.1 g, 88.85 %) as a light yellow solid.

**LCMS [ESI, M+1]:**177.8 (RT: 0.695 min, Purity: 97.07%).

### Step-2: Synthesis of ethyl 2-(imidazo[1,2-a] pyridin-3-yl) thiazole-4-carboxylate

To a stirred solution of imidazo[1,2-a] pyridine-3-carbothioamide (1.1 g, 6.21 mmol) in EtOH (11 mL) was added ethyl 3-bromo-2-oxopropanoate (1.45g, 7.45 mmol) at rt. The resulting reaction mixture was stirred at 70 °C for 4h. Progress of the reaction was monitored by TLC and LCMS analysis. After completion of reaction, the reaction mixture was directly concentrated u/vacuum, then the crude was poured into water (70 mL) and extracted with EtOAc (3 x 40 mL). Combined organic layer was dried over Na₂SO₄, filtered and concentrated u/vacuum to get crude material, which was triturated using DCM (10 mL) and n-pentane (2 x 20 mL) to get ethyl 2-(imidazo[1,2-a] pyridin-3-yl) thiazole-4-carboxylate (0.65 g, 38.32 %) as a light brown solid.
**LCMS [ESI, M+1]:** 273.9 (RT: 1.158min, Purity: 86.12%),

### Step-3: Synthesis of 2-(imidazo[1,2-a] pyridin-3-yl) thiazole-4-carboxylic acid

To a stirred solution of ethyl 2-(imidazo[1,2-a] pyridin-3-yl) thiazole-4-carboxylate (0.65 g, 2.37 mmol) in EtOH (6.5mL, 10V) was added 2N NaOH solution (6.5 mL, 10V) at rt. The resulting reaction mixture was stirred at 70°C for 1h. Progress of the reaction was monitored by TLC and LCMS analysis. After completion of reaction, the reaction mixture was cooled at rt and evaporated u/vacuum to get crude material, which was dissolved in water (50 mL) and extracted with EtOAc (2 x 100 mL). Separated aqueous layer was acidify using 6N HCl solution (pH=~4) to get solid precipitated. Solid material was filtered and dried u/vacuum to get 2-(imidazo[1,2-a] pyridin-3-yl) thiazole-4-carboxylic acid (0.4 g, 68.58 %) as light brown solid.

**LCMS [ESI, M+1]:** 246.1 (RT: 0.956min, Purity: 92.84%).

**¹H NMR (400 MHz, DMSO):** δ 9.82 (d, *J*=6.8 Hz, 1H), 9.04 (s, 1H), 8.63 (s, 1H), 8.07 (d, *J*=9.2 Hz, 1H), 7.99(t, *J*=7.6 Hz, 1H), 7.67 (t, *J*=6.8 Hz, 1H).

### Step-4: Synthesis of tert-butyl 4-(2-(imidazo[1,2-a] pyridin-3-yl)-N-isopropylthiazole-4-carboxamido) piperidine-1-carboxylate

To a stirred solution of 2-(imidazo[1,2-a] pyridin-3-yl) thiazole-4-carboxylic acid (0.4 g, 1.63 mmol) in DMF (4mL, 10V) was added HATU (0.93 g, 2.44 mmol) at rt. After 30min, tert-butyl 4-(isopropyl amino) piperidine-1-carboxylate (0.47 g, 1.95 mmol) and DIPEA (0.83 mL, 4.89 mmol) was added at rt and resulting reaction mixture was stirred at rt for 16 h. Progress of the reaction was monitored by TLC and LCMS analysis. After completion of reaction, the reaction mixture was poured into ice cold water (60 mL) and extracted with EtOAc (3 x 30mL). Combined organic layer was dried over Na₂SO₄, filtered and concentrated u/vacuum to get crude material, which was purified by flash column chromatography (product eluted at 94% EtOAc in Hexane) to get tert-butyl 4-(2-(imidazo[1,2-a] pyridin-3-yl)-N-isopropylthiazole-4-carboxamido) piperidine-1-carboxylate (0.3 g, 39.17 %) as a light yellow liquid.
**LCMS [ESI, M+1]:** 470.1 (RT: 1.707 min, Purity: 94.99%),

### Step-5: Synthesis of 2-(imidazo[1,2-a] pyridin-3-yl)-N-Isopropyl-N-(piperidin-4-yl) thiazole-4-carboxamide hydrochloride (SLN5-X-0974)

To a stirred solution of tert-butyl 4-(2-(imidazo[1,2-a] pyridin-3-yl)-N-isopropylthiazole-4-carboxamido) piperidine-1-carboxylate (0.3 g, 0.63 mmol) in DCM (3mL, 10V) was added 4M HCl in Dioxane (1.5 mL, 5V) at rt. The reaction mixture was stirred at rt for 1h. Progress of reaction was monitored by TLC and LCMS analysis. After completion of reaction, the reaction mixture was concentrated u/vacuum to get crude material, which was purified by trituration using diethyl ether/n-Pentane (1:1, 30mL) and DCM (10mL) to get 2-(imidazo[1,2-a] pyridin-3-yl)-N-Isopropyl-N-(piperidin-4-yl) thiazole-4-carboxamide hydrochloride (0.2 g, 77.12 % yield) as a light yellow solid.
**LCMS [ESI, M+1]:** 370.0 (RT: 0.788min, Purity: 100%),
**HPLC Purity:** RT:3.821min, Purity: 98.53%,
**¹H NMR (400 MHz, MeOD):** δ9.94 (d, *J*=6.4 Hz, 1H), 8.93 (s, 1H), 8.19-8.10 (m, 3H), 7.73 (t, *J*=6.8 Hz, 1H), 4.35 (bs, 1H), 3.71 (bs, 1H), 3.52 (d, *J*=10.8 Hz, 2H), 3.32-3.14 (m, 4H), 1.98 (d, *J*=11.6 Hz, 2H), 1.61-1.38 (m, 6H).

### Experimental protocol for compound SLN-X-0975

### Synthesis (S)-N-isopropyl-2-(2-methylimidazo[1,2-a] pyridin-3-yl)-N-(pyrrolidin-3-yl) thiazole-4-carboxamide hydrochloride

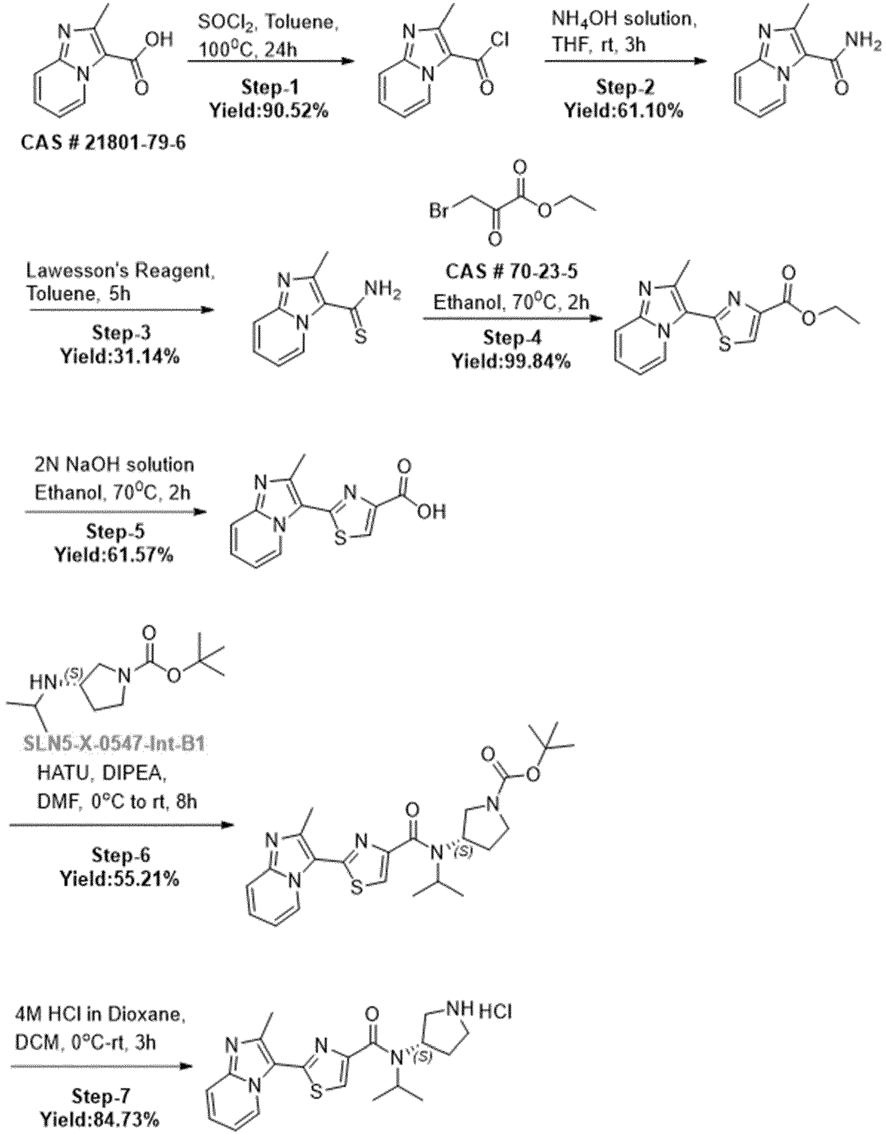

### Steps-1 to 5: Synthesis of Synthesis of 2-(2-methylimidazo[1,2-a]pyridin-3-yl)thiazole-4-carboxylic acid (SLN5-X-0973-Int-A5)

### 2-(2-methylimidazo[1,2-a]pyridin-3-yl)thiazole-4-carboxylic acid was prepared following steps 1 to 5 of the synthesis of compound SLN5-X-0973

### Step-6: Synthesis of tert-butyl (S)-3-(N-isopropyl-2-(2-methylimidazo[1,2-a] pyridin-3-yl) thiazole-4-carboxamido) pyrrolidine-1-carboxylate

To a stirred solution of 2-(2-methylimidazo[1,2-a] pyridin-3-yl) thiazole-4-carboxylic acid (0.18 g, 0.69 mmol) in DMF (1.8 mL, 10V) was added HATU (0.52 g, 1.38 mmol) at 0°C. After 15 min, tert-butyl 4-(isopropylamino) piperidine-1-carboxylate (0.19 g, 0.83mmol) and DIPEA (0.5 mL, 2.77 mmol) was added at 0 °C. The reaction mixture was stirred at rt for 8h. Progress of reaction was monitored by TLC analysis. After completion of reaction, the reaction mixture was diluted with sat. NaHCO₃ solution (50mL) and extracted with EtOAc (2 x 30mL). Combine organic layer were dried over Na₂SO₄, filtered and concentrated u/vacuum to get crude material, which was purified by reverse phase column chromatography (product eluted at 52% MeCN in H₂O) to get tert-butyl (S)-3-(N-isopropyl-2-(2-methylimidazo[1,2-a] pyridin-3-yl) thiazole-4-carboxamido) pyrrolidine-1-carboxylate (0.18 g, 55.21 % yield) as a yellow sticky solid.
**LCMS [ESI, M+1]:** 470.4 (RT: 1.946 min, Purity: 98.75%)

### Step-7: Synthesis of (S)-N-isopropyl-2-(2-methylimidazo[1,2-a] pyridin-3-yl)-N-(pyrrolidin-3-yl) thiazole-4-carboxamide hydrochloride (SLN5-X-0975):

To a stirred solution of tert-butyl (S)-3-(N-isopropyl-2-(2-methylimidazo[1,2-a] pyridin-3-yl) thiazole-4-carboxamido) pyrrolidine-1-carboxylate (0.18g, 0.38mmol) in DCM (1.8mL, 10V) was added 4M HCl in Dioxane (0.9mL, 5V) at 0°C. The reaction mixture was stirred at rt for 3h. Progress of reaction was monitored by TLC analysis. After completion of reaction, the reaction mixture was concentrated u/vacuum to get crude material, which was triturated by using diethyl ether (2 x 10mL) to get (S)-N-isopropyl-2-(2-methylimidazo[1,2-a] pyridin-3-yl)-N-(pyrrolidin-3-yl) thiazole-4-carboxamide hydrochloride (0.12 g, 84.73%) as a yellow solid.
**LCMS [ESI, M+1]:** 370.0 (RT: 0.987 min, Purity: 96.50%),
**HPLC Purity:** RT: 4.567 min, Purity: 97.76%,
**Chiral HPLC Purity:** RT: 8.42 min, Purity: 98.14%,
**¹H NMR (400 MHz, MeOD):** δ 9.94-9.92 (m, 1H), 8.36 (s, 1H), 8.16-8.12 (m, 1H), 8.06-8.03 (m, 1H), 7.70-7.66 (m, 1H), 4.55-4.49 (m, 2H), 3.91-3.85 (m, 1H), 3.75 (dd, J=12, 2.8 Hz, 1H), 3.58-3.52 (m, 1H), 3.32-3.29 (m, 1H), 2.92 (s, 3H), 2.60-2.42 (m, 2H), 1.38-1.34 (m, 6H).

### Experimental protocol for compound SLN5-X-0976:

### Synthesis of (R)-N-isopropyl-2-(2-methylimidazo[1,2-a] pyridin-3-yl)-N-(pyrrolidin-3-yl) thiazole-4-carboxamide hydrochloride

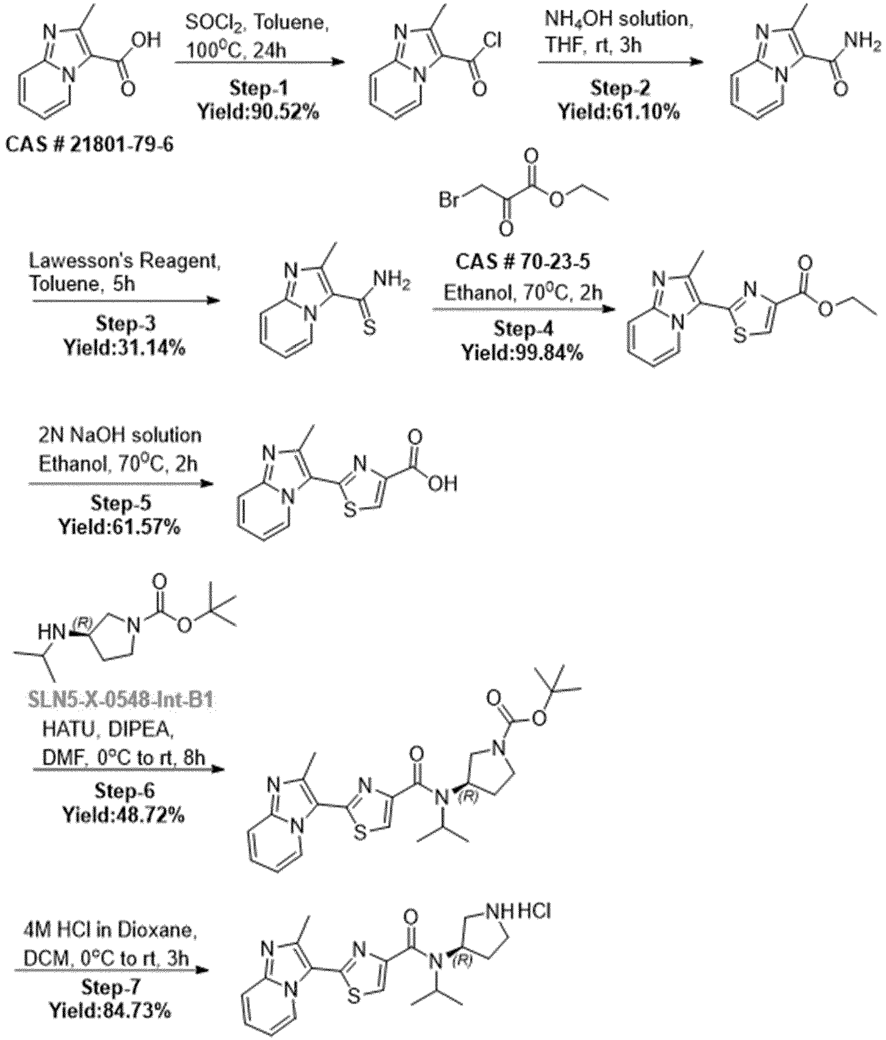

### Steps-1 to 5: Synthesis of Synthesis of 2-(2-methylimidazo[1,2-a]pyridin-3-yl)thiazole-4-carboxylic acid (SLN5-X-0973-Int-A5)

### 2-(2-methylimidazo[1,2-a]pyridin-3-yl)thiazole-4-carboxylic acid was prepared following steps 1 to 5 of the synthesis of compound SLN5-X-0973

### Step-6: Synthesis of tert-butyl (R)-3-(N-isopropyl-2-(2-methylimidazo[1,2-a] pyridin-3-yl) thiazole-4-carboxamido) pyrrolidine-1-carboxylate

To a stirred solution of 2-(2-methylimidazo[1,2-a] pyridin-3-yl) thiazole-4-carboxylic acid (0.17 g, 0.65 mmol) in DMF (1.7 mL, 10V) was added HATU (0.49 g, 7.70 mmol) at 0 °C. After 15 min, tert-butyl (R)-3-(isopropylamino) pyrrolidine-1-carboxylate (0.17 g, 0.78 mmol) and DIPEA (0.46 mL, 2.60 mmol) was added at 0 °C. The reaction mixture was stirred at rt for 8h. Progress of reaction was monitored by TLC analysis. After completion of reaction, the reaction mixture was diluted with sat. NaHCO₃ solution (50mL) and extracted with EtOAc (2 x 30 mL). Combine organic layer were dried over Na₂SO₄, filtered and concentrated u/vacuum to get crude material, which was purified by reverse phase column chromatography (product eluted at 50% MeCN in H₂O) to get tert-butyl (R)-3-(N-isopropyl-2-(2-methylimidazo[1,2-a] pyridin-3-yl) thiazole-4-carboxamido) pyrrolidine-1-carboxylate (0.15 g, 48.72 % yield) as a yellow sticky solid.
**LCMS [ESI, M+1]:** 470.3 (RT: 1.483 min, Purity: 98.15%)

### Step-7: Synthesis of (R)-N-isopropyl-2-(2-methylimidazo[1,2-a] pyridin-3-yl)-N-(pyrrolidin-3-yl) thiazole-4-carboxamide hydrochloride (SLN5-X-0976)

To a stirred solution of tert-butyl (R)-3-(N-isopropyl-2-(2-methylimidazo[1,2-a] pyridin-3-yl) thiazole-4-carboxamido) pyrrolidine-1-carboxylate (0.15 g, 0.31 mmol) in DCM (1.5 mL, 10V) was added 4M HCl in Dioxane (1.5 mL, 10V) at 0°C. The reaction mixture was stirred at rt for 3h. Progress of reaction was monitored by TLC analysis. After completion of reaction, the reaction mixture was concentrated u/vacuum to get crude material, which was triturated by using diethyl ether (2 x 20 mL) to get (R)-N-isopropyl-2-(2-methylimidazo[1,2-a] pyridin-3-yl)-N-(pyrrolidin-3-yl) thiazole-4-carboxamide hydrochloride (0.1 g, 84.73 % yield) as a pale yellow solid.
**LCMS [ESI, M+1]:** 370.3 (RT: 1.120 min, Purity: 98.03%),
**HPLC Purity:** RT: 4.576 min, Purity: 98.20%,
**Chiral HPLC Purity:** RT: 6.94 min, Purity: 96.46%,
**¹H NMR (400 MHz, MeOD):** δ 9.95-9.92 (m, 1H), 8.36 (s, 1H), 8.16-8.12 (m, 1H), 8.05-8.03 (m, 1H), 7.70-7.66 (m, 1H), 4.55-4.49 (m, 2H), 3.83 (t, *J* = 11.2 Hz, 1H), 3.75 (dd, *J* = 12, 2.4 Hz, 1H), 3.57-3.52 (m, 1H), 3.32-3.23 (m, 1H), 2.92 (s, 3H), 2.56-2.45 (m, 2H), 1.38-1.33 (m, 6H).

### Experimental protocol for compound SLN5-X-0977:

### Synthesis of ((S)-2-(imidazo[1,2-a] pyridin-3-yl)-N-Isopropyl-N-(pyrrolidin-3-yl) thiazole-4-carboxamide hydrochloride

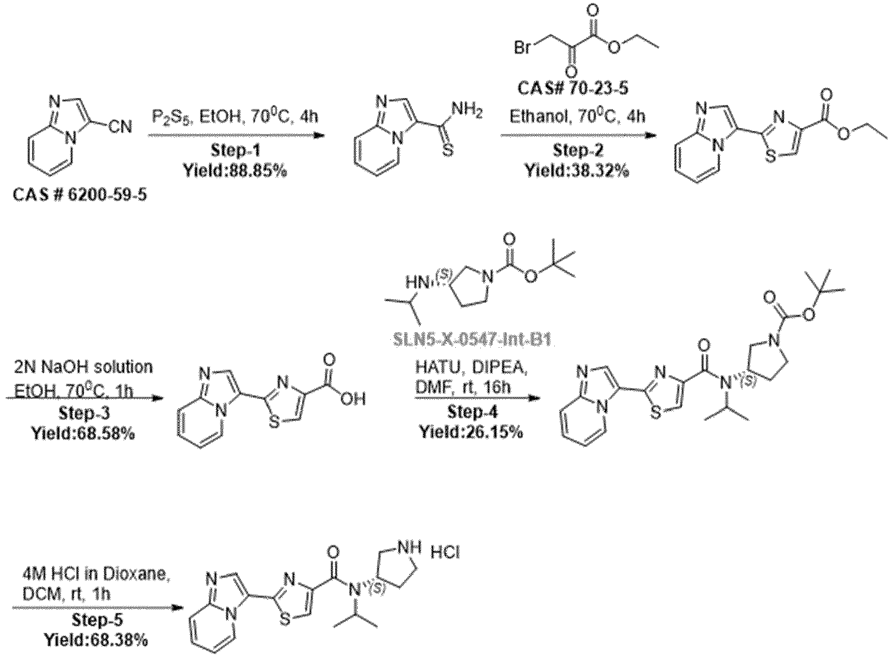

### Steps 1 to 3: Synthesis of 2-(imidazo[1,2-a] pyridin-3-yl) thiazole-4-carboxylic acid

### 2-(imidazo[1,2-a] pyridin-3-yl) thiazole-4-carboxylic acid was prepared following steps 1 to 3 of the synthesis of compound SLN5-X-0974

### Step-4: Synthesis of tert-butyl (S)-3-(2-(imidazo[1,2-a] pyridin-3-yl)-N-isopropylthiazole-4-carboxamido) pyrrolidine-1-carboxylate

To a stirred solution of 2-(imidazo[1,2-a] pyridin-3-yl) thiazole-4-carboxylic acid (0.35 g, 1.02 mmol) in DMF (3.5 mL, 10V) was added HATU (0.8g, 1.53mmol) at rt. After 30 min, tert-butyl (S)-3-(isopropylamino) pyrrolidine-1-carboxylate (0.34 g, 1.02 mmol) and DIPEA (5.2 mL, 3.06 mmol) was added at rt and resulting reaction mixture was stirred at rt for 16h. Progress of the reaction was monitored by TLC and LCMS analysis. After completion of reaction, the reaction mixture was poured into ice cold water (100mL) and extracted with EtOAc (3 x 50 mL). Combined organic layer was dried over Na₂SO₄, filtered and concentrated u/vacuum to get crude material, which was purified by reverse phase column chromatography (product eluted at 75% MeCN in water) to get tert-butyl (S)-3-(2-(imidazo[1,2-a] pyridin-3-yl)-N-isopropylthiazole-4-carboxamido) pyrrolidine-1-carboxylate (0.17 g, 26.15 % yield) as a light yellow liquid.
**LCMS [ESI, M+1]:** 456.1 (RT: 1.629 min, Purity: 96.61%),

### Step-5: Synthesis of (S)-2-(imidazo[1,2-a] pyridin-3-yl)-N-Isopropyl-N-(pyrrolidin-3-yl) thiazole-4-carboxamide hydrochloride (SLN5-X-0977):

To a stirred solution of tert-butyl (S)-3-(2-(imidazo[1,2-a] pyridin-3-yl)-N-isopropylthiazole-4-carboxamido) pyrrolidine-1-carboxylate (0.17 g, 0.37 mmol) in DCM (1.7 mL, 10V) was added 4M HCl in Dioxane (0.85 mL, 5V) at rt. The reaction mixture was stirred at rt for 1h. Progress of reaction was monitored by TLC and LCMS analysis. After completion of reaction, the reaction mixture was concentrated u/vacuum to get crude material, which was purified by trituration using diethyl ether (30mL) and DCM (10 mL) to get pure ((S)-2-(imidazo[1,2-a] pyridin-3-yl)-N-Isopropyl-N-(pyrrolidin-3-yl) thiazole-4-carboxamide hydrochloride (0.1 g, 68.38 % yield) as light brown sticky solid.
**LCMS [ESI, M+1]:** 356.0 (RT: 0.81 min, Purity: 98.18%),
**HPLC Purity:** RT: 3.82 min, Purity: 97.61%,
**Chiral HPLC Purity:** RT: 10.71 min, Purity: 100%
**¹H NMR (400 MHz, MeOD):** δ 9.92 (d, *J*=8.4 Hz, 1H), 8.93 (s, 1H), 8.28 (s, 1H), 8.18-8.09 (m, 2H), 7.72 (t, *J*=8.8 Hz, 1H), 4.59-4.48 (m, 2H), 3.84 (t, *J*=8.4 Hz, 1H), 3.74 (dd, *J*=12, 2 Hz, 1H), 3.57-3.51 (m, 1H), 3.32-3.25 (m, 1H), 2.57-2.44 (m, 2H), 1.40-1.35 (m, 6H).

### Experimental protocol for compound SLN5-X-0978:

### Synthesis of (R)-2-(imidazo[1,2-a] pyridin-3-yl)-N-Isopropyl-N-(pyrrolidin-3-yl) thiazole-4-carboxamide hydrochloride

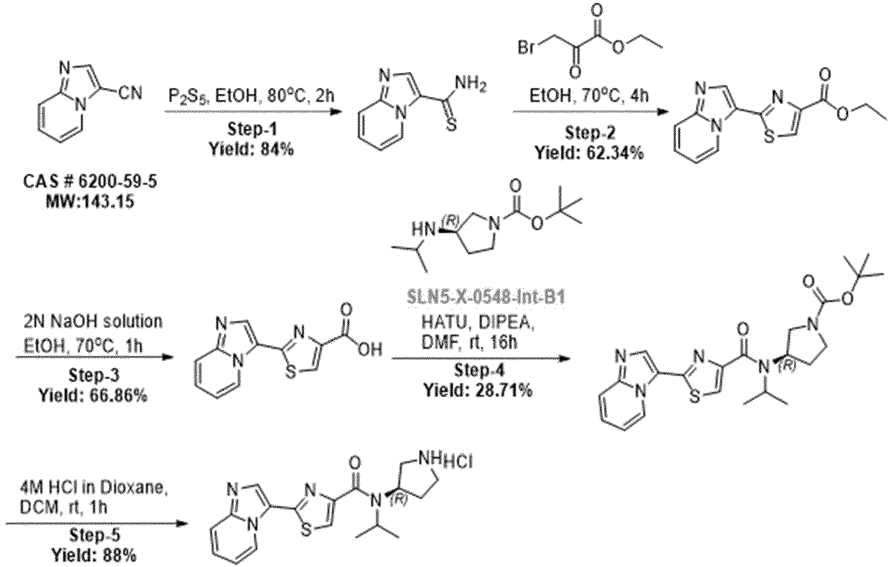

### Steps 1 to 3: Synthesis of 2-(imidazo[1,2-a] pyridin-3-yl) thiazole-4-carboxylic acid

### 2-(imidazo[1,2-a] pyridin-3-yl) thiazole-4-carboxylic acid was prepared following steps 1 to 3 of the synthesis of compound SLN5-X-0974

### Step-4: Synthesis of tert-butyl (R)-3-(2-(imidazo[1,2-a] pyridin-3-yl)-N-isopropylthiazole-4-carboxamido) pyrrolidine-1-carboxylate

To a stirred solution of 2-(imidazo[1,2-a] pyridin-3-yl) thiazole-4-carboxylic acid (1.5 g, 6.11 mmol) in DMF (15mL, 10V) was added HATU (3.48 g, 9.17mmol) at rt. After 30min, tert-butyl (R)-3-(isopropyl amino) pyrrolidine-1-carboxylate (1.39 g, 6.10 mmol) and DIPEA (5.2mL, 3.06 mmol) was added at rt and resulting reaction mixture was stirred at rt for 16h. Progress of the reaction was monitored by TLC and LCMS analysis. After completion of the reaction. The reaction mixture was poured into ice cold water (75 mL) and extracted with EtOAc (2 X 50 mL). The combined organic layer was dried over Na₂SO₄, filtered and concentrated u/vacuum to get crude material, which was purified by normal phase column chromatography (product eluted at 4% MeOH in DCM) to get tert-butyl (R)-3-(2-(imidazo[1,2-a] pyridin-3-yl)-N-isopropylthiazole-4-carboxamido) pyrrolidine-1-carboxylate (0.8 g, 29.62 % yield) as a pale-yellow liquid.
**LCMS [ESI, M+1]:** 456.1 (RT: 1.528 min, Purity: 97.25 %),

### Step-5: Synthesis of (R)-2-(imidazo[1,2-a] pyridin-3-yl)-N-Isopropyl-N-(pyrrolidin-3-yl) thiazole-4-carboxamide hydrochloride (SLN5-X-0978)

To a stirred solution of tert-butyl (R)-3-(2-(imidazo[1,2-a] pyridin-3-yl)-N-isopropylthiazole-4-carboxamido) pyrrolidine-1-carboxylate (0.8 g, 1.75 mmol) in DCM (8 mL, 10V) was added 4M HCl in dioxane (4.0 mL, 5V) at rt. The reaction mixture was stirred at rt for 1h. Progress of reaction was monitored by TLC and LCMS analysis. After completion of reaction, the reaction mixture was concentrated u/vacuum to get crude material, which was purified by reverse phase column chromatography (product eluted in 3% ACN in Water) followed by trituration using diethyl ether (20mL x 2) and DCM (10mL) to get (R)-2-(imidazo[1,2-a] pyridin-3-yl)-N-Isopropyl-N-(pyrrolidin-3-yl) thiazole-4-carboxamide hydrochloride (0.55 g, 88 %) as a light-yellow sticky solid.
**LCMS [ESI, M+1]:** 356.0 (RT: 0.977 min, Purity: 98.53 %),
**HPLC Purity:** RT: 3.35min, Purity: 99.89%,
**Chiral HPLC purity:** RT: 3.78, Purity: 98.88%,
**¹H NMR (400 MHz, MeOD):** δ 9.93 (d, *J* = 6.8Hz, 1H), 8.94 (s, 1H), 8.29 (s, 1H), 8.19 - 8.10 (m, 2H), 7.72 (t, *J* = 7.6Hz, 1H), 4.57 - 4.49 (m, 2H), 3.86 - 3.82 (m, 1H), 3.74 (d, *J* = 8.0Hz, 1H), 3.57 - 3.51 (m, 1H), 3.28 - 3.25 (m, 1H), 2.56 - 2.45 (m, 2H), 1.40 - 1.34 (m, 6H).

### Experimental protocol for compound SLN5-X-0979:

### Synthesis of tert-butyl 4-(N-isopropyl-2-(pyrazolo[1,5-a] pyridin-3-yl)thiazole-4-carboxamido)piperidine-1-carboxylate

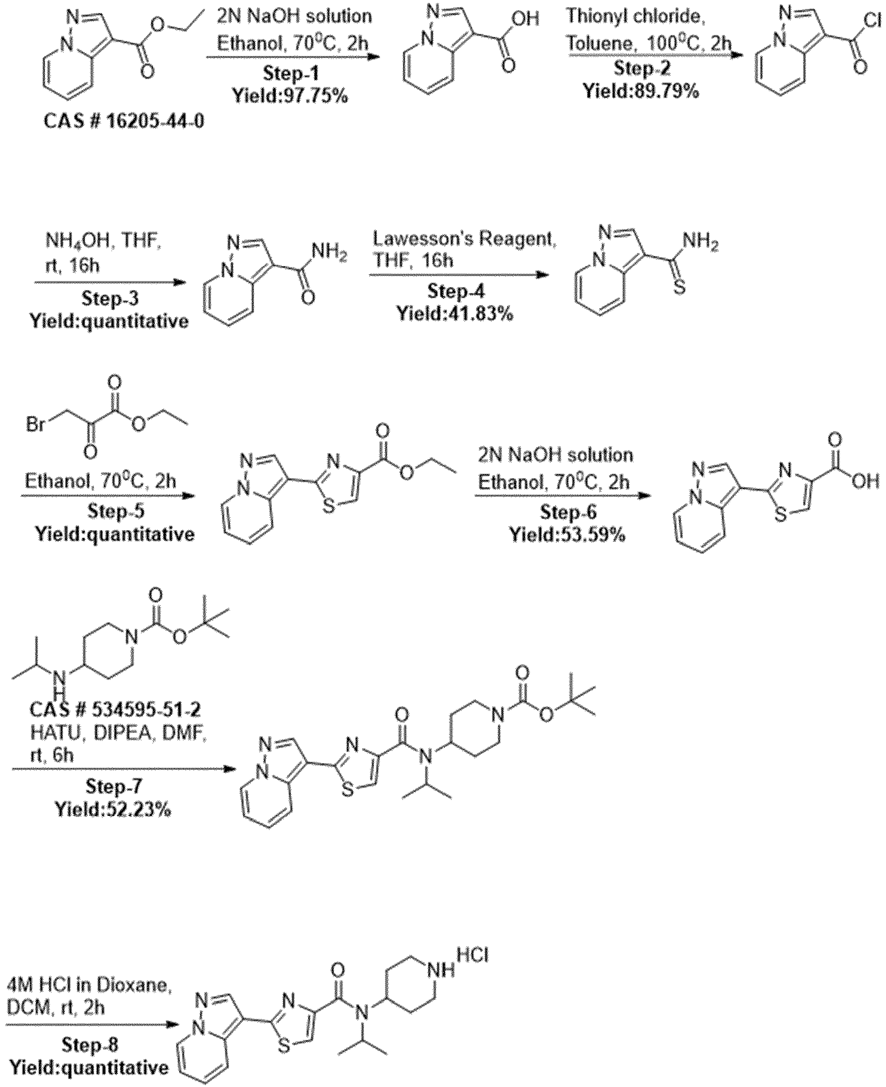

### Step-1: Synthesis of pyrazolo[1,5-a]pyridine-3-carboxylic acid)

To a stirred solution of ethyl pyrazolo[1,5-a] pyridine-3-carboxylate (3.0 g, 15.72 mmol) in EtOH (30 mL, 10V) was added 2N NaOH solution (30 mL, 10V) at rt and the resulting reaction mixture was stirred at 70 °C for 2h. Progress of the reaction was monitored by TLC analysis. After completion of reaction, the reaction mixture was cooled at rt and solvent was evaporated u/vacuum, then reaction mixture was acidify using 1N HCl solution (pH=~4) to get solid precipitate. Solid material was filtered and dried u/vacuum to get pyrazolo[1,5-a] pyridine-3-carboxylic acid (2.5 g, 97.75 % yield) as an off white solid.
**LCMS [ESI, M+1]:** 162.8 (RT: 1.044 min, Purity: 100%)
**¹H NMR (400 MHz, DMSO): δ** 12.46 (s, 1H), 8.86-8.84 (m, 1H), 8.40 (s, 1H), 8.09-8.06 (m, 1H), 7.58-7.54 (m, 1H), 7.15-7.11 (m, 1H).

### Step-2: Synthesis of pyrazolo[1,5-a]pyridine-3-carbonyl chloride

To a stirred solution of pyrazolo[1,5-a] pyridine-3-carboxylic acid (0.5 g, 3.08 mmol) in toluene (6 mL, 12V) was added SOCl₂ (1.16 mL, 16.03 mmol) dropwise at 0 °C. The resulting reaction mixture was stirred at 100 °C for 2 h. Progress of the reaction was monitored by TLC analysis. After completion of reaction, the reaction mixture was cooled at rt and evaporated u/vacuum to get pyrazolo[1,5-a] pyridine-3-carbonyl chloride (1.0 g, 89.79 %) as a light brown solid. Desired product was confirmed on TLC (using KMnO₄ stain) and forwarded to next step. Note that 0.5g x 2 batches were performed for this step and the yield was combined (another batch crude material was mixed for the next step)
**LCMS [ESI, M+1]:** Not supported mass (Purity = 100%)

### Step-3: Synthesis of pyrazolo[1,5-a] pyridine-3-carboxamide

To a stirred solution of pyrazolo[1,5-a] pyridine-3-carbonyl chloride (0.5 g, 27.7 mmol) in THF (5 mL, 10V) was added NH₄OH solution (35% in H₂O) (2.19 mL, 4.3V) dropwise at 0°C. Than the reaction mixture was stirred at rt for 16 h. Progress of the reaction was monitored by TLC analysis. After completion of reaction, reaction mixture was evaporated u/vacuum to get pyrazolo[1,5-a] pyridine-3-carboxamide (2.5g, quantitative yield) as a light brown solid. 0.5 g x 5 batches were performed for this step and the yield was combined.
**LCMS [ESI, M+1]:** 162.2 (RT: 1.207 min, Purity: 98.89%),

### Step-4: Synthesis of pyrazolo[1,5-a]pyridine-3-carbothioamide

To a stirred solution of pyrazolo[1,5-a] pyridine-3-carboxamide (1.25 g, 7.75 mmol) in THF (18 mL, 15V) was added Lawesson's Reagent (4.7 g, 11.63 mmol) at 0°C. The resulting reaction mixture was stirred at rt for 16h. Progress of reaction was monitored by TLC analysis. After completion of reaction, the reaction mixture was diluted in sat. NaHCO₃ solution (200 mL) and extracted with EtOAc (3 X 150 mL). Combine organic layer was washed with sat. NaCl solution (100 mL), dried over Na₂SO₄, filtered and concentrated u/vacuum to get crude material, which was purified by normal phase column chromatography (product eluted at 50 % EtOAc in hexane) to get pyrazolo[1,5-a] pyridine-3-carbothioamide (1.15 g, 41.83% yield) as a yellow solid. 1.25 g x 2 batches were performed for this step and the yield was combined.
**LCMS [ESI, M+1]:** 177.7 (RT: 1.115 min, Purity: 99.55%),

### Step-5: Synthesis of ethyl 2-(pyrazolo[1,5-a] pyridin-3-yl) thiazole-4-carboxylate

To a stirred solution of pyrazolo[1,5-a] pyridine-3-carbothioamide (1.2 g, 6.7 mmol) in EtOH (12 mL, 10V) was added ethyl 3-bromo-2-oxopropanoate (2.63 g, 13.5 mmol) at rt. The resulting reaction mixture was stirred at 70 °C for 2 h. Progress of reaction was monitored by TLC analysis. After completion of reaction, the reaction mixture was cooled at rt and the solvent was evaporated to get crude material. Crude material was dissolved in water (100mL) and extracted with EtOAc (3 X 50 mL). Combine organic layer was dried over Na₂SO₄, filtered and concentrated u/vacuum to get ethyl 2-(pyrazolo[1,5-a] pyridin-3-yl) thiazole-4-carboxylate (2.5 g, quantitative yield) as a yellow solid.
**LCMS [ESI, M+1]:** 273.84 (RT: 1.540 min, Purity: 79.32%),

### Step-6: Synthesis of 2-(pyrazolo[1,5-a] pyridin-3-yl) thiazole-4-carboxylic acid

To a stirred solution of ethyl 2-(pyrazolo[1,5-a] pyridin-3-yl) thiazole-4-carboxylate (2.5 g, 9.14 mmol) in EtOH (25 mL, 10V) was added 2N NaOH solution (25 mL, 10V) at rt. The resulting reaction mixture was stirred at 70°C for 2h. Progress of reaction was monitored by TLC analysis. After completion of reaction, the reaction mixture was cooled at rt and solvent was evaporated to get crude material, which was dissolved in water (30mL) and acidify with 1N HCl (pH=~4) to get solid precipitated. Solid material was filtered and dried u/vacuum to get 2-(pyrazolo[1,5-a] pyridin-3-yl) thiazole-4-carboxylic acid (1.2 g, 53.59 % yield) as a light brown solid.
**LCMS [ESI, M+1]:** 246.08 (RT: 1.644 min, Purity: 99.71%),

### Step-7: Synthesis of tert-butyl 4-(N-isopropyl-2-(pyrazolo[1,5-a] pyridin-3-yl)thiazole-4-carboxamido) piperidine-1-carboxylate

To a stirred solution of 2-(pyrazolo[1,5-a] pyridin-3-yl) thiazole-4-carboxylic acid (0.3 g, 12.2 mmol) in DMF (6 mL, 20V) was added HATU (0.93 g, 2.44 mmol) at 0 °C. After 15 min, tert-butyl 4-(isopropylamino) piperidine-1-carboxylate (0.38 g, 15.9 mmol) and DIPEA (0.9 mL, 4.89 mmol) was added at 0 °C. The reaction mixture was stirred at rt 6 h. Progress of reaction was monitored by TLC analysis. After completion of reaction, the reaction mixture was poured into ice cold water (50 mL) to get solid precipitate. Solid material was filtered and dried u/vacuum to get the crude material, which was purified by normal phase column chromatography (product eluted at 40% EtOAc in Hexane) to get tert-butyl 4-(N-isopropyl-2-(pyrazolo[1,5-a] pyridin-3-yl)thiazole-4-carboxamido)piperidine-1-carboxylate (0.3 g, 52.23 %) as a white solid.

**LCMS [ESI, M+1]:** 470.31 (RT: 2.399 min, Purity: 97.95%)

### Step-8: Synthesis of N-isopropyl-N-(piperidin-4-yl)-2-(pyrazolo[1,5-a]pyridin-3-yl)thiazole-4-carboxamide hydrochloride (SLN5-X-0979):

To a stirred solution of tert-butyl 4-(N-isopropyl-2-(pyrazolo[1,5-a] pyridin-3-yl) thiazole-4-carboxamido) piperidine-1-carboxylate (0.3 g, 0.63 mmol) in DCM (3mL, 10V) was added 4M HCl in Dioxane (3 mL, 5V) at 0 °C. The reaction mixture was stirred at rt for 2 h. Progress of reaction was monitored by TLC analysis. After completion of reaction, the reaction mixture was concentrated u/vacuum to get crude material, which was triturated using diethyl ether to get N-isopropyl-N-(piperidin-4-yl)-2-(pyrazolo[1,5-a]pyridin-3-yl)thiazole-4-carboxamide hydrochloride (0.29 g, quantitative yield) as a pale yellow solid.
**LCMS [ESI, M+1]:** 370.1 (RT: 1.167 min, Purity: 97.58%),
**HPLC Purity:** RT: 7.87 min, Purity: 95.59%,
**¹H NMR (400 MHz, MeOD):** δ 8.69 (d, *J*=6.8 Hz, 1H), 8.52 (s, 1H), 8.32 (d, *J*=8.4 Hz, 1H), 7.88 (s, 1H), 7.54 (t, *J*=15.2 Hz, 1H), 7.13-7.09 (m, 1H), 4.43 (bs, 1H), 3.67 (bs, 1H), 3.51 (d, *J*=1.6 Hz, 2H), 3.16-3.14 (m, 4H), 1.97-1.95 (m, 2H),1.37-1.30 (m, 6H).

### Experimental protocol for Compound SLN5-X-0980:

### Synthesis of (S)-N-isopropyl-2-(pyrazolo[1,5-a] pyridin-3-yl)-N-(pyrrolidin-3-yl) thiazole-4-carboxamide hydrochloride

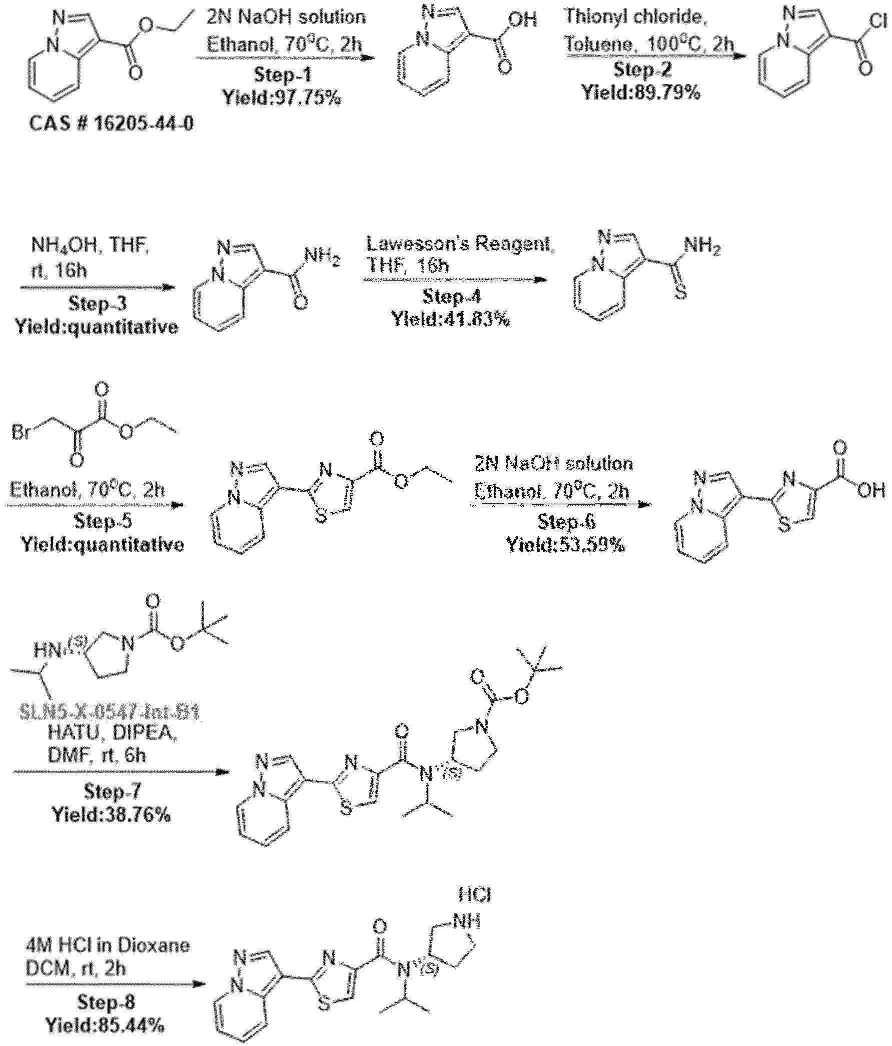

### Steps-1 to 6: Synthesis of Synthesis of 2-(pyrazolo[1,5-a] pyridin-3-yl) thiazole-4-carboxylic acid (SLN5-X-0979-IntA6)

### 2-(pyrazolo[1,5-a] pyridin-3-yl) thiazole-4-carboxylic acid was prepared following steps 1 to 6 of the synthesis of compound SLN5-X-0979

### Step-7: Synthesis of tert-butyl (S)-3-(N-isopropyl-2-(pyrazolo[1,5-a]pyridin-3-yl)thiazole-4-carboxamido)pyrrolidine-1-carboxylate

To a stirred solution of 2-(pyrazolo[1,5-a] pyridin-3-yl) thiazole-4-carboxylic acid (0.25 g, 1.01 mmol) in DMF (5mL, 20V) was added HATU (0.77 g, 2.03 mmol) at 0 °C. After 15min, tert-butyl (S)-3-(isopropylamino) pyrrolidine-1-carboxylate (0.3 g, 1.32 mmol) and DIPEA (0.75 mL, 4.07mmol) was added at 0 °C. The reaction mixture was stirred at rt 6h. Progress of reaction was monitored by TLC analysis. After completion of reaction, the reaction mixture was poured into ice cold water (50mL) to get solid precipitated. Solid material was filtered and dried u/reduce pressure to get crude material, which was purified by normal phase column chromatography (product eluted at 40% EtOAc in hexane) to get tert-butyl (S)-3-(N-isopropyl-2-(pyrazolo[1,5-a] pyridin-3-yl) thiazole-4-carboxamido) pyrrolidine-1-carboxylate (0.18 g, 38.76 % yield) as a brown sticky solid.
**LCMS [ESI, M-56]:** 400.3 (RT: 2.266 min, Purity: 87.10 %)

### Step-8: Synthesis of (S)-N-isopropyl-2-(pyrazolo[1,5-a]pyridin-3-yl)-N-(pyrrolidin-3-yl)thiazole-4-carboxamide hydrochloride (SLN5-X-0980)

To a stirred solution of tert-butyl (S)-3-(N-isopropyl-2-(pyrazolo[1,5-a] pyridin-3-yl) thiazole-4-carboxamido) pyrrolidine-1-carboxylate (0.18 g, 0.39 mmol) in DCM (1.8 mL, 10V) was added 4M HCl in Dioxane (0.9 mL, 5V) at rt. The reaction mixture was stirred at rt for 2h. Progress of reaction was monitored by TLC analysis. After completion of reaction, the reaction mixture was concentrated u/vacuum to get crude material, which was triturated using diethyl ether (3 x 20 mL) to get (S)-N-isopropyl-2-(pyrazolo[1,5-a] pyridin-3-yl)-N-(pyrrolidin-3-yl) thiazole-4-carboxamide hydrochloride (0.12 g, 85.44 %) as a pale yellow solid.
**LCMS [ESI, M+1]:** 356.0 (RT: 1.17 min, Purity: 97.24%),
**HPLC Purity:** RT: 7.82 min, Purity: 95.40%,
**Chiral HPLC Purity:** RT: 9.89min, Purity: 95.08%,
**¹H NMR (400 MHz, MeOD):** δ 8.71-8.69 (m, 1H), 8.52 (s, 1H), 8.33-8.30 (m, 1H), 7.92 (s, 1H), 7.56-7.52 (m, 1H), 7.14-7.10 (m, 1H), 4.69-4.66 (m, 1H), 4.47-4.45 (m, 1H), 3.84 (t, *J*=5.2 Hz, 1H), 3.72-3.69 (m, 1H), 3.54-3.49 (m, 1H), 3.32-3.26 (m, 1H), 2.54-2.43 (m, 2H), 1.39-1.34 (m, 6H).

### Experimental protocol for compound SLN5-X-0981:

### Synthesis (R)-N-isopropyl-2-(pyrazolo[1,5-a] pyridin-3-yl)-N-(pyrrolidin-3-yl) thiazole-4-carboxamide hydrochloride

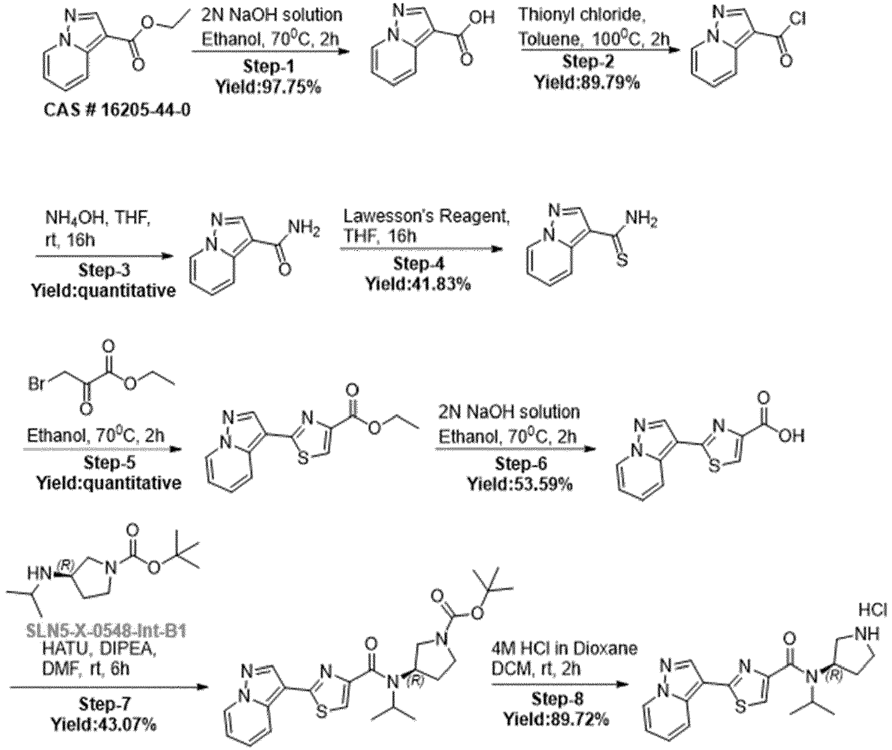

### Steps-1 to 6: Synthesis of Synthesis of 2-(pyrazolo[1,5-a] pyridin-3-yl) thiazole-4-carboxylic acid (SLN5-X-0979-Int-A6)

### 2-(pyrazolo[1,5-a] pyridin-3-yl) thiazole-4-carboxylic acid was prepared following steps 1 to 6 of the synthesis of compound SLN5-X-0979

### Step-7: Synthesis of tert-butyl (R)-3-(N-isopropyl-2-(pyrazolo[1,5-a] pyridin-3-yl) thiazole-4-carboxamido) pyrrolidine-1-carboxylate

To a stirred solution of 2-(pyrazolo[1,5-a]pyridin-3-yl)thiazole-4-carboxylic acid (0.25 g, 1.01 mmol) in DMF (5 mL, 20V) was added HATU (0.77 g, 2.03 mmol) at 0 °C. After 15 min, tert-butyl (R)-3-(isopropylamino)pyrrolidine-1-carboxylate (0.3 g, 1.32 mmol) and DIPEA (0.75 mL, 4.07 mmol) at 0 °C. The reaction mixture was stirred at rt for 6 h. Progress of reaction was monitored by TLC analysis. After completion of reaction, the reaction mixture was poured into ice cold water (50 mL) to get solid precipitate. Solid material was filtered and dried u/vacuum to get crude material, which was purified by normal phase column chromatography (product eluted at 50% EtOAc in hexane) to get tert-butyl (R)-3-(N-isopropyl-2-(pyrazolo[1,5-a] pyridin-3-yl) thiazole-4-carboxamido) pyrrolidine-1-carboxylate (0.2 g, 43.07 % yield) as a yellow Solid.
**LCMS [ESI, M-56]:** 400.3 (RT: 2.20min, Purity: 90.12%)

### Step-8: Synthesis of (R)-N-isopropyl-2-(pyrazolo[1,5-a] pyridin-3-yl)-N-(pyrrolidin-3-yl) thiazole-4-carboxamide hydrochloride

To a stirred solution of tert-butyl (R)-3-(N-isopropyl-2-(pyrazolo[1,5-a] pyridin-3-yl) thiazole-4-carboxamido) pyrrolidine-1-carboxylate (0.2 g, 0.43 mmol) in DCM (2 mL, 10V) was added 4M HCl in Dioxane (1 mL, 5V) at 0 °C. The reaction mixture was stirred at rt for 2 h. Progress of reaction was monitored by TLC analysis. After completion of reaction, the reaction mixture was concentrated u/vacuum to get crude material, which was triturated using diethyl ether (3 x 10mL) to get (R)-N-isopropyl-2-(pyrazolo[1,5-a] pyridin-3-yl)-N-(pyrrolidin-3-yl) thiazole-4-carboxamide hydrochloride (0.14 g, 89.72 %) as a yellow brown solid.
**LCMS [ESI, M+1]:** 356.1 (RT: 1.16 min, Purity: 98.79%),
**HPLC Purity:** RT: 4.01 min, Purity: 97.32%,
**Chiral HPLC Purity:** RT: 9.19 min, Purity: 95.10%,
**¹H NMR (400 MHz, MeOD):** δ 8.70 (d, *J*=6.8 Hz, 1H), 8.52 (s, 1H), 8.32 (d, *J*=8.8 Hz, 1H), 7.92 (s, 1H), 7.57-7.52 (m, 1H), 7.14-7.10 (m, 1H), 4.71-4.64 (m, 1H), 4.47-4.45 (m, 1H), 3.86 (t, *J*=2.4 Hz, 1H), 3.72-3.69 (m, 1H), 3.55-3.49 (m, 1H), 3.29-3.23 (m, 1H), 2.57-2.42 (m, 2H), 1.39-1.32 (m, 6H).

### Experimental protocol for compound SLN5-X-1290:

### Synthesis of 2-(imidazo[1,2-a] pyridin-3-yl)-N-Methyl-N-(piperidin-4-yl)-1H-imidazole-4-carboxamide hydrochloride

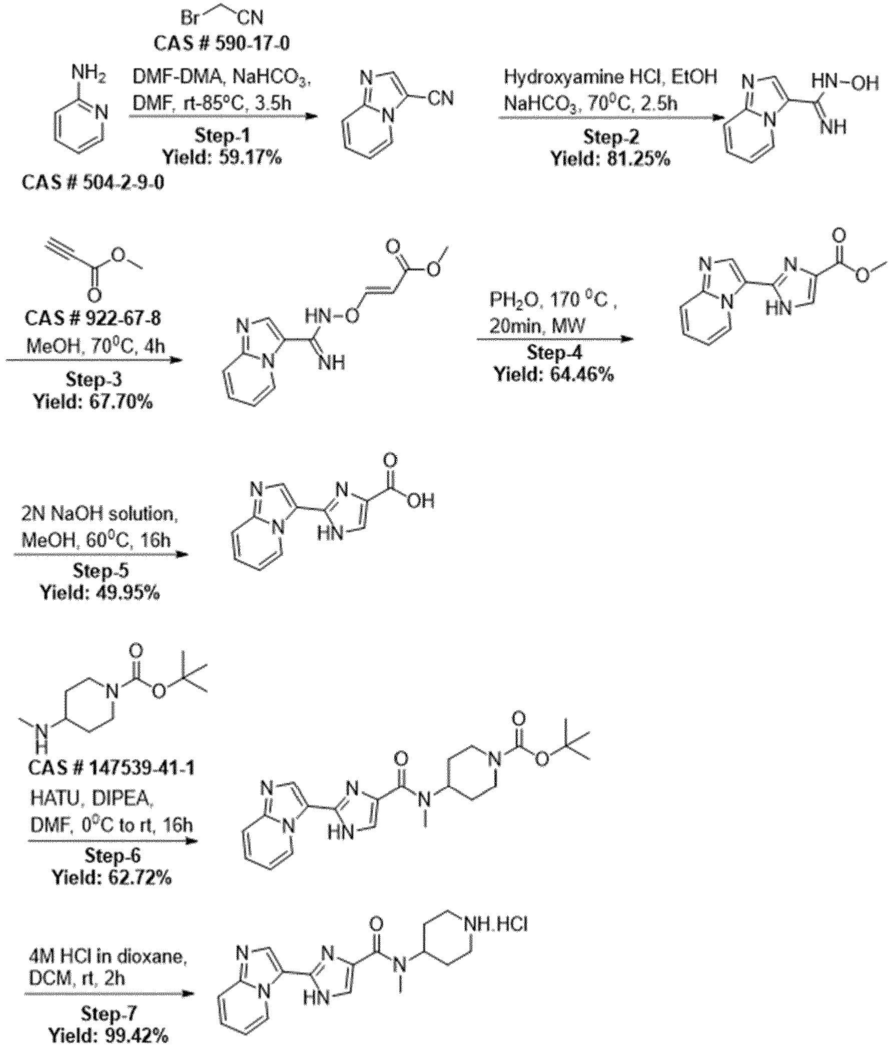

### Step-1: Synthesis of imidazo[1,2-a] pyridine-3-carbonitrile:

To a stirred solution of pyridin-2-amine (10.0 g, 106.20 mmol) in DMF (100 mL, 10V) was added N,N-Dimethylformamide Dimethyl Acetal (35.56 mL, 265.6 mmol) at rt. The resulting reaction mixture was stirred at 65 °C for 2 h. than reaction mixture was cooled at rt and 2-bromoacetonitrile (9.63 mL, 138.0 mmol) and NaHCO₃ (13.38 g, 159.3 mmol) was added at rt. The resulting reaction mixture was stirred at 85 °C for 1.5 h. Progress of the reaction was monitored by TLC analysis. After completion of reaction, the reaction mixture was cooled at rt, diluted with water (200 mL) and extracted with EtOAc (3 X 100mL). Combine organic layer were dried over Na₂SO₄, filtered and concentrated u/vacuum to get crude material, which was purified by normal phase column chromatography (product eluted at 100 % DCM in MeOH) to get imidazo[1,2-a] pyridine-3-carbonitrile (9.0 g, 59.17 % yield) as a light brown solid.
**LCMS [ESI, M+1]:** 143.9 (RT: 1.036 min, Purity: 100%),

### Step-2: Synthesis of N-hydroxyimidazo[1,2-a] pyridine-3-carboximidamide

To a stirred solution of Hydroxyamine HCl (3.64 g, 52.3mmol) in H₂O (15mL, 5V) was added NaHCO₃ (4.40 g, 52.3 mmol) at 0°C. After 15 min, imidazo[1,2-a] pyridine-3-carbonitrile (3.0 g, 20.95 mmol) in EtOH (30 ml, 10V)) was added at rt. The resulting reaction mixture was stirred at 70°C for 2.5 h. Progress of the reaction was monitored by TLC analysis. After completion of reaction, reaction mixture was evaporated u/vacuum to get crude material, which was purified by normal phase column chromatography (product eluted at 10% MeOH in DCM) to get N-hydroxyimidazo[1,2-a] pyridine-3-carboximidamide (3.0 g, 81.25 % yield) as an off white solid.
**LCMS [ESI, M+1]:** 176.9 (RT: 0.273 min, Purity: 60.26% & RT: 0.320 min, Purity: 39.74%),

### Step-3: Synthesis of methyl (E)-3-((imidazo[1,2-a] pyridine-3-carboximidamido)

### oxy) acrylate

To a stirred solution of N-hydroxyimidazo[1,2-a] pyridine-3-carboximidamide (1.5g, 8.5 mmol) in MeOH (15 mL, 10V) was added methyl propiolate (2.14 g, 25.5 mmol) at rt. The resulting reaction mixture was stirred at 70 °C for 4 h. Progress of reaction was monitored by TLC analysis. After completion of reaction, reaction mixture was evaporated u/vacuum to get crude material, which was purified by normal phase column chromatography (product eluted at 5% MeOH in DCM) to get methyl (E)-3-((imidazo[1,2-a] pyridine-3-carboximidamido) oxy) acrylate (3.0 g, 67.70 % yield) as a brown solid.

Note: Performed on 1.5 g x 2 batches for this step, and yield was combined.
**LCMS [ESI, M+1]:** 260.88 (RT: 1.076 min, Purity: 72.78% & 1.103 min, Purity: 25.56%),

### Step-4: Synthesis of methyl 2-(imidazo[1,2-a] pyridin-3-yl)-1H-imidazole-4-carboxylate

Methyl (E)-3-((imidazo[1,2-a] pyridine-3-carboximidamido) oxy) acrylate (0.5 g, 1.92 mmol) in PH₂O (2.5 mL, 5V) was stirred at 170°C for 20 min in microwave irradiation. Progress of reaction was monitored by TLC analysis. After completion of reaction, the reaction mixture was concentrated u/vacuum, add n-pentane to get solid material, which was purified by normal phase column chromatography (product eluted at 3% MeOH in DCM) to get methyl 2-(imidazo[1,2-a] pyridin-3-yl)-1H-imidazole-4-carboxylate (1.8 g, 64.46 % yield) as a light brown solid.

**Note:** 6 reactions were carried out in parallel (each of 0.5 g).

**LCMS [ESI, M+1]:** 242.9 (RT: 0.957 min, Purity: 90.57%),

### Step-5: Synthesis of 2-(imidazo[1,2-a] pyridin-3-yl)-1H-imidazole-4-carboxylic acid

To a stirred solution of methyl 2-(imidazo[1,2-a] pyridin-3-yl)-1H-imidazole-4-carboxylate (1.7 g, 7.01 mmol) in MeOH (17 mL, 10V) was added 2N NaOH solution (17 mL, 10V) at rt. The resulting reaction mixture was stirred at 70°C for 16h. Progress of reaction was monitored by TLC analysis. After completion of reaction, the reaction mixture was cooled at rt and concentrate u/vacuum to get crude material, which was dissolved in water and acidify using 1N HCl solution (pH=~4) to get solid precipitated. Solid material was filtered and dried u/vacuum to get 2-(imidazo[1,2-a] pyridin-3-yl)-1H-imidazole-4-carboxylic acid (0.8 g, 49.95 % yield) as a light brown solid.

**LCMS [ESI, M+1]:** 228.8 (RT: 0.783 min, Purity: 95.78%),
**¹H NMR (400 MHz, DMSO):** δ 13.52 (bs, 2H), 10.01 (d, *J* = 6.8 Hz, 1H), 8.79 (s, 1H), 8.08-7.96 (m, 3H), 7.64 (t, *J* = 13.2 Hz, 1H).

### Step-6: Synthesis of tert-butyl 4-(2-(imidazo[1,2-a] pyridin-3-yl)-N-methyl-1H-imidazole-4-carboxamido) piperidine-1-carboxylate

To a stirred solution of 2-(imidazo[1,2-a] pyridin-3-yl)-1H-imidazole-4-carboxylic acid (0.3 g, 1.31 mmol) in DMF (3 mL, 10V) was added HATU (0.99 g, 2.62 mmol) at 0 °C. After 15 min, tert-butyl 4-(methylamino) piperidine-1-carboxylate (0.36 g, 1.70 mmol) and DIPEA (0.94 mL, 5.25 mmol) was added at 0 °C. The reaction mixture was stirred at rt 16h. Progress of reaction was monitored by TLC analysis. After completion of reaction, the reaction mixture was diluted with ice cold water (50 mL) and extracted with EtOAc (2 X 30 mL). Combine organic layer were dried over Na₂SO₄, filtered and concentrated u/vacuum to get crude material, which was purified by reverse phase column chromatography (product eluted at 52% MeCN in H₂O) to get tert-butyl 4-(2-(imidazo[1,2-a] pyridin-3-yl)-N-methyl-1H-imidazole-4-carboxamido) piperidine-1-carboxylate (0.35 g, 62.72 % yield) as a brown solid.
**LCMS [ESI, M+1]:** 425.4 (RT: 1.757 min, Purity: 100%)

### Step-7: Synthesis of 2-(imidazo[1,2-a] pyridin-3-yl)-N-Methyl-N-(piperidin-4-yl)-1H-imidazole-4-carboxamide hydrochloride (SLN5-X-1290):

To a stirred solution of tert-butyl 4-(2-(imidazo[1,2-a] pyridin-3-yl)-N-methyl-1H-imidazole-4-carboxamido) piperidine-1-carboxylate (0.35 g, 0.82 mmol) in DCM (3.5 mL, 10V) was added 4M HCl in Dioxane (1.75 mL, 5V) at 0 °C. The reaction mixture was stirred at rt for 2h. Progress of reaction was monitored by TLC analysis. After completion of reaction, the reaction mixture was concentrated u/vacuum to get crude material, which was triturated by using diethyl ether (10 ml x 3) to get 2-(imidazo[1,2-a] pyridin-3-yl)-N-Methyl-N-(piperidin-4-yl)-1H-imidazole-4-carboxamide hydrochloride (0.3 g, quantitative yield) as a brown solid.
**LCMS [ESI, M+1]:** 324.8 (RT: 0.495 min, Purity: 99.49%),
**HPLC Purity:** RT: 5.43 min, Purity: 100%,
**¹H NMR (400 MHz, MeOD):** δ 9.87 (d, *J* = 7.2 Hz, 1H), 8.62 (s, 1H), 8.19-8.11 (m, 2H), 8.02 (bs, 1H), 7.74-7.71 (m, 1H), 4.75 (bs, 1H), 3.57 (d, *J* = 12.4 Hz, 2H), 3.42 (bs, 2H), 3.19-2.85 (m, 3H), 2.25-2.08 (m, 4H).

### Experimental protocol for SLN5-X-1291:

### Synthesis of 2-(imidazo[1,2-a] pyridin-3-yl)-N-Isopropyl-N-(piperidin-4-yl)-1H-imidazole-4-carboxamide hydrochloride (SLN5-X-1291)

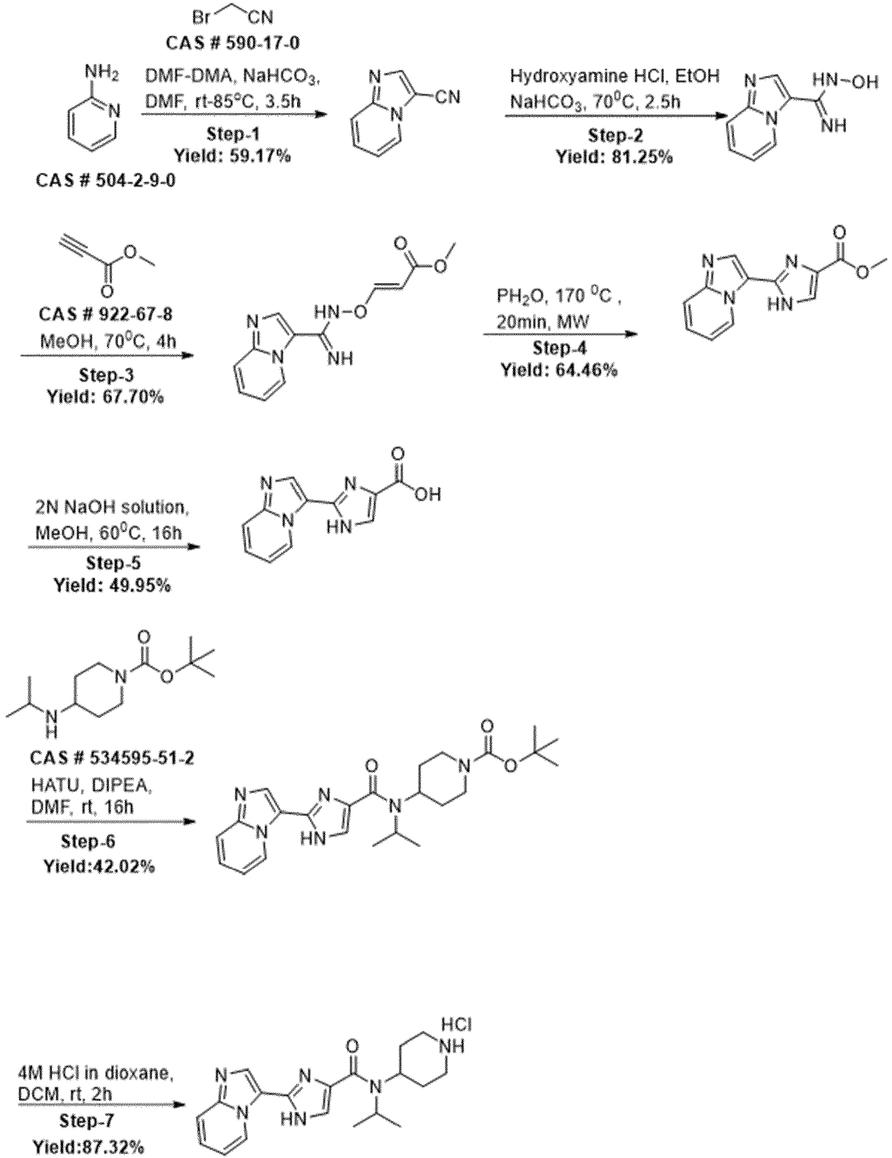

### Steps-1 to 5: Synthesis of 2-(imidazo[1,2-a] pyridin-3-yl)-1H-imidazole-4-carboxylic acid (SLN5-X-1290-Int-A5)

### 2-(Imidazo[1,2-a] pyridin-3-yl)-1H-imidazole-4-carboxylic acid was prepared following steps 1 to 5 of the synthesis of compound SLN5-X-1290

### Step-6: Synthesis of tert-butyl 4-(2-(imidazo[1,2-a] pyridin-3-yl)-N-isopropyl-1H-imidazole-4-carboxamido) piperidine-1-carboxylate

To a stirred solution of 2-(imidazo[1,2-a] pyridin-3-yl)-1H-imidazole-4-carboxylic acid (0.3 g, 1.31 mmol) in DMF (3mL, 10V) was added HATU (0.99 g, 2.62 mmol) at 0 °C. After 15min, tert-butyl 4-(isopropylamino) piperidine-1-carboxylate (0.41 g, 1.70 mmol) and DIPEA (0.94 mL, 5.25 mmol) was added at 0 °C. The reaction mixture was stirred at rt 16 h. Progress of reaction was monitored by TLC analysis. After completion of reaction, the reaction mixture was diluted with ice cold water (50 mL) and extracted with EtOAc (3 X 30 mL). Combine organic layer were dried over Na₂SO₄, filtered and concentrated u/vacuum to get crude material, which was purified by reverse phase column chromatography (product eluted at 52% MeCN in H₂O) to get tert-butyl 4-(2-(imidazo[1,2-a] pyridin-3-yl)-N-isopropyl-1H-imidazole-4-carboxamido) piperidine-1-carboxylate (0.25 g, 42.02 % yield) as a brown solid.
**LCMS [ESI, M+1]:** 452.9 (RT: 1.35 min, Purity: 89.51%)

### Step-7: Synthesis of 2-(imidazo[1,2-a] pyridin-3-yl)-N-Isopropyl-N-(piperidin-4-yl)-1H-imidazole-4-carboxamide hydrochloride (SLN5-X-1291):

To a stirred solution of tert-butyl 4-(2-(imidazo[1,2-a] pyridin-3-yl)-N-isopropyl-1H-imidazole-4-carboxamido) piperidine-1-carboxylate (0.25 g, 0.55 mmol) in DCM (2.5 mL, 10V) was added 4M HCl in dioxane (1.25 mL, 5V) at 0 °C. The reaction mixture was stirred at rt for 2h. Progress of reaction was monitored by TLC analysis. After completion of reaction, the reaction mixture was concentrated u/vacuum to get crude material, which was triturated using diethyl ether (10 mL x 2) to get 2-(imidazo[1,2-a] pyridin-3-yl)-N-Isopropyl-N-(piperidin-4-yl)-1H-imidazole-4-carboxamide hydrochloride (0.17 g, 87.32 % yield) as a light brown solid.
**LCMS [ESI, M+1]:** 352.80 (RT: 0.915 min, Purity: 95.35%),
**HPLC Purity:** RT: 6.75 min, Purity: 96.32%,
**¹H NMR (400 MHz, MeOD):** δ 10.06 (s, 1H), 8.53 (s, 1H), 8.16-8.07 (m, 2H), 7.80 (s, 1H), 7.72-7.68 (m, 1H), 5.10 (bs, 2H), 3.65 (bs, 1H), 3.51 (d, *J* = 10 Hz, 2H), 3.18-3.14 (m, 3H), 1.96 (bs, 2H), 1.44 (d, *J* = 5.2 Hz, 6H).

### Experimental protocol for compound SLN5-X-1292:

### Synthetic scheme of SLN5-X-1292:

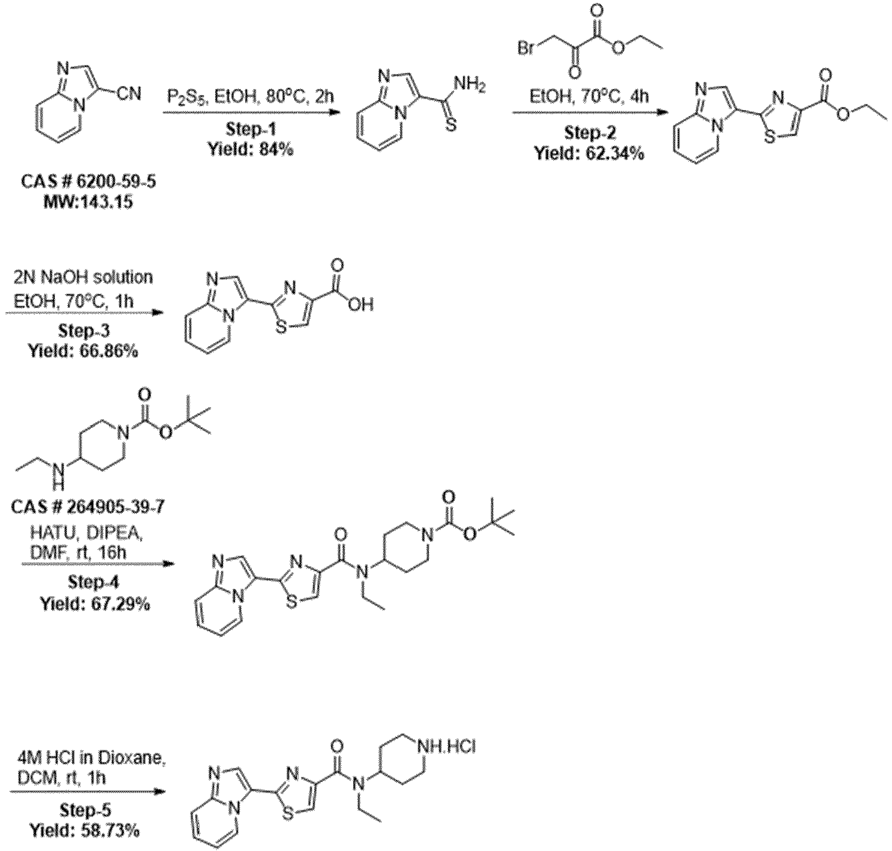

### Steps 1 to 3: Synthesis of 2-(imidazo[1,2-a] pyridin-3-yl) thiazole-4-carboxylic acid

### 2-(imidazo[1,2-a] pyridin-3-yl) thiazole-4-carboxylic acid was prepared following steps 1 to 3 of the synthesis of compound SLN5-X-0974

### Step-4: Synthesis of tert-butyl 4-(N-ethyl-2-(imidazo[1,2-a]pyridin-3-yl)thiazole-4-carboxamido)piperidine-1-carboxylate

To a stirred solution of 2-(imidazo[1,2-a] pyridin-3-yl)thiazole-4-carboxylic acid (0.8 g, 3.26 mmol) in DMF (8 mL) was added HATU (1.86 g, 4.89 mmol) and DIPEA (1.68 mL, 9.78 mmol) at rt. After 30min, tert-butyl 4-(ethylamino)piperidine-1-carboxylate (0.89 g, 3.91 mmol) was added at rt and resulting reaction mixture was stirred at rt for 16 h. Progress of the reaction was monitored by TLC. After completion of reaction, the reaction mixture was poured into ice cold water (70 mL) and extracted with EtOAc (3 X 70 mL). Combined organic layer was dried over Na₂SO₄, filtered and concentrated u/vacuum to get crude material, which was purified by reverse phase column chromatography (product eluted at 65% MeCN in water) to get tert-butyl 4-(N-ethyl-2-(imidazo[1,2-a]pyridin-3-yl)thiazole-4-carboxamido)piperidine-1-carboxylate (1.0 g, 67.29 %) as a brown solid.
**LCMS [ESI, M+1]:** 456.2 (RT: 1.46 min, Purity: 88.20 %),

### Step-5: Synthesis of N-ethyl-2-(imidazo[1,2-a]pyridin-3-yl)-N-(piperidin-4-yl)thiazole-4-carboxamide hydrochloride (SLN5-X-1292):

To a stirred solution of tert-butyl 4-(N-ethyl-2-(imidazo[1,2-a]pyridin-3-yl)thiazole-4-carboxamido)piperidine-1-carboxylate (1.9 g, 2.19 mmol) in DCM (10 mL, 10V) was added 4M HCl in Dioxane (5 mL, 5V) at rt. The reaction mixture was stirred at rt for 1h. Progress of reaction was monitored by TLC. After completion of reaction, the reaction mixture was concentrated u/vacuum to get crude material, which was diluted with water (20mL) and extracted EtOAc (3 X 15 mL), separated aqueous layer were lyophilized to get N-ethyl-2-(imidazo[1,2-a]pyridin-3-yl)-N-(piperidin-4-yl)thiazole-4-carboxamide hydrochloride (0.96 g, 58.73 %) as a brown solid.
**LCMS [ESI, M+1]:** 355.8(RT: 0.889min, Purity:99.46%),
**HPLC Purity:** RT: 4.255min, Purity: 96.81%,
**¹H NMR (400 MHz, MeOD):** δ 9.90 (d, *J*=6.8 Hz, 1H), 8.95 (s, 1H), 8.25 (s, 1H), 8.20-8.11 (m, 2H), 7.76-7.72 (m, 1H), 4.40-4.27 (m, 1H), 3.66-3.47 (m, 4H), 3.27-3.15 (m, 1H), 3.02 (bs, 1H), 2.60-2.57 (m, 1H), 2.17-2.15 (m, 3H), 1.39-1.29 (m, 3H).

### Experimental protocol for compound SLN5-X-1293:

### Synthesis of 2-(imidazo[1,2-a]pyridin-3-yl)-N-methyl-N-(piperidin-4-yl)thiazole-4-carboxamide hydrochloride (SLN5-X-1293)

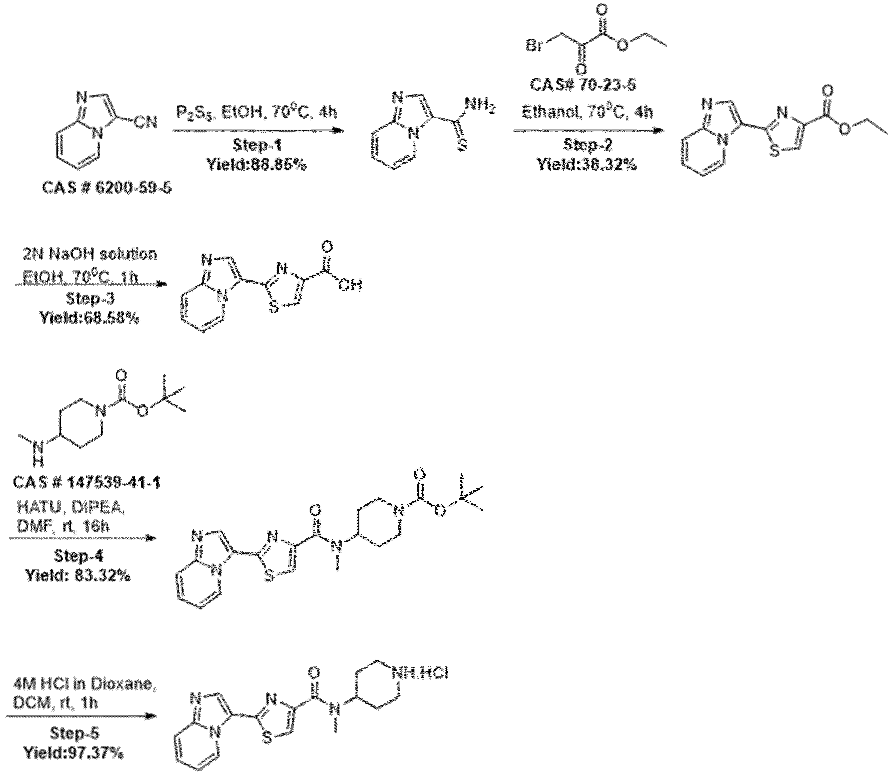

### Steps 1 to 3: Synthesis of 2-(imidazo[1,2-a] pyridin-3-yl) thiazole-4-carboxylic acid

### 2-(imidazo[1,2-a] pyridin-3-yl) thiazole-4-carboxylic acid was prepared following steps 1 to 3 of the synthesis of compound SLN5-X-0974

### Step-4: Synthesis of tert-butyl 4-(2-(imidazo[1,2-a]pyridin-3-yl)-N-methylthiazole-4-carboxamido)piperidine-1-carboxylate

To a stirred solution of 2-(imidazo[1,2-a] pyridin-3-yl)thiazole-4-carboxylic acid (0.8 g, 3.26 mmol) in DMF (8 mL) was added HATU (1.86g, 4.89 mmol) and DIPEA (1.68 mL, 9.78 mmol) at rt. After 30min, tert-butyl 4-(ethylamino) piperidine-1-carboxylate (0.84 g, 3.91 mmol) was added at rt and resulting reaction mixture was stirred at rt for 16 h. Progress of the reaction was monitored by TLC. After completion of reaction, the reaction mixture was poured into ice cold water (70mL) and extracted with EtOAc (3 X 70 mL). Combined organic layer was dried over Na₂SO₄, filtered and concentrated u/vacuum to get crude material, which was purified by reverse phase column chromatography (product eluted at 70% MeCN in water) to get tert-butyl 4-(2-(imidazo[1,2-a] pyridin-3-yl)-N-methylthiazole-4-carboxamido) piperidine-1-carboxylate (1.2 g, 83.32 %) as a brown solid.
**LCMS [ESI, M+1]:** 442.1 (RT: 1.371 min, Purity: 96.52%),

### Step-5: Synthesis of 2-(imidazo[1,2-a]pyridin-3-yl)-N-methyl-N-(piperidin-4-yl)thiazole-4-carboxamide hydrochloride (SLN5-X-1293):

To a stirred solution of tert-butyl 4-(2-(imidazo[1,2-a]pyridin-3-yl)-N-methylthiazole-4-carboxamido)piperidine-1-carboxylate (1.2 g, 2.71 mmol) in DCM (12 mL, 10V) was added 4 M HCl in Dioxane (6 mL, 5V) at rt. The reaction mixture was stirred at rt for 1h. Progress of reaction was monitored by TLC. After completion of reaction, the reaction mixture was concentrated u/vacuum to get crude material, which was diluted with water (20mL) and extracted EtOAc (2 X 20 mL), separated aqueous layer were lyophilized to get 2-(imidazo[1,2-a]pyridin-3-yl)-N-methyl-N-(piperidin-4-yl)thiazole-4-carboxamide hydrochloride (1.0 g, 97.37 %) as a light brown solid.
**LCMS [ESI, M+1]:** 341.7(RT: 0.83min, Purity:100%),
**HPLC Purity:** RT: 4.00min, Purity: 99.16%,
**¹H NMR (400 MHz, MeOD):** δ 9.97-9.91 (m, 1H), 8.94 (s, 1H), 8.28 (s, 1H), 8.19-8.10 (m, 2H), 7.73 (t, *J* = 6.8 Hz, 1H), 4.72-4.41 (m, 1H), 3.59 (d, *J* = 11.6 Hz, 1H), 3.50-3.41 (m, 1H), 3.23 (bs, 3H), 3.15-2.97 (m, 2H), 2.29-2.14 (m, 4H).

### Experimental protocol for SLN5-X-1420 (Batch-02):

### Synthesis of (S)-2-(7-fluoroimidazo[1,2-a] pyridin-3-yl)-N-Isopropyl-N-(pyrrolidin-3-yl) thiazole-4-carboxamide hydrochloride (SLN5-X-1420)

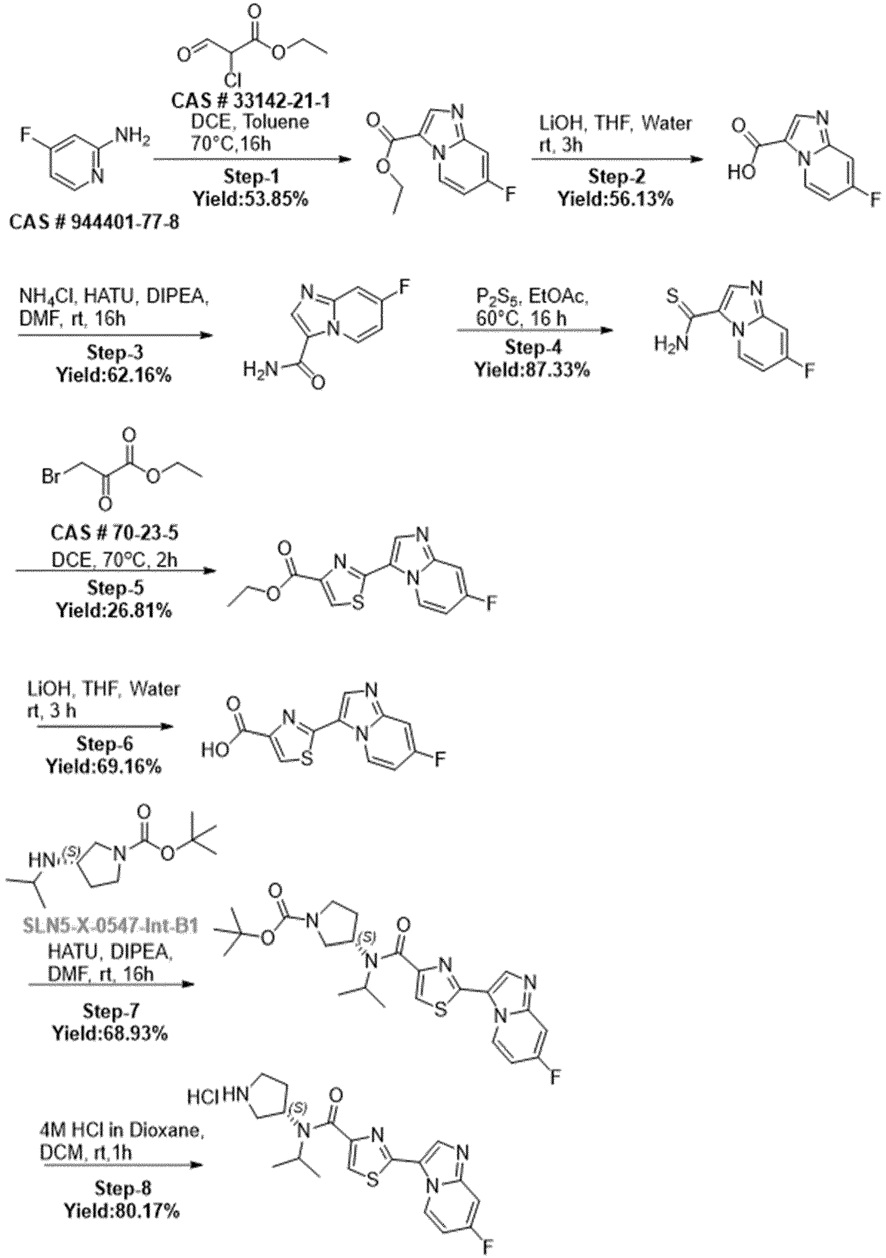

### Step-1: Synthesis of ethyl 7-fluoroimidazo[1,2-a] pyridine-3-carboxylate

To the stirred solution of 4-fluoropyridin-2-amine (25.0 g, 222.9 mmol) in DCE (100 mL, 4V) was added ethyl 2-chloro-3-oxopropanoate (44.5 g, in 445 mL toluene) at 0 °C. After addition the reaction mixture was stirred at 70 °C for 16 h. The progress of the reaction was monitored on TLC. After completion of reaction, the reaction mixture was poured in to the sat. NaHCO₃ solution (1000 mL) and extracted with DCM (3 X 500mL). Then combined organic layer was dried over Na₂SO₄ and evaporated under reduced pressure to get crude material. The crude was purified by normal phase column chromatography using 35% EtOAc in hexane to get ethyl 7-fluoroimidazo[1,2-a] pyridine-3-carboxylate (75 g, 53.85 % yield) as a pale yellow solid.

**LCMS [ESI, M+1]:** 209.1 (RT: 1.70 min, Purity: 97.97%)
**¹H NMR (400 MHz, DMSO-d6):** δ 9.26-9.23 (m, 1H), 8.29 (s, 1H), 7.73 (dd, J =2.4,10.0 Hz, 1H), 7.33 - 7.28 (m, 1H), 4.36 (q, J = 7.2,14.4 Hz, 2H), 1.34 (t, J = 7.2 Hz, 3H). Note: Performed in 3 parallel reactions, each of 25.0 g (25.0 g X 3).

### Step-2: Synthesis of 7-fluoroimidazo[1,2-a] pyridine-3-carboxylic acid

To the stirred solution of ethyl 7-fluoroimidazo[1,2-a] pyridine-3-carboxylate (35.0 g, 168.1 mmol) in THF (350 mL, 10V) was added solution of lithium hydroxide in water (35.27 g, 840.5 mmol, 350.0 mL) at 0 °C. After addition the reaction mixture was stirred at room temperature for 3h. The progress of the reaction was monitored on TLC. After completion of reaction, the reaction mixture was concentrated u/v. The crude was diluted with water (500 mL) extracted with EtOAc (1 × 500 mL). then the Aq. layer was acidified by using 1 N HCl solution (pH = 2-3). precipitated solid was filtered and dried under reduced pressure to get 7-fluoroimidazo[1,2-a] pyridine-3-carboxylic acid (34.0 g, 56.13 %) as an off white solid.

**LCMS [ESI, M+1]:** 181.1 (RT: 0.37 min, Purity: 97.87%).

Note: Performed in 2 parallel reactions, each of 35.0 g (35.0 g X 2).

### Step-3: Synthesis of 7-fluoroimidazo[1,2-a]pyridine-3-carboxamide

To a stirred solution of 7-fluoroimidazo[1,2-a] pyridine-3-carboxylic acid (17.0 g, 94.3 mmol) in DMF (340 mL, 20V), was added HATU (53.78 g, 141.5 mmol) and N, N-Diisopropylethylamine (48.65 mL, 282.9 mmol) at room temperature. The reaction mixture was stirred at room temperature for 30 min. Then ammonium chloride (10.09 g, 188.7 mmol) was added at room temperature. Then reaction mixture was stirred at room temperature for 16h. Progress of reaction was monitored by TLC, after completion of reaction, the reaction mixture was poured into ice-cold water (1000 mL). The precipitated solid was filtered and dried under reduced pressure to get 7-fluoroimidazo[1,2-a] pyridine-3-carboxamide (21.0 g, 62.16 %) as an off white solid.

**LCMS [ESI, M+1]:** 180.0 (RT: 3.47 min, Purity: 97.75%).

Note: Performed in 2 parallel reactions, each of 34.0 g (17.0 g X 2).

### Step-4: Synthesis of 7-fluoroimidazo[1,2-a] pyridine-3-carbothioamide

To a stirred solution of 7-fluoroimidazo[1,2-a] pyridine-3-carboxamide (10.5g, 58.6 mmol) in EtOAc (210 mL, 20V), was added P₂S₅ (26.04 g, 11.7 mmol) at 0 °C. The reaction mixture was stirred at 60 °C for 16 h. Progress of reaction was monitored by TLC, after completion of reaction, the reaction mixture was quenched into a sat. NaHCO₃ solution (1500 mL) and extracted with EtOAc (4 X 500 mL). Then combined organic layer was dried over Na₂SO₄ and evaporated under reduced pressure to get crude material. crude was trituration with n-pentane (3 X 300 mL) and dried under reduce pressure to get 7-fluoroimidazo[1,2-a] pyridine-3-carbothioamide (20.0 g, 87.33 % yield) as a yellow solid.

**LCMS [ESI, M+1]:** 196.0 (RT: 1.877min, Purity: 80.0%).

Note: Performed in 2 parallel reactions, each of 10.5 g (10.5 g X 2).

### Step-5: Synthesis of ethyl 2-(7-fluoroimidazo[1,2-a] pyridin-3-yl) thiazole-4-carboxylate

To the stirred solution of 7-fluoroimidazo[1,2-a] pyridine-3-carbothioamide (10.0 g, 51.2 mmol) in DCE (200mL, 10V) at 0°C. Added ethyl 3-bromo-2-oxopropanoate (24.99 g, 128.2 mmol). The reaction mixture was stirred at 70°C for 2h. Progress of the reaction was monitored on TLC. after completion of reaction, the reaction mixture was diluted with water (1500 mL) and extracted with DCM (3 X 700 mL). Then combined organic layer was dried over Na₂SO₄ and evaporated under reduced pressure to get crude material. The crude material was purified by reverse phase column chromatography using 76% ACN in water to get ethyl 2-(7-fluoroimidazo[1,2-a] pyridin-3-yl) thiazole-4-carboxylate (8.0 g, 26.81 %) as a light brown solid.

**LCMS [ESI, M+1]:** 292.1 (RT:1.91 min, Purity: 95.60%).

Note: Performed in 2 parallel reactions, each of 10.0 g (10.0 g X 2).

### Step-6: Synthesis of 2-(7-fluoroimidazo[1,2-a] pyridin-3-yl) thiazole-4-carboxylic acid

To the stirred solution of ethyl 2-(7-fluoroimidazo[1,2-a] pyridin-3-yl) thiazole-4-carboxylate (8.0 g, 27.49 mmol) in THF (80 mL, 10V) was added solution of lithium hydroxide in water (5.76 g, 137.4 mmol, 80 mL) at 0 °C. After addition the reaction mixture was stirred at room temperature for 3 h. The progress of the reaction was monitored on TLC. After completion of reaction, the reaction mixture was concentrated under reduced pressure. The crude was diluted with water (200 mL) extracted with EtOAc (2 X 200 mL). then Aq. layer was acidified by using 1N HCl solution until (pH=~2-3). precipitated solid was filtered and dried under reduced pressure to get 2-(7-fluoroimidazo[1,2-a] pyridin-3-yl) thiazole-4-carboxylic acid (5.0 g, 69.16 %) as a light brown solid.

**LCMS [ESI, M+1]:** 264.0 (RT:1.495 min, Purity: 100%).

### Step-7: Synthesis of tert-butyl (S)-3-(2-(7-fluoroimidazo[1,2-a] pyridin-3-yl)-N-isopropyl thiazole-4-carboxamido) pyrrolidine-1-carboxylate

To a stirred solution of 2-(7-fluoroimidazo[1,2-a] pyridin-3-yl) thiazole-4-carboxylic acid (5.0 g, 19.0 mmol) in DMF (100 mL, 20V), was added HATU (10.83 g, 28.5 mmol) and N, N-Diisopropylethylamine (9.8 mL, 57.0 mmol) at 0 °C. The reaction mixture was stirred at 0 °C for 30 min. Then tert-butyl (S)-3-(isopropyl amino) pyrrolidine-1-carboxylate (5.20 g, 22.8 mmol) was added at 0 °C. Then reaction mixture was stirred at room temperature for 16 h. Progress of reaction was monitored by TLC, after completion of reaction, the reaction mixture was poured into ice-cold water (250 mL), precipitated solid was filtered and dried under reduced pressure to get crude material. The crude was purified by normal phase column chromatography using 3% MeOH in DCM to get tert-butyl (S)-3-(2-(7-fluoroimidazo[1,2-a] pyridin-3-yl)-N-isopropylthiazole-4-carboxamido) pyrrolidine-1-carboxylate (6.2 g, 68.93 % yield) as a light brown solid.

**LCMS [ESI, M+1]:** 474.2 (RT: 2.26 min, Purity: 94.66%).

### Step-8: Synthesis of (S)-2-(7-fluoroimidazo[1,2-a] pyridin-3-yl)-N-Isopropyl-N-(pyrrolidin-3-yl) thiazole-4-carboxamide hydrochloride (SLN5-X-1420):

To the stirred solution of tert-butyl (S)-3-(2-(7-fluoroimidazo[1,2-a] pyridin-3-yl)-N-isopropylthiazole-4-carboxamido) pyrrolidine-1-carboxylate (6.2 g, 13.09 mmol) in DCM (62 mL, 10V) was added 4M HCl in dioxane (31.0 mL, 5V) at 0 °C. The reaction mixture allowed to stir at room temperature for 1h. Progress of the reaction was monitored on TLC, after completion of reaction. The reaction mixture was concentrated under vacuum. The crude material was purified by reverse phase column chromatography using 8% ACN in water to get ((S)-2-(7-fluoroimidazo[1,2-a] pyridin-3-yl)-N-Isopropyl-N-(pyrrolidin-3-yl) thiazole-4-carboxamide hydrochloride (4.3 g, 80.17 % yield) as a pale yellow solid.
**LCMS [ESI, M+1]:** 374.1 (RT: 1.13min, Purity: 98.89%),
**HPLC Purity:** RT: 5.12min, Purity: 99.0%
**Chiral HPLC Purity:** RT: 4.78min, Purity: 99.32%
**¹H NMR (400 MHz, DMSO-d6)** δ 9.59 (t, J = 5.6 Hz, 2H), 8.80 (bs, 1H), 8.72 (s, 1H), 8.18 (s, 1H), 7.88 (dd, *J* = 2.4 Hz, 9.2 Hz, 1H), 7.58-7.54 (m, 1H), 4.35(bs, 2H), 3.58 - 3.51(m, 2H), 3.38 - 3.33 (m, 1H), 3.17 - 3.13 (m, 1H), 2.28(s, 2H), 1.25 (bs, 6H).

**Table 1: Compounds according to the present invention, chemical structures, and names. All compounds were isolated as the corresponding HCl salts**

| **Compound/ Formula Number** | **Structure** | **Chemical Name** |
|---|---|---|
| A | 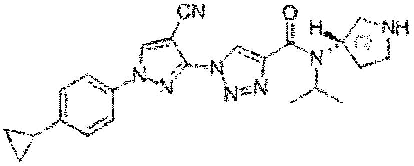 | N-[(S)-3-pyrrolidinyl]-N-isopropyl-1-[4-cyano-1-(p-cyclopropylphenyl)-3-pyrazolyl]-1H-1,2,3-triazole-4-carboxamide |
| B | 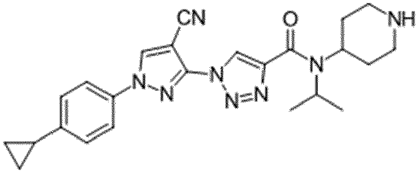 | N-isopropyl-N-4-piperidyl-1-[4-cyano-1 -(p-cy clopropylphenyl)-3-pyrazolyl]-1H-1,2,3-triazole-4-carboxamide |
| C | 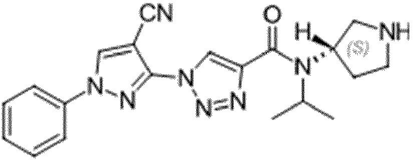 | N-[(S)-3-pyrrolidinyl]-N-isopropyl-1-(4-cyano-1-phenyl-3-pyrazolyl)-1H-1,2,3-triazole-4-carboxamide |
| D | 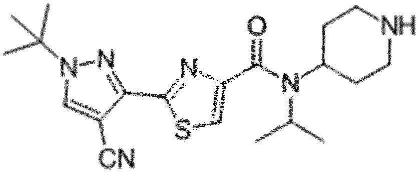 | N-isopropyl-N-4-piperidyl-2-[1-(tert-butyl)-4-cyano-3 - pyrazolyl]-1,3-thiazole-4-carboxamide |
| E | 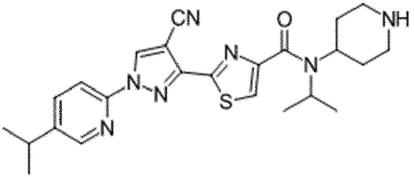 | N-isopropyl-N-4-piperidyl-2-[4-cyano-1-(5-isopropyl-2-pyridyl)-3-pyrazolyl]-1,3-thiazole-4-carboxamide |
| F | 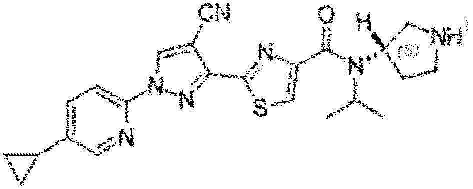 | N-[(S)-3-pyrrolidinyl]-N-isopropyl-2-[4-cyano-1-(5-cyclopropyl-2-pyridyl)-3 - pyrazolyl]-1,3-thiazole-4-carboxamide |
| G | 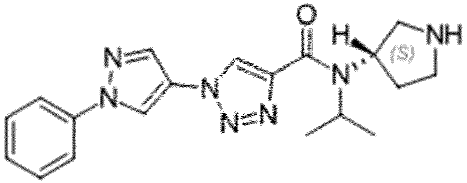 | N-[(S)-3-pyrrolidinyl]-N-isopropyl-1-(1-phenyl-4-pyrazolyl)-1H-1,2,3-triazole-4-carboxamide |
| H | 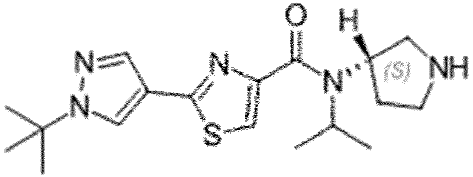 | N-[(S)-3-pyrrolidinyl]-N-isopropyl-2-[1-(tert-butyl)-4-pyrazolyl]-1,3-thiazole-4-carboxamide |
| I | 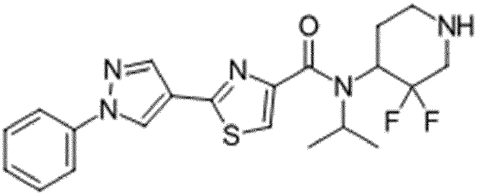 | N-(3,3-difluoro-4-piperidyl)-N-isopropyl-2-(1-phenyl-4-pyrazolyl)-1,3-thiazole-4-carboxamide |
| J | 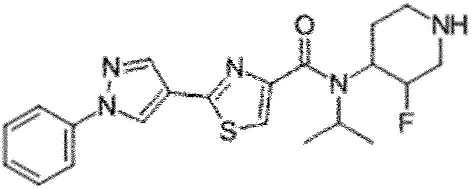 | N-(3-fluoro-4-piperidyl)-N-isopropyl-2-(1-phenyl-4-pyrazolyl)-1,3-thiazole-4-carboxamide |
| K | 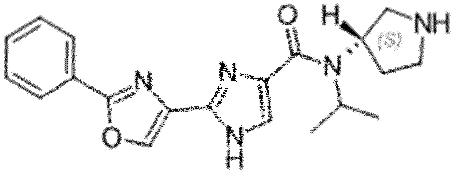 | N-[(S)-3-pyrrolidinyl]-N-isopropyl-2-(2-phenyl-1,3-oxazol-4-yl)-4-imidazolecarboxamide |
| L | 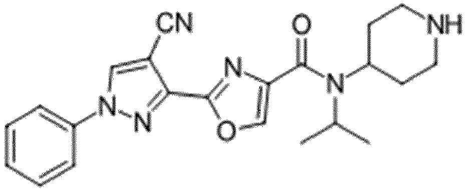 | N-isopropyl-N-4-piperidyl-2-(4-cyano-1-phenyl-3-pyrazolyl)-1,3-oxazole-4-carboxamide |
| M | 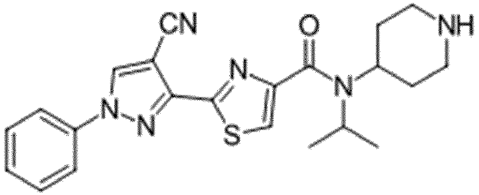 | N-isopropyl-N-4-piperidyl-2-(4-cyano-1-phenyl-3-pyrazolyl)-1,3 -thiazole-4-carboxamide |
| N | 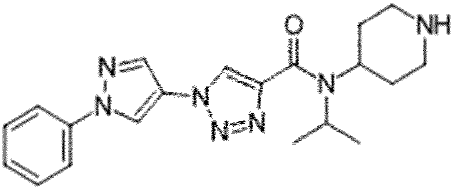 | N-isopropyl-N-4-piperidyl-1-(1-phenyl-4-pyrazolyl)-1H-1,2,3-triazole-4-carboxamide |
| SLN5-X-0973 | 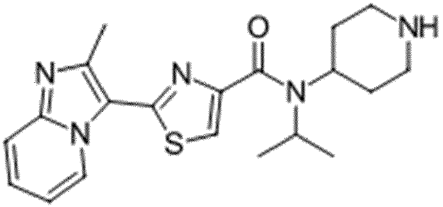 | N-isopropyl-2-(2-methylimidazo[1,2-a] pyridin-3-yl)-N-(piperidin-4-yl) thiazole-4-carboxamide |
| SLN5-X-0974 | 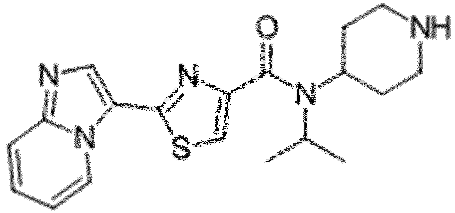 | 2-(imidazo[1,2-a] pyridin-3-yl)-N-Isopropyl-N-(piperidin-4-yl) thiazole-4-carboxamide |
| SLN5-X-0975 | 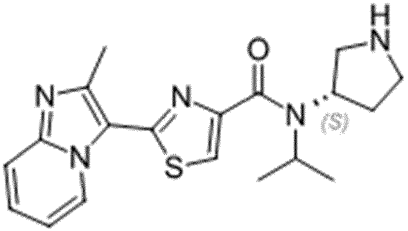 | (S)-N-isopropyl-2-(2-methylimidazo[1,2-a] pyridin-3-yl)-N-(pyrrolidin-3-yl) thiazole-4-carboxamide |
| SLN5-X-0976 | 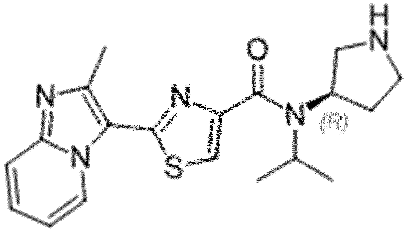 | (R)-N-isopropyl-2-(2-methylimidazo[1,2-a] pyridin-3-yl)-N-(pyrrolidin-3-yl) thiazole-4-carboxamide |
| SLN5-X-0977 | 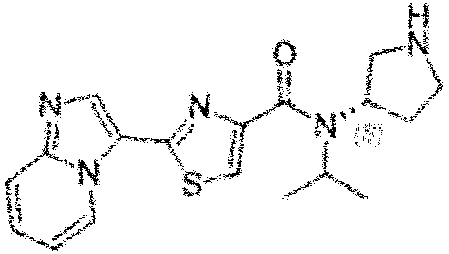 | ((S)-2-(imidazo[1,2-a] pyridin-3-yl)-N-Isopropyl-N-(pyrrolidin-3-yl) thiazole-4-carboxamide |
| SLN5-X-0978 | 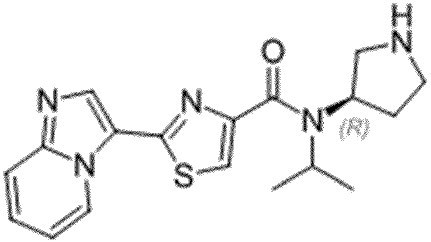 | (R)-2-(imidazo[1,2-a] pyridin-3-yl)-N-Isopropyl-N-(pyrrolidin-3-yl) thiazole-4-carboxamide |
| SLN5-X-0979 | 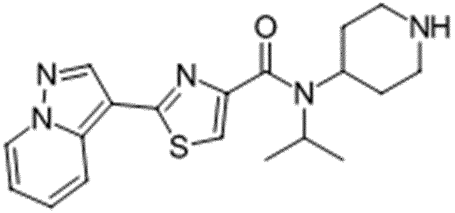 | tert-butyl 4-(N-isopropyl-2-(pyrazolo[1,5-a] pyridin-3-yl)thiazole-4-carboxamido)piperidine-1 - carboxylate |
| SLN5-X-0980 | 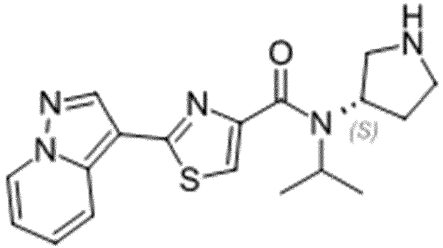 | (S)-N-isopropyl-2-(pyrazolo[1, 5-a] pyridin-3-yl)-N-(pyrrolidin-3-yl) thiazole-4-carboxamide |
| SLN5-X-0981 | 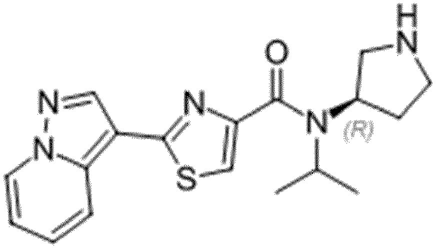 | (R)-N-isopropyl-2-(pyrazolo[1,5-a] pyridin-3-yl)-N-(pyrrolidin-3-yl) thiazole-4-carboxamide |
| SLN5-X-1290 | 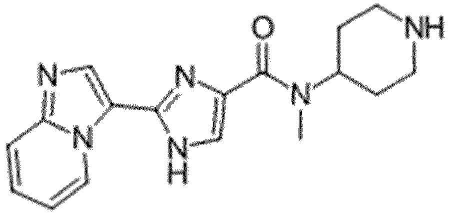 | 2-(imidazo[1,2-a] pyridin-3-yl)-N-Methyl-N-(piperidin-4-yl)-1H-imidazole-4-carboxamide |
| SLN5-X-1291 | 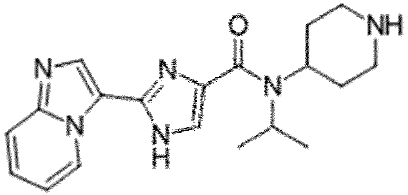 | 2-(imidazo[1,2-a] pyridin-3-yl)-N-Isopropyl-N-(piperidin-4-yl)-1H-imidazole-4-carboxamide |
| SLN5-X-1292 | 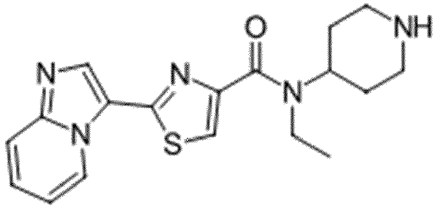 | N-ethyl-2-(imidazo[1,2-a]pyridin-3-yl)-N-(piperidin-4-yl)thiazole-4-carboxamide |
| SLN5-X-1293 | 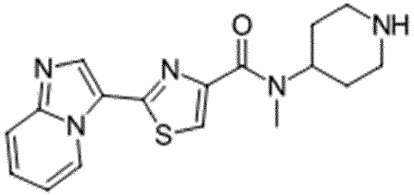 | 2-(imidazo[1,2-a]pyridin-3-yl)-N-methyl-N-(piperidin-4-yl)thiazole-4-carboxamide |
| SLN5-X-1420 | 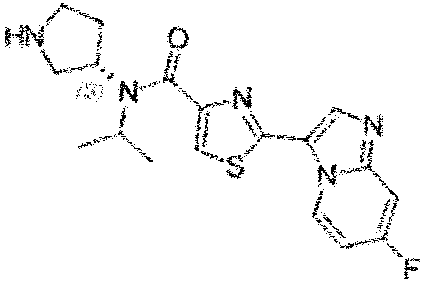 | (S)-2-(7-fluoroimidazo[1,2-a] pyridin-3-yl)-N-Isopropyl-N-(pyrrolidin-3-yl) thiazole-4-carboxamide |

### Biological assays and data

As stated above, the compounds of the present invention induce and/or stimulate autophagy and are useful in treating autophagy-related diseases. The biological activity of the compounds of the present invention can be determined by any appropriate test to determine the ability to induce and/or stimulate autophagy.

### Assessment of stimulation of autophagy

Compounds of this invention were assessed for their ability to stimulate autophagy using one or more of the assays as described below.

### Assay to measure lysosome and autolysosome formation

Lysosomes play a fundamental role in the autophagic pathway by fusing with autophagosomes and creating 'autolysosomes' in order to digest their contents. Stimulation of lysosome and autolysosome formation by a compound is indicative of a stimulation of autophagy. The ability of compounds to stimulate lysosome and autolysosome formation, and thus autophagy, in live cells was assessed via fluorescent microscopy using various fluorescent stains for labelling and tracking acidic organelles (including lysosomes and autolysosomes) such as: LysoView^{™} 650 (70059 and 70059-T, Biotium), LysoViewTM633 (70058 and 70058-T, Biotium) and LysoTracker^{™} Deep Red (L12492, ThermoFisher Scientific). The cellular phenotype was quantitatively assessed for the induction of acidic vesicle formation and compared to a non-treated control, thus providing a measure of the ability of the compound under investigation to stimulate autophagy.

Representative procedure using LysoView^{™}633 dye or LysoTracker^{™} Deep Red dye: Human osteosarcoma U2OS cells (40,000 cells/well) were seeded in a 24 well glass bottom plate (Sensoplate, Greiner Bio-One) and were incubated overnight in a humidified atmosphere at 37 °C and 5% CO₂. Cells were grown in DMEM (Gibco) supplemented with 10% Fetal Bovine Serum (FBS) and 100 units/ml penicillin and 100 µg/ml streptomycin (Invitrogen). After the attachment period, cells were treated with different compounds of interest (at various concentrations in DMSO) or DMSO (non-treated control) in cell culture medium and were incubated for 24 hours. Compound-containing medium was removed, and cells were incubated with pre-warmed cell culture medium containing 1x LysoView^{™} 633 (70058 and 70058-T, Biotium) or 50 nM LysoTracker^{™} Deep Red (L12492, ThermoFisher Scientific) for 45 minutes at 37°C. Finally, cell nuclei were stained for 10 minutes using Hoechst 33342 (1 µg/mL) and then the medium was replaced with fresh medium. The 24 well plate was then fitted into a heated stage on the microscope and cells maintained at 37 °C. Images were captured using appropriate filter set for Cy5 and DAPI detection with the EVOS M7000 Microscope (ThermoFisher Scientific). The cellular phenotype was visually assessed for the induction of acidic vesicle formation relative to the non-treated control; multiple images were acquired and analyzed using ImageJ or Cell Profiler.

### Tandem reporter assay (assay to assess autophagic flux)

Selected compounds of this invention were also assessed for their ability to stimulate autophagy using a U2OS cell line stabling expressing RFP-eGFP-hLC3b (a tandem reporter cell line).

During the process of autophagy, the cargo to be degraded is first enveloped by organelles called autophagosomes. These autophagosomes then fuse with lysosomes, causing them to become acidified and their digestive enzymes to become activated. The term "autophagic flux" is used to represent the dynamic process of autophagy: autophagic flux refers to the whole process of autophagy, including autophagosome formation, maturation, fusion with lysosomes, subsequent breakdown, and the release of macromolecules back into the cytosol (Zhang XJ, Chen S, Huang KX, Le WD. Why should autophagic flux be assessed? Acta Pharmacol Sin. 2013 May; 34(5): 595-9. doi: 10.1038/aps.2012.184. Epub 2013 Mar 11). Compounds that stimulate autophagic flux stimulate the dynamic process of autophagy (the whole process of autophagy).

LC3b is a protein found in the membrane of autophagosomes which has been used to generate genetic reporters of autophagy in cells. In these systems, a tandem fusion of LC3b is engineered with two fluorescent proteins of different wavelengths: one which is acid sensitive (for example eGFP, which fluoresces green) and the other which is acid insensitive (for example RFP, which fluoresces red). Cells expressing RFP-eGFP-hLC3b can be examined using fluorescent microscopy: the autophagosomes present will fluoresce in both channels (either yellow in an overlay of both channels or puncta that are present in both the individual red and green channels) but the autolysosomes present will fluoresce in the RFP (red) channel only. Using a fluorescent microscope, the number of autophagosomes and autolysosomes can be counted, allowing for the monitoring of both the induction of autophagy (total puncta count) and the rate of autophagic flux (ratio of red-only to red-and-green puncta). The influence of small molecules on autophagy induction and flux can be assessed using this system by comparison to a non-treated control.

Representative Procedure: Human osteosarcoma U2OS cells stably expressing RFP-eGFP-hLC3b were seeded (10,000 cells/well) into a 96-well glass-bottom plate (Cell Carrier Ultra, Perkin Elmer) in DMEM Glutamax media (Gibco) supplemented with 10% Fetal Bovine Serum (FBS) and antibiotics (100 u/ml penicillin, 100 µg/ml streptomycin, Invitrogen), then incubated overnight in a humified atmosphere at 37 °C and 5% CO2. The cells were then treated with the compounds of interest, at various concentrations in DMSO, in a duplicated 5- or 10-point dose-response. 8 wells were treated with an equivalent volume of DMSO, 4 with 0.3 µM torin-1 as a positive control (MCE) and 4 wells with 0.4 µM torin-1 plus 0.2 µM bafilomycin (MCE) as a control for flux inhibition. The cells were returned to the incubator and the treatment continued for 24 hours. After treatment, the media was aspirated off and the cells fixed with a solution of 4% formaldehyde + 1% glutaraldehyde (Sigma) in dPBS (plus magnesium and calcium, Gibco). Fixation was allowed to proceed for 15 minutes at room temperature then the fixative solution discarded and replaced with PBS (Gibco) plus Hoechst 33342 (1 µg/ml, Sigma). After 30 minutes the plates were imaged on an Opera Phenix confocal microscope (Perkin-Elmer) using a 40x water objective, collecting using DAPI, mCherry and GFP channels. Cells were detected based upon the staining of their nuclei with DAPI using the Harmony image analysis software (Perkin-Elmer) and the number of autophagosomes (GFP and RFP puncta) and autolysosomes (RFP only puncta) per cell were counted using the spot picking function within the software.

Selected compounds of this invention showed an increase in the number of RFP-only puncta (autolysosomes) relative to a DMSO-treated control (non-treated control) at different concentrations. This is evidence for significant stimulation of autophagic flux (and thus the whole process of autophagy) by the compounds of this invention, and particularly by these compounds under the conditions as tested. Table 2 shows this data for selected compounds. Compounds having an activity designated as "+" provided between a 1% and 30% increase in activity (based on the number of RFP-only puncta [autolysosomes] as a percentage of the DMSO treated control [non-treated control]). Compounds having an activity designated as "++" provided between a 30% and 50% increase in activity (based on the number of RFP-only puncta [autolysosomes] as a percentage of the DMSO treated control [non-treated control]). Compounds having an activity designated as "+++" provided between a 50% and 100% increase in activity (based on the number of RFP-only puncta [autolysosomes]) as a percentage of the DMSO treated control [non-treated control]). Compounds having an activity designated as "++++" provided above a 100% increase in activity (based on the number of RFP-only puncta [autolysosomes]) as a percentage of the DMSO treated control [non-treated control]).

**Table 2: Assessment of autophagy stimulatory activity of compounds based on the change in the number of RFP-only puncta (autolysomes), relative to a DMSO-treated control (non-treated control) in a U2OS cell line stably expressing RFP-eGFP-hLC3b (a tandem reporter cell line)**

| **Compound** | **Compound concentration (mM)** | **Activity** |
|---|---|---|
| A | 1 | ++ |
| B | 1 | ++ |
| C | 1 | ++ |
| D | 1 | + |
| E | 0.9 | ++++ |
| F | 1.3 | + |
| G | 1 | + |
| H | 10 | +++ |
| I | 10 | + |
| J | 5 | +++ |
| K | 7.5 | +++ |
| L | 10 | ++ |
| M | 1 | +++ |
| N | 1 | + |
| SLN5-X-0973 | 1 | + |
| SLN5-X-0974 | 1 | + |
| SLN5-X-0975 | 1 | ++ |
| SLN5-X-0976 | 1 | + |
| SLN5-X-0977 | 1 | + |
| SLN5-X-0978 | 1 | ++++ |
| SLN5-X-0979 | 1 | ++ |
| SLN5-X-0980 | 7.5 | ++++ |
| SLN5-X-0981 | 1 | + |
| SLN5-X-1293 | 1 | + |
| SLN5-X-1420 | 1.2 | ++ |

Hence, the compounds are suitable for oral dosing making them advantageous for the treatment of various conditions related to autophagy as disclosed herein. Particularly good results can be seen with compounds E and SLN5-X-0978.

## Claims

1. A compound according to the general Formula (I) 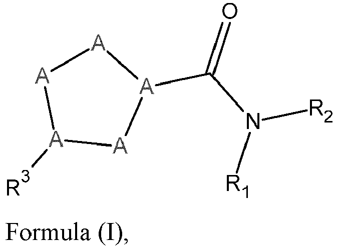 wherein
R¹ is selected from methyl, ethyl, and isopropyl
R² is a chemically possible ring of 5 or 6 atoms selected from saturated C and N and comprising at least one N, wherein the ring is optionally substituted with at least one C₁ to C₄ alkyl or halo,
A is selected from chemically possible saturated or unsaturated C, N, O, and S, comprising at least one N,
R³ is selected from 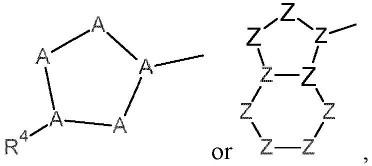 optionally substituted with - CN, halo or methyl, wherein A is as above, and Z is independently selected from chemically possible combinations of C, CH, N and NH
R⁴ is selected from hexyl, -C(CH₃)₃, or 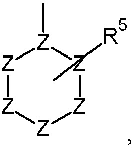
wherein Z is above and R⁵ is independently selected from H, straight or branched C₂ to C₆ alkyl, straight or branched C₁ to C₄ hydroxyalkyl, such as, for example, -CH₂-CH₂-OH, straight or branched C₁ to C₄ hydroxyalkoxy, such as, for example, -O-CH₂-CH₂-OH, halo, trifluoromethyl, cyclopropyl, or cyano, and
a physiologically acceptable salt, a solvate, a hydrate, an enantiomer or a polymorph thereof.

2. A compound according to claim 1 according to the general Formula (II), 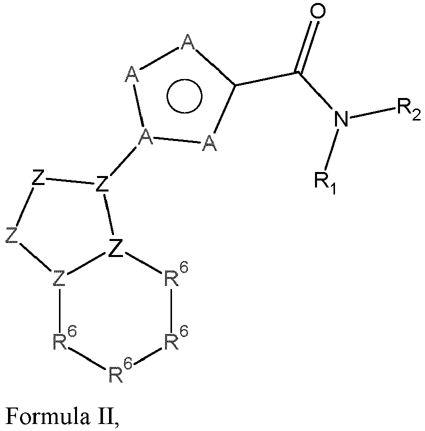
wherein R1, R2, Z and A are as above, R6 is independently selected from chemically possible combinations of C, CH and CH2 and wherein the rings are optionally substituted with -CN, halo or methyl,
a physiologically acceptable salt, a solvate, a hydrate, an enantiomer or a polymorph thereof.

3. A compound according to claim 1 according to the general Formula (III), 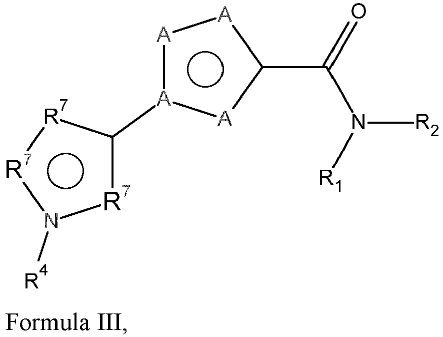
wherein R1, R2, R4 and A are as above, wherein the rings are optionally substituted with -CN, halo or methyl,
R7 is independently selected from chemically possible combinations of C, CH, N and NH, optionally substituted with cyano, and
a physiologically acceptable salt, a solvate, a hydrate, an enantiomer or a polymorph thereof.

4. A compound selected from the following group 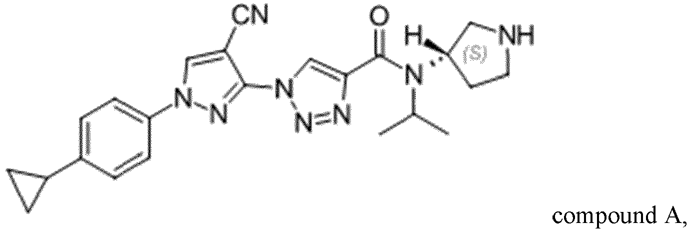 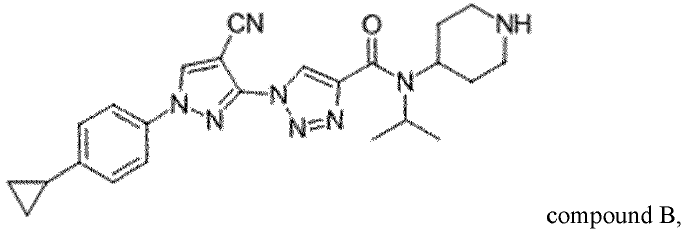 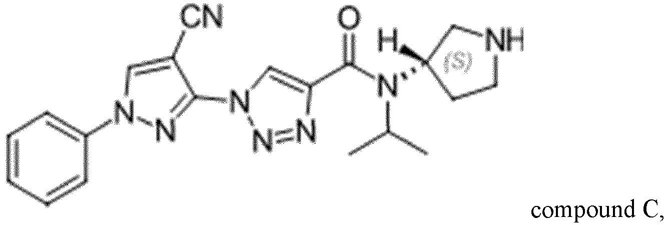 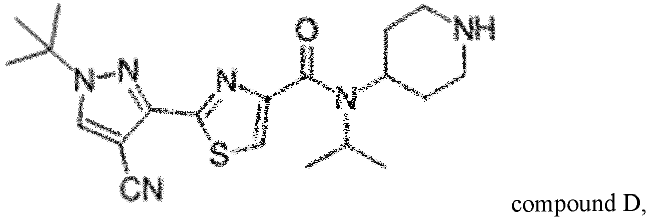 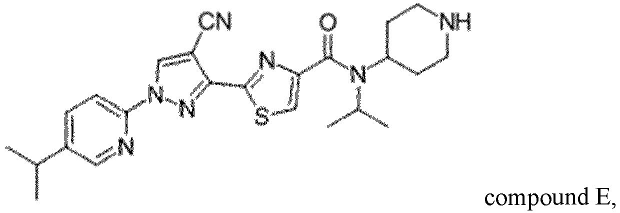 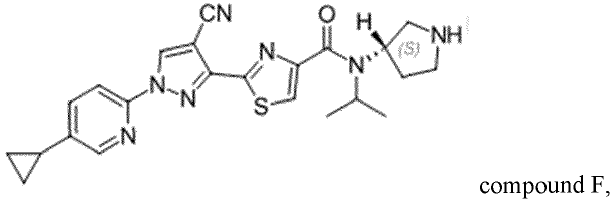 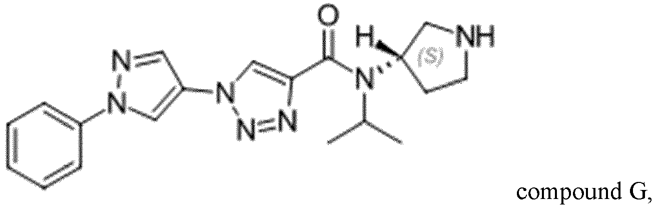 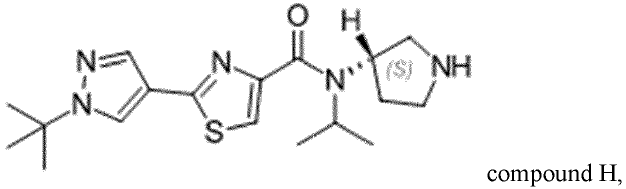 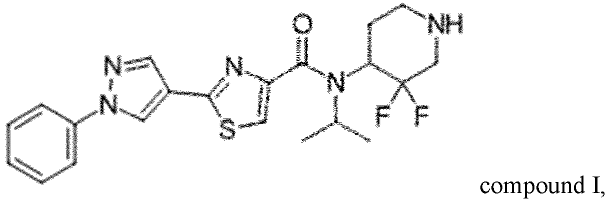 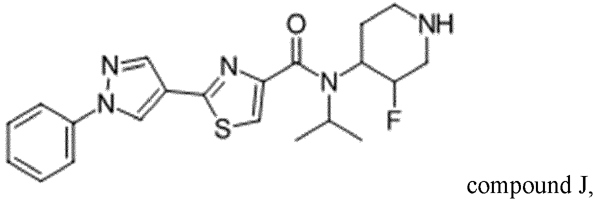 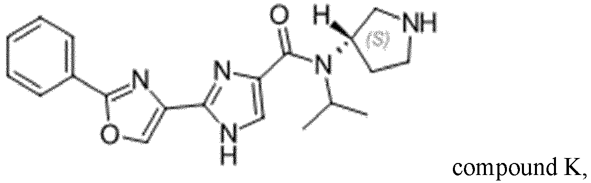 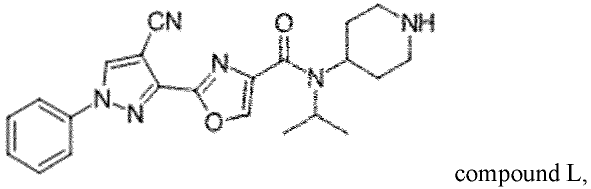 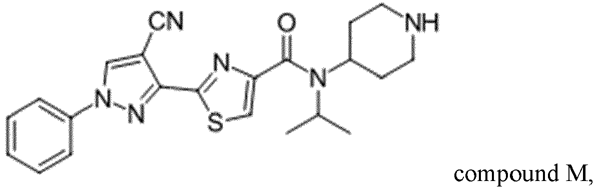 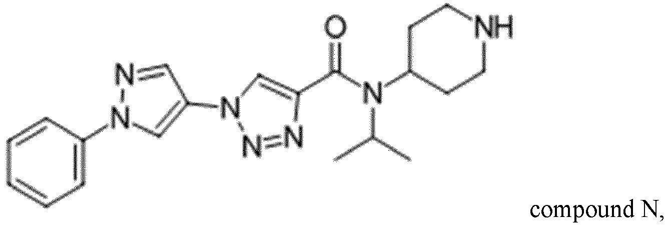 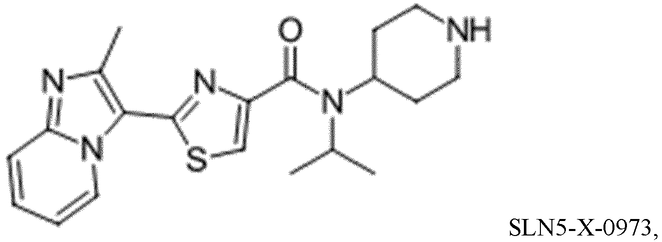 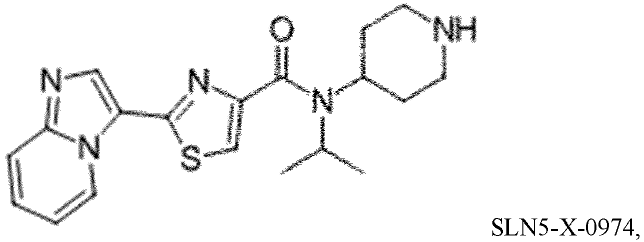 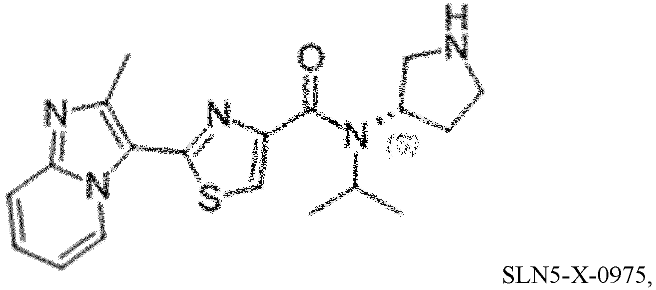 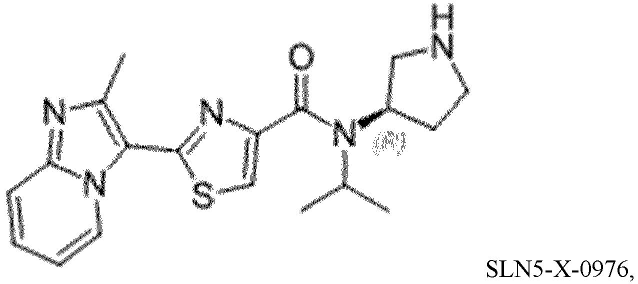 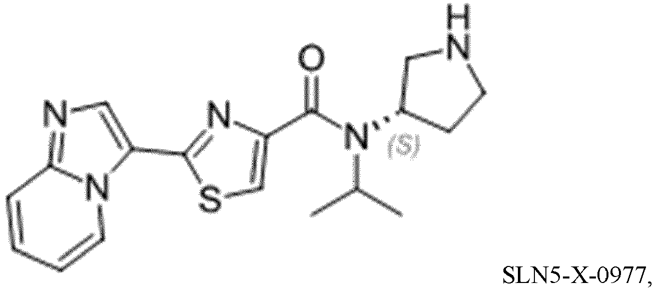 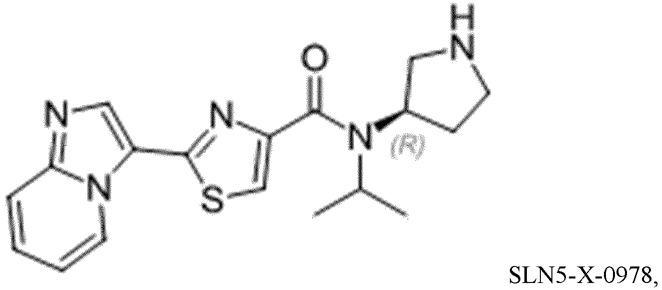 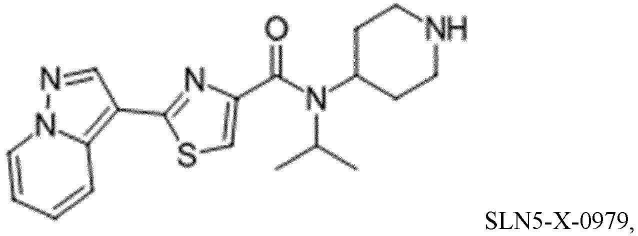 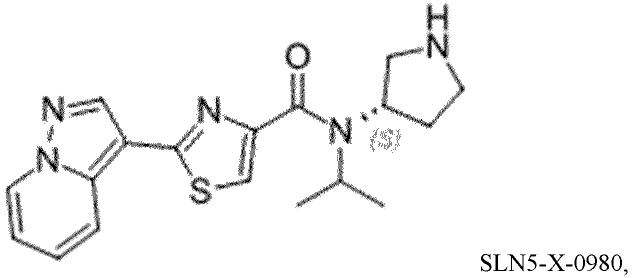 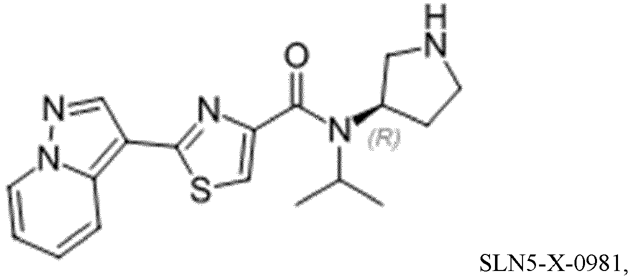 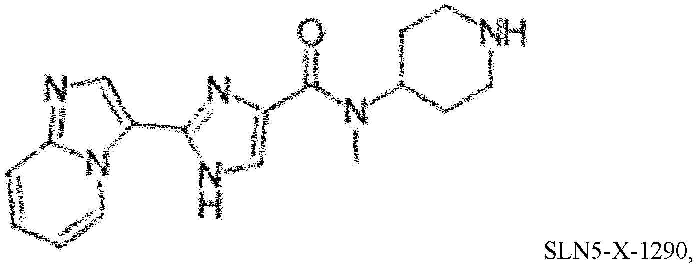 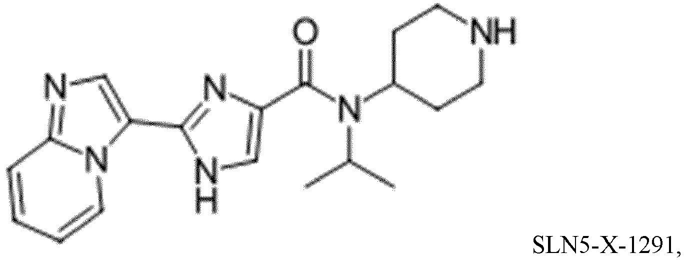 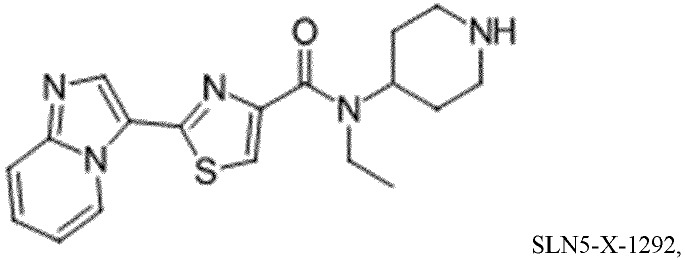 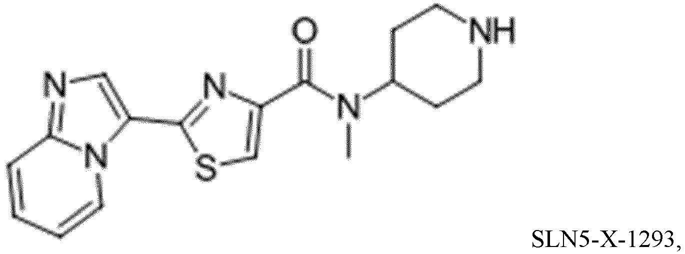 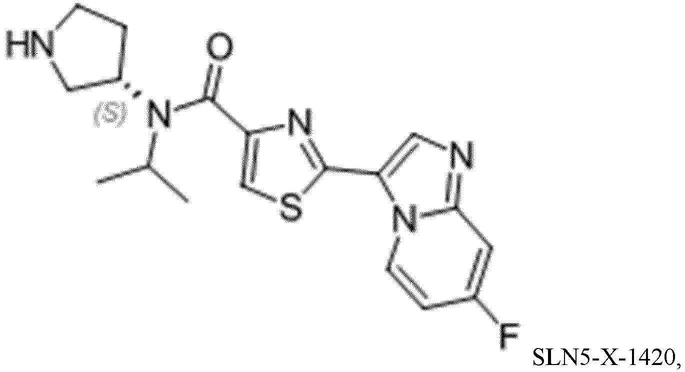 and
a physiologically acceptable salt, a solvate, a hydrate, an enantiomer or a polymorph thereof.

5. A pharmaceutical composition, comprising a pharmaceutically effective amount of the compound according to any one of claims 1 to 4, and a pharmaceutically acceptable carrier.

6. The compound according to any one of claims 1 to 4 or the pharmaceutical composition according to claim 5 for use in the prevention and/or treatment of diseases in a mammalian subject, such as a human.

7. The compound according to any one of claims 1 to 4 or the pharmaceutical composition according to claim 5 for use as an autophagy inducer, wherein preferably said use is cosmetic and/or in vitro.

8. The compound according to any one of claims 1 to 4 or the pharmaceutical composition according to claim 5 for use in the prevention and/or treatment of an autophagy-related disease or condition in a mammalian subject, such as a human.

9. The compound for use according to claim 8, wherein said autophagy-related disease or condition is selected from the group consisting of systemic lupus erythematosus, cancer, liver diseases, al antitrypsin deficiency, Charcot Marie Tooth syndrome, Rett Syndrome, Sickle Cell disease, Wilson Disease, amyloidosis, Gaucher's diseases, lysosomal and glycogen storage disorders, cystic fibrosis; viral infection and diseases, human cytomegalovirus (HCMV) infection, hepatitis B, human immunodeficiency virus infection, Zika virus infection, coronavirus infection, HCoV-229E, HCoV-NL63, betacoronavirus infection, such as HCoV-OC43, SARS-CoV-1, HCoV-HKUl, MERS-CoV or SARS-CoV-2, bacterial infections, metabolic disorders, diabetes, fibrosis, wound healing disorders, Niemann-Pick type C (NPC) disease, fibrinogen storage disease (FSB), inclusion body disease (IBD), muscular dystrophy, Duchenne muscular dystrophy, Limb-girdle muscular dystrophy, myopathy, myofibrillar myopathy, hereditary myopathy, diabetic cardiomyopathy, anti-inflammatory disorders, autoimmune diseases, multiple sclerosis, rheumatoid arthritis, irritable bowel syndrome, Crohn's disease, vascular disorders, coronary artery diseases, myocardial infarction, unstable angina pectoris, atherosclerosis or vasculitis, Behcet's syndrome, giant cell arteritis, polymyalgia rheumatica, Wegener's granulomatosis, Churg- Strauss syndrome, vasculitis, Henoch-Schonlein purpura, Kawasaki disease, viral infection or replication, pox virus infection, herpes virus infection, asthma, allergic rhinitis, COPD, osteoporosis, organ transplant rejection, psoriasis, hypertrophic scarring (keloid formation), adhesion formations following general or gynecological surgery, lung fibrosis, liver fibrosis, kidney fibrosis, disorders caused by intracellular parasites, malaria, tuberculosis, neuropathic pain, postoperative phantom limb pain or postherpetic neuralgia, allergies, antigen induced recall response, immune response suppression, muscle degeneration and atrophy, frailty in aging, spinal cord injury, and diseases and conditions involving misfolded and/or nonfolded proteins that can be effectively treated with compounds that are effective when provided systemically as well as age-related forms of the diseases and conditions (such as cancer, and metabolic syndrome).

10. The compound for use according to claim 8 or 9, wherein said prevention and/or treatment comprises a combination of at least two compounds for use according to claim 8 or 9, and/or a combination with at least one additional pharmaceutically active substance for said autophagy-related disease or condition.

11. The compound for use according to any one of claims 8 to 10, wherein said prevention and/or treatment further comprises detecting and/or monitoring in said subject a response of at least one autophagy-biomarker, preferably selected from the group of BECN1, and the ATG8/LC3 family, including LC3A, LC3B, LC3C), LC3-II, ULK1, p62, NBR1, ATG5 and ATG7.

12. A method for preventing and/or treating an autophagy-related disease or condition in a mammalian subject, such as a human, comprising administering to said mammal an effective amount of at least one compound according to any one of claims 1 to 4 or a pharmaceutical composition according to claim 5.

13. The method according to claim 12, wherein said autophagy-related disease or condition is selected from the group consisting of systemic lupus erythematosus, cancer, liver diseases, al antitrypsin deficiency, Charcot Marie Tooth syndrome, Rett Syndrome, Sickle Cell disease, Wilson Disease, amyloidosis, Gaucher's diseases, lysosomal and glycogen storage disorders, cystic fibrosis; viral infection and diseases, human cytomegalovirus (HCMV) infection, hepatitis B, human immunodeficiency virus infection, Zika virus infection, coronavirus infection, HCoV-229E, HCoV-NL63, betacoronavirus infection, such as HCoV-OC43, SARS-CoV-1, HCoV-HKUl, MERS-CoV or SARS-CoV-2, bacterial infections, metabolic disorders, diabetes, fibrosis, wound healing disorders, Niemann-Pick type C (NPC) disease, fibrinogen storage disease (FSB), inclusion body disease (IBD), muscular dystrophy, Duchenne muscular dystrophy, Limb-girdle muscular dystrophy, myopathy, myofibrillar myopathy, hereditary myopathy, diabetic cardiomyopathy, anti-inflammatory disorders, autoimmune diseases, multiple sclerosis, rheumatoid arthritis, irritable bowel syndrome, Crohn's disease, vascular disorders, coronary artery diseases, myocardial infarction, unstable angina pectoris, atherosclerosis or vasculitis, Behcet's syndrome, giant cell arteritis, polymyalgia rheumatica, Wegener's granulomatosis, Churg- Strauss syndrome, vasculitis, Henoch-Schonlein purpura, Kawasaki disease, viral infection or replication, pox virus infection, herpes virus infection, asthma, allergic rhinitis, COPD, osteoporosis, organ transplant rejection, psoriasis, hypertrophic scarring (keloid formation), adhesion formations following general or gynecological surgery, lung fibrosis, liver fibrosis, kidney fibrosis, disorders caused by intracellular parasites, malaria, tuberculosis, neuropathic pain, postoperative phantom limb pain or postherpetic neuralgia, allergies, antigen induced recall response, immune response suppression, muscle degeneration and atrophy, frailty in aging, spinal cord injury, and diseases and conditions involving misfolded and/or nonfolded proteins that can be effectively treated with compounds that are effective when provided systemically as well as age-related forms of the diseases and conditions (such as cancer, and metabolic syndrome).

14. The method according to claim 12 or 13, further comprising detecting and/or monitoring in said subject a response of at least one autophagy-biomarker, preferably selected from the group of BECN1, and the ATG8/LC3 family, including LC3A, LC3B, LC3C), LC3-II, ULK1, p62, NBR1, ATG5 and ATG7.
